(19) Europäisches Patentamt
European Patent Office
Office européen des brevets

(11) **EP 2 980 735 A1**

(12) **EUROPEAN PATENT APPLICATION**

(43) Date of publication:
**03.02.2016 Bulletin 2016/05**

(51) Int Cl.:
**G06Q 10/06** (2012.01)   **G06Q 40/04** (2012.01)

(21) Application number: **15075016.4**

(22) Date of filing: **30.07.2010**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **31.07.2009 US 230235 P**

(62) Document number(s) of the earlier application(s) in accordance with Art. 76 EPC:
**10805115.2 / 2 460 123**

(71) Applicant: **Global Surface Intelligence Ltd Edinburgh EH2 4AD (GB)**

(72) Inventor: **TYBURSKI, Matthew Gerard Hydes, Maryland 21082 (US)**

(74) Representative: **Gordon, Naoise Padhraic Edward Black & Associates Ltd 4 Woodside Place Glasgow G3 7QF (GB)**

Remarks:
This application was filed on 08-04-2015 as a divisional application to the application mentioned under INID code 62.

(54) **GREENHOUSE GAS GRID AND TRACKING SYSTEM**

(57) A method implemented by a computer for monitoring the status of a target vegetation attribute within a target geographical boundary by generating an allometric model for the target vegetation attribute within the target geographical boundary]and generating a geospatial database including remote sensing imagery for monitoring the target vegetation attribute within the target geographical boundary. The sample of the pixels from a remote sensing image are the result of processing by the geospatial data processing software which removes low quality and/or contaminated pixels from the remote sensing image; and creates periodic composites (i.e., monthly, quarterly, annually) for descriptive statistics from pixels within remote sensing images taken for the same location at different dates to generate a consistent time series and annual data for different years of interest.

FIG.7A

EP 2 980 735 A1

**Description**

CROSS-REFERENCE TO RELATED APPLICATIONS

This application is a PCT International application and is related to and claims priority to US Application no. 12/847,370, by Matthew G. Tyburski, filed July 30, 2010, and to U.S. provisional patent application entitled Greenhouse Gas Monitoring Grid For Terrestrial Carbon Credits having US application number 61/230,235, by Matthew G. Tyburski, filed July 31, 2009 and all of which are incorporated by reference herein.

COPYRIGHT NOTICE

A portion of the disclosure of this patent document contains material to which a claim for copyright is made. The copyright owner has no objection to the facsimile reproduction by anyone of the patent document or the patent disclosure, as it appears in the Patent and Trademark Office patent file or records, but reserves all other copyright rights whatsoever.

BACKGROUND OF THE INVENTION

1. Field

The described embodiments relate to monitoring and reporting of greenhouse gases (GHGs).

2. Description of the Related Art

A sector in the green economy is the trade in greenhouse gases (GHGs), for example, carbon gas, and this sector can be referred to generally as "GHG (e.g., carbon) trading." Using carbon as an example of a greenhouse gas, carbon emissions and offsets are traded under carbon trading mechanisms. Currently, there are both regulated and voluntary carbon trading mechanisms. A carbon trading mechanism is a legal trading scheme or standard that acknowledges certain activities as a carbon credit. One sector in carbon trading is developing carbon offsets from terrestrial (i.e., land-based) carbon sequestration and storage. Carbon is sequestered and stored by plants and/or vegetation. Under certain carbon trading mechanisms, the carbon that is sequestered and stored in plants or vegetation can be monetized as an offset through credible anthropogenic activities. One such activity is known as afforestation, reforestation and/or re-vegetation and involves the human assisted planting of trees to reduce atmospheric GHGs by carbon sequestration. Another credible activity through certain carbon trading mechanisms is known as Reduced Emissions from Deforestation and Degradation (i.e., REDD). REDD relates to the protection, preservation and/or conservation of carbon stored in trees through activities that avoid future potential GHG emissions from deforestation and/or degradation.

SUMMARY OF THE INVENTION

It is an aspect of the embodiments discussed herein to provide an effective and efficient monitoring and reporting of any greenhouse gas, for example, one or more carbon based chemical elements, through an offset activity. According to an aspect of an embodiment, any GHG offset activity related to sustainable and/or improved management of an eco-region(s) (e.g., land, agriculture, water, species), that also reduces and/or removes emissions can be monitored and reported.

The above aspects can be attained by a method and computer system for reporting on a target greenhouse gas within a geographical boundary of an offset project, by compiling policy parameters for the target greenhouse gas; generating a science plan for monitoring the target greenhouse gas for the target geographical boundary of the offset project, based upon the compiled policy parameters; generating an allometric model for the target greenhouse gas within the geographical boundary of the offset project, based upon the science plan of the target greenhouse gas for the geographic boundary, and generating a report for the target greenhouse gas within the target geographical boundary of the offset project based upon the allometric model.

These together with other aspects and advantages which will be subsequently apparent, reside in the details of construction and operation as more fully hereinafter described and claimed, reference being had to the accompanying drawings forming a part hereof.

BRIEF DESCRIPTION OF THE DRAWINGS

**FIG. 1** shows a summary of the full software process.

**FIG. 2A** shows the generic process used to develop a legal / policy analysis.

**FIG. 2B** shows the process used to retrieve text from legal / policy documents.

**FIG. 2C** shows the process used to develop a structured analysis for legal/policy documents.

**FIG. 2D** shows the generic process for the legal / policy analysis from FIG. 2a applied to voluntary mechanisms.

**FIG. 3** shows a generalized flowchart of the carbon cycle defined by the IPCC.

**FIG. 4A** shows the process used to retrieve text from the database for current and planned satellite missions.

**FIG. 4B** shows the output from the assessment of a satellite sensor's data continuity for monitoring the project lifetime of an offset project site.

**FIG.** 4C shows the process used to retrieve text from publications for relevant science on monitoring vegetation attribute(s) with the identified satellite instrument.

**FIG. 4D** shows the generic process used to develop a science plan.

**FIG. 4E** shows the generic process used to develop a science plan applied to examples for monitoring vegetation growth and stocks with MODIS imagery.

**FIG. 5A** shows an example of the process used to develop Allometric Equations 1.

**FIG. 5B** shows an example of the process used to implement Allometric Equations 1 with remote sensing imagery.

**FIG. 5C** shows an example of the process used to develop Allometric Equations 1.

**FIG. 5D** shows an example of the process used to develop Allometric Equations 2.

**FIG. 6A** shows the process used to develop the geospatial database and the contents that are stored on it.

**FIG. 6B** shows an example of a georeferenced file and a file with a gridcode.

**FIG. 7A** shows the generic process for pre-processing the raw remote sensing imagery stored in the primary database.

**FIG. 7B** shows an example of the generic process for pre-processing the raw remote sensing imagery stored in the primary database.

**FIG. 8A** shows the generic process to develop Allometric Equations 1.

**FIG. 8B** shows an example for the generic process to develop Allometric Equations 1.

**FIG. 9A** shows the first half of the generic process to develop Allometric Equations 2.

**FIG. 9B** shows an example for the first half of the generic process to develop Allometric Equations 2.

**FIG. 9C** shows examples for sampling a remote sensing imagery for a client's vector file with samples of vegetation attribute(s).

**FIG. 10A** shows the second half of the generic process to develop Allometric Equations 2.

**FIG. 10B** shows an example for the second half of the generic process to develop Allometric Equations 2.

**FIG. 10C** shows the illustrative outputs from developing Allometric Equations 2 from a Random Forest training model.

**FIG. 10D** shows the text outputs from developing Allometric Equations 2 from a Random Forest training model.

**FIG. 11 A** shows the generic process for implementing Allometric Equations 1.

**FIG. 11 B** shows an example for the generic process used for implementing Allometric Equations 1

**FIG. 11C** shows atlases used in implementing Allometics 1 in geospatial data processing software.

**FIG. 12A** shows the generic process for implementing Allometric Equations 2 with remote sensing imagery.

**FIG. 12B** shows an example for the generic process for implementing Allometric Equations 2 with remote sensing imagery.

**FIG. 12C** shows an example of the outputs of Allometric Equations 2 in a spreadsheet.

**FIG. 12D** shows an example of the mapped outputs from converting the spreadsheet output in **FIG. 12C** to geospatial data.

**FIG. 13A** shows the generic process used to obtain a digital boundary for a project site from a client.

**FIG. 13B** shows an example for the generic process used to obtain a digital boundary for a project site from a client.

**FIG. 14A** shows the generic process used to sample the client's digital boundary for a vegetation attribute and other geospatial data stored on the databases.

**FIG. 14B** shows an example for the generic process used to sample the client's digital boundary for a vegetation attribute and other geospatial data stored on the databases.

**FIG. 14C** shows an example of the process used to sample the geospatial data for a vegetation attribute with a client's digital boundary.

**FIG. 15** shows the generic process used to develop a report for a client.

**FIG. 16A** shows the Central Database.

**FIG. 16B** shows the assembly for the final report.

FIG. 17 is a functional block diagram of a computer for the embodiments of the invention.

DETAILED DESCRIPTION OF THE PREFERRED EMBODIMENTS

It is an aspect of the embodiments discussed herein to provide an effective and efficient monitoring and reporting of any GreenHouse Gas (GHG), for example, one or more carbon based chemical elements, in a credible offset activity under a trading mechanism. According to an aspect of an embodiment, any GHG offset activity related to sustainable and/or improved management of an ecoregion(s) (e.g., land, agriculture, water, species), that also reduce and/or remove emissions can be monitored and reported.

The embodiments are described by referring as an example to carbon and monitoring of one or more vegetation attributes as a carbon offset activity, however, the embodiments are not limited to carbon and carbon offset activities, but other GHGs and corresponding eco-region offset activities can be monitored and reported under any trading scheme.

The embodiments referring to the example of vegetation attributes define a GHG as the composition of one or more chemical element(s) (e.g., carbon) that are manifested in the physical property and/or structure of a plant and/or vegetation and are required for monitoring and/or reporting of an offset activity under any trading scheme. This definition for GHGs in the embodiments is not limited to vegetation, however, and can mean any composition of one or more physical elements(s) for monitoring of ecosystem function at an eco-region that are required for monitoring and/or reporting of an offset activity under any trading scheme.

Using carbon offset trading as an example, a project developer in carbon trading is a legal entity that intends to develop a carbon offset project. Project developers gain accreditation for a project in which they intend to develop as a carbon offset. Trading mechanisms accredit projects. The project developer will sell an accredited carbon offset credit to a polluter who is a legal entity that emits carbon through an industrial activity (i.e., a "carbon footprint"). The purchase of the credible carbon offset by a polluter means the polluter's carbon footprint is reduced and/or null (i.e., "carbon neutral"). Carbon trading mechanisms provide guidance on how a project developer must manage land and comply with monitoring and reporting of GHG emissions and removals at the accredited project site. Information on GHG emissions and removals at a project site is required for fulfilling the monitoring and reporting requirements for the carbon trading mechanism.

For example, the monitoring of GHG emissions and removals is reported in project design documents, project validation documents and project verification documents required for compliance and crediting for an accredited project under the carbon trading mechanism. This patent relates to a software process, executed by a computer, for monitoring GHG offset activities, for example, terrestrial carbon sequestration and storage, to fulfill the compliance guidelines under trading mechanisms relevant to the client. The monitoring information for carbon sequestration and storage is used by a client to monitor, track and/or report GHG emissions and removals at the accredited project site.

An example of the described process is for monitoring and reporting GHG emissions and removals at a project site to fulfill compliance under a relevant carbon trading mechanism. The process in this patent may also have incidental and/or collateral industrial utility for land owners and/or other related industry that owns land and is seeking to appraise GHG emissions, removals and/or make assessments of other relevant vegetation attributes in a specific plot of land. The latter may also include the internal offsetting by a company. The process is distinguished from research that is of a purely philosophical pursuit, because fulfilling monitoring and reporting compliance under a trading mechanism is explicit in the process. Academic research is defined here as monitoring vegetation and/or the terrestrial environment without the explicit intent to comply with any and/or all trading mechanisms. Without intellectually embedding the process in compliance guidance for GHG (e.g., carbon) trading, the process would have no defined industrial application. The final output of the process in this patent is a report that project developers of GHG offsets (e.g., terrestrial carbon offsets) use to show how their monitoring is compliant when they report to a relevant trading mechanism.

According to an aspect of an embodiment, a method and system is provided for reporting on a target greenhouse gas within a geographical boundary of an offset project, by compiling policy parameters for the target greenhouse gas; generating a science plan for monitoring the target greenhouse gas for the target geographical boundary of the offset project, based upon the compiled policy parameters; generating an allometric model for the target greenhouse gas within the geographical boundary of the offset project, based upon the science plan of the target greenhouse gas for the geographic boundary, and generating a report for the target greenhouse gas within the target geographical boundary of the offset project based upon the allometric model.

According to another aspect of an embodiment, the following operations are provided: Step 1) Develop a Legal/Policy Analysis for a vegetation attribute, Step 2) Develop a Science Plan to monitor a vegetation attribute, Step 3) Develop a Geospatial Database to monitor a vegetation attribute, Step 4) Develop Allometric Equations to monitor a vegetation attribute, Step 5) Implement Allometric Equations with remote sensing imagery to monitor a vegetation attribute, Step 6) Obtain a Client's Boundary of a project site, Step 7) Sample a Client's Boundary for a project site with the geospatial data for a vegetation attribute, Step 8) Develop a Report describing a vegetation attribute at a client's project site.

Specifically, Step 1) is developed from the legal requirements for monitoring a vegetation attribute relevant to a client. Step 1) can be updated and/or edited on the content for monitoring a vegetation attribute with updates, changes and/or revisions to existing law, for new law, for new clients and between different projects that may have different requirements for monitoring a vegetation attribute. Step 2) for developing a Science Plan is completed with input from Step 1) and is dependent on the outputs from Step 1). This means that the Science Plan can be edited, changed, updated, and/or revised with changes to the content of Step 1). Step 3) for developing a Geospatial Database is completed with input from Step 2) and is dependent on the outputs from Step 2). This means that the geospatial data used to develop a Geospatial Database in Step 3) can be edited, changed, updated, and/or revised with changes to the content of Step 2). Step 4) for developing Allometric Equations is completed with input from Step 2) and is dependent on the outputs from Step 2). This means that the methods used to develop Allometric Equations in Step 4) can be edited, changed, updated, and/or revised with changes to the content of Step 2). Step 5) for implementing Allometric Equations with remote sensing imagery is completed with input from Step 2) and is dependent on the outputs from Step 2) and Step 4). This means that the methods used to implement Allometric Equations with remote sensing imagery in Step 5) can be edited, changed, updated, and/or revised with changes to the content of Step 2) and Step 4). Step 6) for obtaining a Client's Boundary for a project site is applicable to any boundary in a geospatial data file obtained from any client. Step 7) for sampling the Client's Boundary for a project site is dependent on the geospatial data used to develop a Geospatial Database in Step 3), the outputs for implementing Allometric Equations with remote sensing imagery in Step 5) and the project site Boundary File obtained from the client in Step 6). This means that the outputs for sampling a Client's Boundary in Step 7) can be edited, changed, updated, and/or revised with changes to the content in Step 3), Step 5) and Step 6). Step 8) for reporting a vegetation attribute to a client is dependent on aforementioned steps from Step 1) to Step 7). This means that the outputs for developing a Report for a vegetation attribute at a client project site in Step 8) can be edited, changed, updated, and/or revised with changes to the content in Step 1) through Step 7).

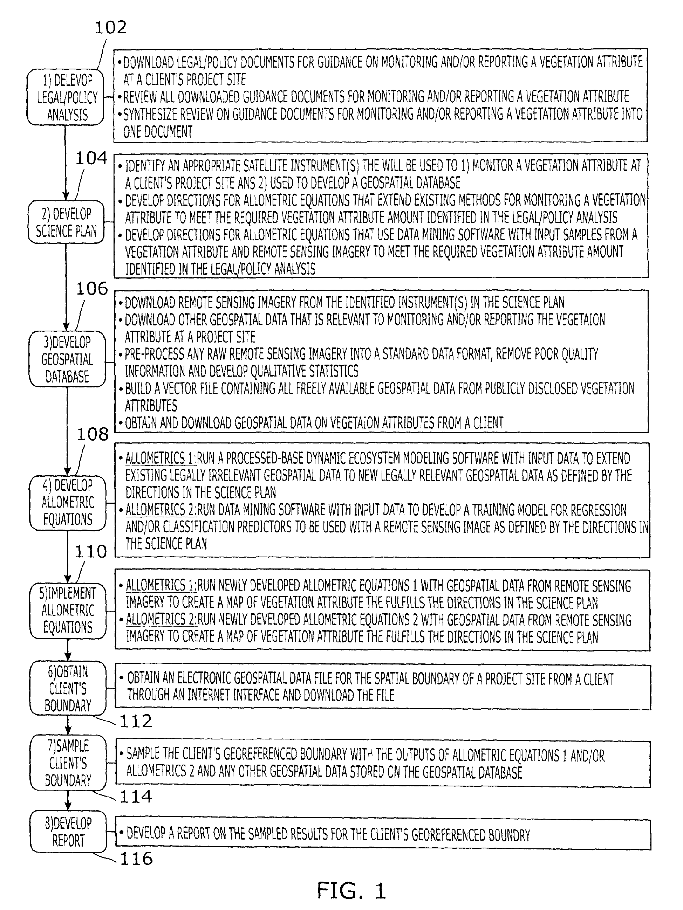

**FIG. 1** shows the embodiment of the full process in more detail. In 102, a legal/policy analysis is developed for the monitoring and/or reporting for a vegetation attribute(s) under relevant GreenHouse Gas (GHG) trading schemes and/or standards to a client. The client will intend to monetize the vegetation attribute(s) at a project site(s) under the relevant GHG trading schemes and/or standards. GHG trading schemes and/or standards are also defined as a trading mechanism that provides a legal obligation between the project developer client and other legal entities. Examples of vegetation attributes that a client can monetize for activities at a project site are terrestrial carbon sequestration and storage. Documents related to guidance on monitoring and/or reporting vegetation attributes under the trading mechanism are stored on a database.

First, a review is completed on one or more (for example, all) documents related to international law and policy guidance on monitoring and/or reporting the relevant vegetation attribute. Second, a review is completed on documents related to monitoring and/or reporting under regulated trading mechanism(s) that support the monetization for the intended vegetation attribute(s) by the client. Third, a review is completed on documents related to monitoring and/or reporting under voluntary trading mechanism(s) that support the monetization for the intended vegetation attribute(s) by the client. The term "review" in this context refers to the use of text retrieval software and/or search technology to key word retrieve/search for relevant information on monitoring and/or reporting of a vegetation attribute from one or more target policies, for example, the three types of aforementioned legal/policy information (e.g., legal/policy databases, documents, etc.) related to the target vegetation attribute. A user and/or computer implemented synthesis is completed on the retrieved information. A report can then be assembled on a word processing program that first provides a top-down synthesis on one or more (for example, all) monitoring and/or reporting requirements for the vegetation attribute in documents with the most wide ranging legal implications to the trading mechanism relevant to the client. The report next provides a bottom-up summary on how the monitoring and/or reporting for the vegetation attribute in the specific trading mechanism relevant to the client is then related back up to documents with wider-ranging legal implications. The report is either stored in electronic media on a computer hard drive and/or is printed out in hard copy with printer. The report can be updated and/or edited to include revisions and/or updates to legal / policy documents and/or amended to include new legal/policy documents relevant to monitoring and/or reporting of a vegetation attribute by a client.

In **104** from **FIG. 1,** a science plan is developed from the output of 102. The science plan first develops a strategic review of current and future planned remote sensing instrument capabilities onboard satellite missions. The strategic review of remote sensing instruments combined with the knowledge obtained from the legal/policy review from 102 is used to identify an appropriate satellite sensor to monitor the client's vegetation attribute. An intelligence assessment is developed on the current knowledge base in peer-reviewed journal articles for methods and/or techniques in monitoring a vegetation attribute with the remote sensing instrument defined in the strategic review. The science plan next uses the information developed in intelligence assessment that define the current knowledge base in public access to define directions for methods and/or techniques that will be used to meet the monitoring and/or reporting requirements required by the client that are defined in the report for the legal/policy analysis in **102.**

The new directions are developed by a user for two approaches to monitor a vegetation attribute with remote sensing imagery through the development and implementation of allometric equations (i.e., fractions, regressions and/or classifications functions). An allometric model (also referred to as an allometric equation) with respect to a vegetation attribute as an example is defined as 1) using fractions to relate a biophysical element of a vegetation attribute to another biophysical element of a vegetation attribute and/or 2) using regression and/or classification functions to relate a physical measurement of a vegetation attribute (e.g., obtained from geospatial data for a targeted vegetation attribute) to digital information measurable in pixels of a remote sensing image.

According to an aspect of an embodiment, allometric equations 1 and/or 2 will be used toextend and/or build on existing methods and/or techniques that at present do not fulfill the monitoring requirements required by a client that are defined by the legal/policy analysis in **102.** The basic concept of Allometric Equations 1 is to develop an extension of existing science to meet the monitoring and/or reporting requirements defined in **102.** Allometric Equations 1 develop fractions learned from processed-based dynamic ecosystem modeling software that are implemented as an extension to existing remote sensing-derived methods for monitoring a vegetation attribute, that with the added extensions meet the requirements for monitoring and/or reporting identified in **102.**

Allometric Equations 2 use data mining software with physical samples of vegetation attribute(s) collected from a target geographical boundary (e.g., the ground) and remote sensing imagery to develop predictive regression and/or classification functions that are implemented with a full remote sensing image(s) to meet the requirements for monitoring and/or reporting identified in **102.** In **106,** a database(s) is developed that is based on the information developed in the science plan. The directions to implement methods and/or techniques for monitoring a vegetation attribute contained in the science plan will be used in **108** and **110** to develop and implement the allometric equations with the geospatial database developed in **106.** Therefore, the science plan is used as an intellectual bridge between what is required by the client to comply with relevant monitoring and/or reporting requirements for a vegetation attribute(s) defined by **102** and the following steps in **106** through **116** that develop and implement the monitoring of the vegetation attribute and report the sampled vegetation attribute at a project site to the client. The science plan is drafted on a word processing program as a report, stored in electronic media on a computer hard drive and/or is printed out in hard copy on a printer. The science plan report can be updated and/or edited to include revisions and/or updates to the legal/policy analysis report completed in **102.**

In **106** from **FIG. 1,** a geospatial database is developed from the science plan that was developed in **104.** The geospatial database is comprised of two types of data: 1) freely available geospatial data and 2) geospatial data that is purchased on behalf the client. Geospatial data is defined as data and information that are referenced to a location on the Earth's surface. The geospatial data is in either raster and/or vector file format. Remote sensing data is in raster file format. Vegetation attribute data can be either in raster and/or vector file format. The freely available geospatial data is downloaded from internet accessible archives and/or websites. Remote sensing imagery is from either satellite-borne active and/or passive sensors. Remote sensing imagery may also be from sensor instruments onboard an unmanned aerial vehicle. Freely available remote sensing imagery is downloaded and stored on the geospatial database. Freely available climate, elevation and soil data is downloaded and stored on the geospatial database. Freely available data for a vegetation attribute is downloaded and stored on the geospatial database. Peer-reviewed literature and trading mechanism reports that disclose geospatial data for vegetation attributes are downloaded and stored on the geospatial database. Official government disclosures of geospatial data for vegetation attributes are downloaded and stored on the geospatial database. Other freely available geospatial data can be downloaded and stored on the geospatial database at the request of the client and/or with updates to the science plan. In the case of geospatial data that is remote sensing imagery, the data can be pre-processed from the raw downloaded data in a number of ways to change the file storage type, remove poor quality information, and develop qualitative statistics. In the case of geospatial data that is of a vegetation attribute, whether in a text publication and/or in a geospatial data file format, the data can be converted to a new geospatial file that combines one or more (for example, all) geospatial data for the vegetation attribute into one file. Geospatial data for a vegetation attribute is obtained from the client via the internet, downloaded and placed into a unique geospatial data file that is confidential and only for use in monitoring activities for the client. Downloaded geospatial data and other relevant information in **106** are stored in electronic media on a hard drive. The contents of the geospatial database are dependent on the strategic review, intelligence assessment and directions contained in the science plan.

In **108** from **FIG. 1,** the two types of allometeric equations are mentioned that are developed by following the directions from science plan in **104.** Allometric Equations 1 are developed to quantify a vegetation attribute(s) from a dynamic ecosystem modeling software that is stored on the hard drive and installed on a computer workstation. The dynamic ecosystem modeling software is processed with input geospatial data from the geospatial database in **106.** The fractions developed for Allometric Equations 1 partition target vegetation attribute as 100 percent to other targeted vegetation attributes that are a fraction of the 100 percent. The new fractions are the output of Allometric Equations 1 in **108.**

Allometric Equations 2 are developed with the data mining software to train a predictive model for an input physical sample of a vegetation attribute(s) with a sample of pixels from an input remote sensing image, where input samples are stored on the geospatial database. Input data from physical samples of vegetation attribute(s) have a geographical coordinate on the Earth's surface. A physical sample is defined as one or more of: 1) a geo-referenced sample for a vegetation attribute that was obtained on the ground (i.e., on the terrestrial surface of the earth); 2) any geospatial data for vegetation attribute(s) that was created from a ground sample(s) and is disclosed as a map in either a raster and/or vector file; 3) standard remote sensing products that use ground data to validate and/ verify the standard product. The point of the second two definitions for a physical sample is to data mine pre-existing geospatial data that is publically disclosed, has an associated peer-review publication and/or is an official government disclosure of a vegetation attribute, but the underlying mathematical process used to develop the publically disclosed geospatial data is not replicable by the user. Data mining this publically disclosed geospatial data is completed by extracting a mathematical function that will replicate an output with the input remote sensing data that is very similar (i.e., with a high value for the coefficient of determination) to the publically disclosed geospatial data. Input data from remote sensing imagery is a sample of digital pixel information from the remote sensing image(s) at the same geographical coordinate of each vegetation attribute. The input physical sample(s) for the vegetation attribute(s) are used as the target variable in data mining software. Target variable means the y-axis variable that is used as an actual sample to train the prediction model for input(s) variables on the x-axis in the data-mining software. The sample(s) from the remote sensing imagery are used as the input x-axis variable(s) that will be trained to predict the target y-axis variable in the data-mining software. The data mining software develops a predictive regression and/or classification training model between the geospatial samples for the target vegetation attribute and the pixel samples from the remote sensing image(s). The predictive training model is the output of Allometric Equations 2 in 108. The outputs of 108 are stored in electronic media on a hard drive and can be printed out on a printer.

In **110** from **FIG. 1,** Allometric Equations 1 and Allometric Equations 2 that were developed in **109** are implemented with remote sensing imagery and the directions contained in the science plan in **104.** Allometric Equations 1 are implemented by processing existing remote sensing-derived vegetation attributes that do not meet the monitoring requirements for the vegetation attribute identified in **102**. The implementation of Allometrics 1 for the newly developed extensions in **108** transform pre-existing information that is legally insignificant to monitoring and/or reporting requirements for a vegetation attribute indentified in 102 to new information about a vegetation attribute that is legally significant and matches the requirements for monitoring and/reporting identified in **102**. Allometics 2 are implemented by first using the data mining software to process the geospatial data contained in the full remote sensing imagery with the predictive training model developed for the vegetation attribute in 108. The data mining software scores (i.e., models) the geospatial data/information in the full the remote sensing imagery with the predictive training model. The scoring (i.e., modeling) transforms the original digital geospatial data/information contained in the remote sensing image to new information that is a prediction for the vegetation attribute. The outputs from the data mining software are then converted to a geospatial map of the vegetation attribute. The outputs of Allometric Equations 1 and Allometric Equations 2 are new geospatial data about the vegetation attribute projected as a map. The outputs of 110 are stored in electronic media on a hard drive as a database and can be printed out on a printer.

In **112** from **FIG**. **1,** an electronic geospatial data file for the geographical area and/or boundary of the client's project site is obtained through an internet interface (i.e., email and/or a website). The client's geospatial data file is downloaded from the internet interface and stored in electronic format on the hard drive.

In **114** from **FIG. 1,** geospatial data processing software is used to overlay the client's geospatial data file for a project boundary on the outputs of Allometric Equations 1 and/or Allometric Equations 2 from **110** and other geospatial data stored on the geospatial database developed in 106. The geospatial data processing software is next used to sample and/or clip the client's project boundary file for the outputs of Allometric Equations 1 and/or Allometric Equations 2 and any other geospatial data stored on the geospatial database developed in **106**. The newly sampled outputs are stored in electronic format on a hard drive.

In **116** from **FIG. 1,** a report is developed from the outputs of steps **102** to **114.** The report assembles the outputs of **102** through **114** into one document and provides a synthesis of the material in relation to monitoring the vegetation attribute at the client's project site. The report is drafted in electronic media with a word processing program. The report is stored in electronic format on a hard drive and/or printed in hard copy. The report is transmitted to the client electronically through an internet interface (i.e., email and/or a web-site).

The legal/policy analysis is completed to provide intellectual input to the science plan. If the science has no link with the legal compliance for monitoring and/or reporting under a trading mechanism, the science is merely a philosophical pursuit because it is without direction from a relevant trading mechanism, which in practice means that the science that is not informed by the law/policy may not provide a measurement of a vegetation attribute that is fungible with an emission unit under the law. Therefore, before any process related to monitoring and/or reporting can be conceived or implemented, the legal requirements defined in guidance documents must first be identified and defined.

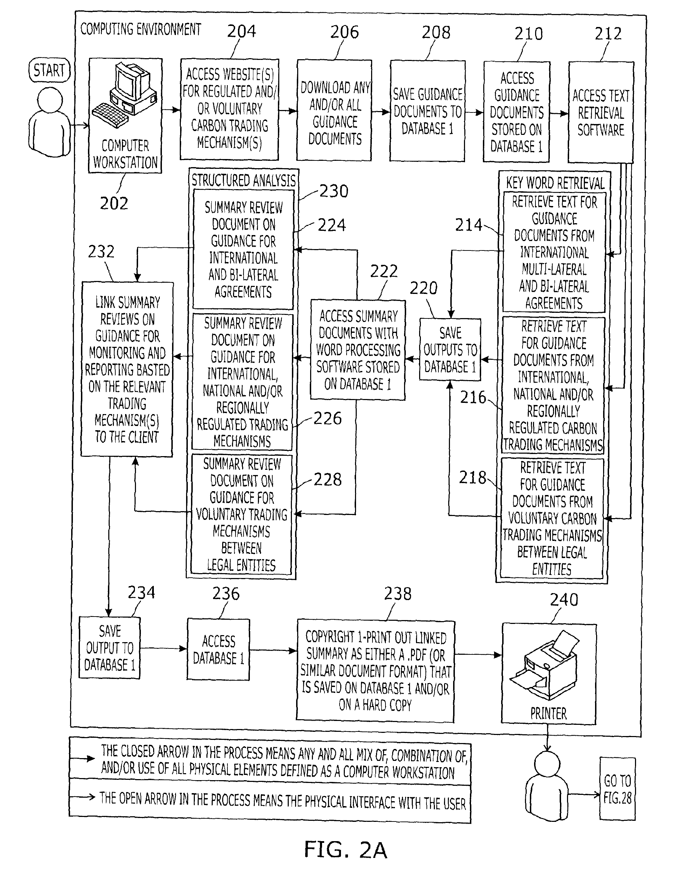

**FIG. 2A** shows the generic process used to develop a legal/policy analysis. A computer workstation **202** includes a screen display(s), processor(s), hard drive(s), a keyboard, a mouse, a router connected to the internet and other physical elements related to a computer workstation, etc. A computer workstation **202** in this context can store, retrieve, process and/or output data and/or communicate with other computers. The computer workstation is used to access the internet websites in **204** for the following: i) international multi-lateral and/or bilateral agreements, frameworks, protocols, and/or policy related the mitigation of climate change; ii) United Nations (UN), national and/or regional (i.e., sub-national and/or within a national territory) regulated trading schemes and/or standards; iii) voluntary trading schemes and/or standards. Material related to monitoring and reporting found through **204** is downloaded to the computer workstation in **206** and saved on Database 1 in **208.** Once saved, the documents related to monitoring and/or reporting are accessed in **210** by the computer workstation. Text retrieval software is accessed in **212** by the computer workstation. The retrieval software can search for targeted key words in an electronic document, pull relevant text off the document and input the text to a new document. The reason text retrieval software is used is because many of the guidance documents for mitigating climate change are in excess of hundreds of pages and automated word search retrieval software is used to expedite the time to review the documents. The retrieved "summaries" and/or "reviews" for monitoring and/or reporting the vegetation attribute(s) in guidance documents are dealt with in for the following order: i) international multi-lateral and bi-lateral agreements on mitigation of climate change and/or greenhouse gases (GHGs) in **214;** ii) international, national and regional regulated trading mechanisms related to climate change and/or GHGs in **216;** and iii) in **218,** voluntary trading mechanisms related to climate change and GHGs. Each of the three summaries/reviews on legal frameworks and trading mechanisms are saved and stored on Database 1 in **220** and accessed with the computer workstation in **222**. A structured analysis is completed in **230** by linking each of the summary documents for guidance on climate change mitigation in **224,** regulated trading mechanisms in **226** and voluntary trading mechanisms in 228. The output in **232** for the structured analysis is a summary linking all monitoring and reporting guidance in one document, which is then saved to Database 1 in **234.** The outputs of 232 are accessed in **236,** and defined as Copyright 1 in **238** that is printed out in either a Portable Document Format (i.e, .pdf and/or similar file format) digital file and/or in hard copy with a printer in **240.**

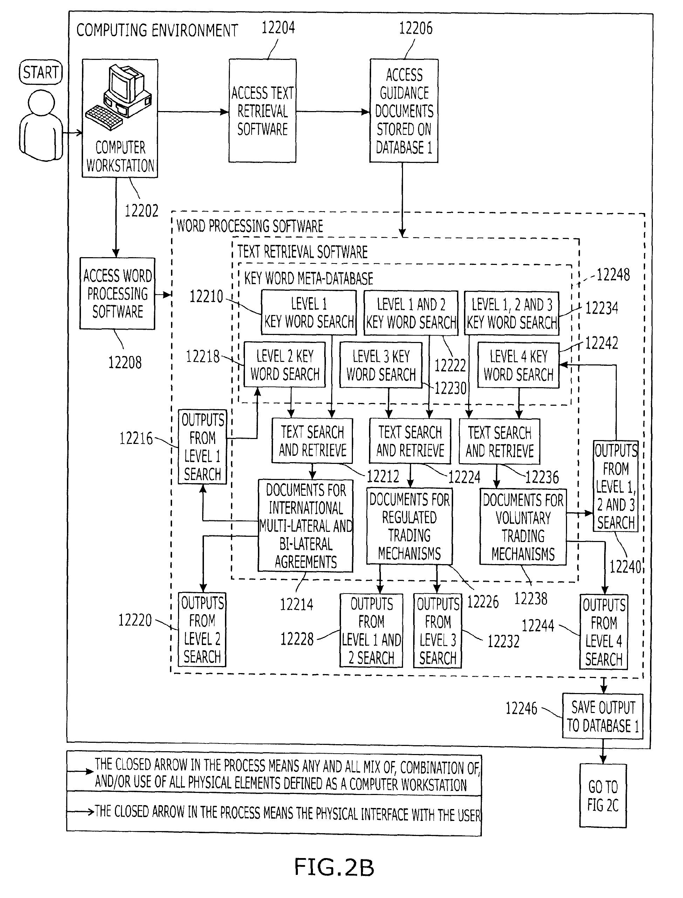

**FIG. 2B** provides a more detailed description of the process used to retrieve text for key words from **FIG. 2A.** A user accesses a computer workstation in **12202** that includes screen display(s), processor(s), hard drive(s), a keyboard, a mouse, a router connected to the internet and other physical elements related to a computer workstation, etc. A computer workstation **12202** in this context can store, retrieve, process and/or output data and/or communicate with other computers. The computer workstation is used to access text retrieval software in **12204** and word processing software in **12208**. In **12206,** the guidance documents relevant to the client from steps **214** to **218** that are stored on Database 1 are accessed. The key word search is comprised of two levels for each of the three groupings of guidance documents. The Level 1 key word search in **12210** is for words/phrases and/or sentences that relate to, specify or define a target green house gas including emission, measurement and/or monitoring of the target greenhouse gas and/or an eco-region, in the context of a target policy being searched for compliance, for example: the vegetation attribute (i.e., "biomass"); "monitor"; "report"; "verification"; "carbon"; "definition"; "remote sensing"; "model," etc. The key words are loaded into the text retrieval software by the user in **12212** and the search is completed for the documents defined as multilateral and bi-lateral agreements in **214**. The outputs are a new file that is loaded in the word processing software. The user reviews the retrieved text in **12216** and identifies new key words for a Level 2 key word search in **12218** for documents defined in **214**. Examples of Level 2 key words are the following: "dead wood", "litter", "soil", "respiration", "decomposition", "production", "stocks", "land cover", "forest land"; "grassland"; "tier"; "process"; "ecosystem"; "model"; "deforestation"; "degradation"; "devegetation"; etc. Mathematical symbols may also be retrieved in the Level 2 key word for key equations, such as: "$\Delta$"; "+"; "-"; "=" etc. The outputs of the Level 2 key word text retrieval are reviewed by the user in **12220**. In **12222,** the Level 1 and 2 key words are used by the user for key words to search and retrieve text in **12224** for the guidance documents for monitoring and/or reporting for the vegetation attribute defined from **216** as regulated trading mechanisms in **12226**. The outputs from the Level 1 and Level 2 key word search for guidance documents on regulated trading mechanisms in **12228** are reviewed by the user and used identify a Level 3 key word search in **12230**. The Level 3 key word search adds examples of the following: "IPCC"; "baseline"; "additionality"; etc. The text in the guidance documents for the regulated trading mechanisms is searched for the Level 3 key words and the outputs are reviewed in **12232**. The key words for Levels 1, 2 and 3 are used in **12234** as inputs to the text retrieval software in **12236** for target guidance documents in **12238** defined from **218** as voluntary trading mechanisms. The text outputs from the retrieval for the voluntary documents are reviewed in **12240** and used by the user to identify Level 4 key words in **12242**. Examples used as Level 4 key words are the following: "eligible"; "activity"; "AFOLU"; "project"; etc. The Level 4 key words are loaded in the text retrieval software and the search is completed for the guidance documents on the voluntary trading mechanism. The text outputs retrieved from the Level 4 key word search are reviewed by the user in **12244.** Tables and figures are also retrieved in any of the search levels when the tables and figures are described by a key word. One or more outputs (for example, all) outputs from the text retrieval are stored on Database 1 in **12246**. In **12248,** key words from the key word search are stored on a meta-database in Database 1. According to an aspect of an embodiment, keywords are specified according to legal/policy information terminology to compile policy parameters for a target greenhouse gas of the legal/policy information.

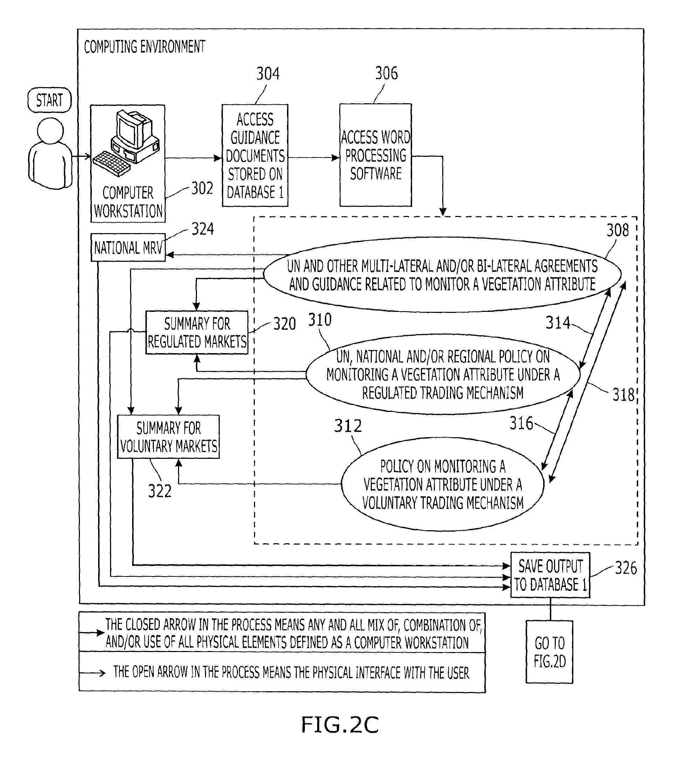

**FIG. 2C** provides a more detailed description of the structured analysis from **230** in **FIG. 2A.** The reason for this hierarchical approach to structuring the compliance documents for monitoring and/or reporting the vegetation attribute is because the vegetation attribute will be monetized by the client as a commodity in mitigating climate change. The legal/policy synthesis must show how the guidance on monitoring is fungible across the legal/policy framework landscape that deals with monitoring and/or reporting. Alternatively, if the monitoring guidance, and thus the monitoring, is not fungible, such as a disconnect between international policy guidance on climate change mitigation and a voluntary mechanism, the client's accredited voluntary offset from the vegetation attribute may not be interchangeable under international treaties and/or regulated markets with a polluter's emission footprint. Furthermore, if the methods and science used to monitor the vegetation attribute do not comply with the monitoring and/or reporting guidance, in practice this would mean that the techniques used to quantify an amount of the vegetation attribute may not create a fungible offset valuation for the client to sell to a polluter through a trading mechanism. A computer workstation in **302** includes screen display(s), processor(s), hard drive(s), a keyboard, a mouse, a router connected to the internet and other physical elements related to a computer workstation, etc. A computer workstation **302** in this context can store, retrieve, process and/or output data and/or communicate with other computers. In **304,** the computer workstation is used to access the summaries for guidance documents in steps **224-228** from **FIG. 2A** and word processing software in **306.** The structured analysis uses compliance requirements in monitoring and reporting for international agreements, frameworks, protocols and/or policy on mitigation of climate change in **308**. The user organizes the key word retrieval outputs from **12216** and **12220** into paragraphs. The text context in paragraphs are first organized by increasing page number for the same source guidance document and then organized by earliest to the most recent publication date for the source guidance document. The text content in **308** defines the science and math that is used to monitor the vegetation attribute. The content in **308** then explains how the science methods are organized and ranked into different tiers and/or approaches, and how higher ranked tiers and/or approaches supersede methods with lower rankings. Next, UN, national and/or regional regulated (i.e., legal mandated and/or required by the law) mechanisms are reviewed in **310**. The user first organizes the key word retrieval outputs from **12228** and **12232** into paragraphs. The text content in the paragraphs are first organized by increasing page number for the same source guidance document and then organized by earliest to the most recent publication date for the source guidance document. The text content in **310** defines the science and math that is used to monitor the vegetation attribute for the regulated mechanism. **310** also includes discussion of any and/or all types of crediting activities under the regulated mechanism that require the monitoring of the vegetation attribute when the clients reports project activities to the mechanism. The content in **310** also lists one or more (for example, all) references to the source documents used in **308**. Voluntary mechanisms are reviewed in **312**. The user organizes the key word retrieval outputs from **12240** and **12244** into paragraphs. The text content in the paragraphs are first organized by increasing page number for the same source guidance document and then organized by earliest to the most recent publication date for the source guidance document. The text content in **312** defines the science, math and/or the specific text that states how monitoring the vegetation attribute should be completed for the voluntary mechanism. **312** also includes discussion of any and/or all types of crediting activities under the regulated mechanism that require the monitoring of the vegetation attribute when the clients reports project activities to the mechanism. The content in **312** lists all references to the source documents used in **308** and **310**. The width of the circle in **308-312** also indicates the level of compliance application for the specified guidance document and degree in which the guidance document ranges in application to other guidance documents. For instance, compliance requirements in international agreements and policy on climate change mitigation are generally wider ranging than national and voluntary carbon markets, and thus it is more important to link compliance in both regulated and voluntary trading mechanisms back to international GHG agreements. Furthermore, regulated and voluntary guidance often cite international guidance on mitigation as the preferred method in monitoring and/or reporting. This is because international guidance, such as the IPCC's Good Practice Guidance, explains how one or more (for example, all) countries must report standardized and comparable annual emissions and removals as a signatory to the UNFCCC. Thus, project developers must comply with similar methods of monitoring and/or reporting. After each level in the hierarchy for guidance documents is organized from the text retrieval outputs, a bottom-up summary is written by the user at the end of each section for the regulated and/or voluntary mechanism(s). The bottom-up summary states how the lower tiered guidance document relates to the wider ranging guidance document. The term "relate" in this context means to establish a logical intellectual connection between two guidance documents and/or a statement about how one guidance document compares to another guidance document. In **314,** the user states how the contents for the regulated emissions trading mechanism(s) from **310** relate to the contents from **308** for international multi-lateral and/or bi-lateral agreements, frameworks, protocols, etc.,. In **316,** the user states how the contents for the voluntary trading mechanism(s) from **312** relate to the contents for the regulated trading mechanism(s) from **310**. In **318,** the user states how the contents for voluntary trading schemes from **312** relate to the contents for the international multi-lateral and bi-lateral agreements from **308**. The outputs from steps **308, 310** and **314** are combined in the word processing software as the output for the summary on the regulated trading mechanism(s) relevant to the client in **320**. The outputs from **308, 310, 312, 314, 316** and **318** are combined in the word processing software as the output the summary on the voluntary trading mechanism(s) relevant to the client in **322**. National and/or regional monitoring, reporting and verification (MRV) for a regulated cap and trade systems require an independent assessment and comparison to meet the IPCC's definitions for verification. The output from **308** is used in **324** for a summary on guidance documents for MRV of national and/or regional cap and trade systems. The decision to include any and/or all of outputs **308-316** is at the discretion of the client and dependent upon the specific trading mechanism relevant to the client as aforementioned. The outputs from **320, 322,** and/or **324** are saved to Database 1 in **326.**

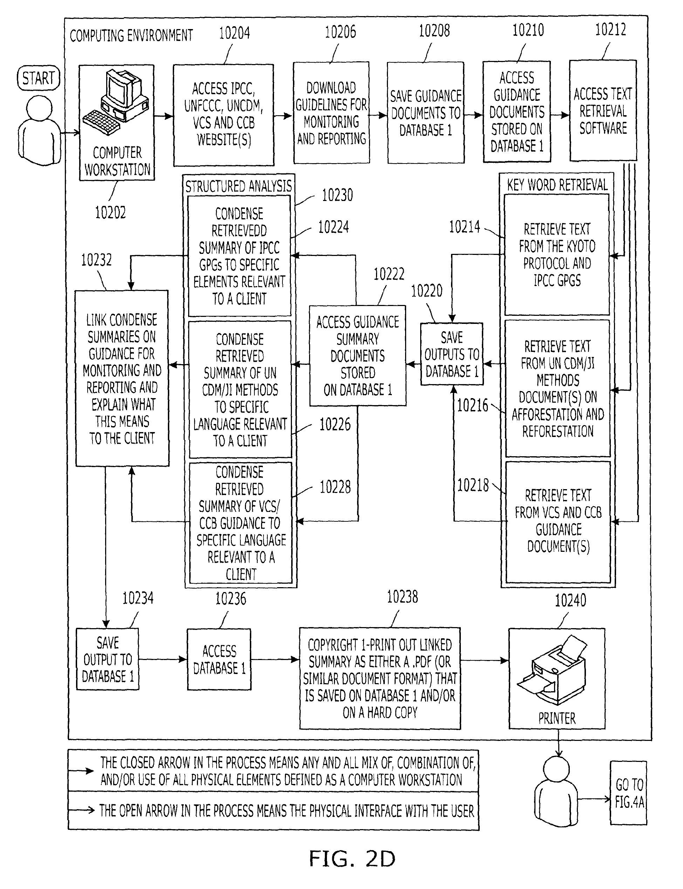

**FIG. 2D** shows an example for the generic process for a legal/policy analysis applied to guidance documents for the Voluntary Carbon Standard (VCS) and the Climate, Community and Biodiversity Alliance Standard (CCBA). The difference between FIG. 2A and 2D is that FIG. 2D shows the actual implementation of the generic process FIG. 2A. A computer workstation **10202** includes a screen display(s), processor(s), hard drive (s), a keyboard, a mouse, a router connected to the internet and other physical elements related to a computer workstation, etc. A computer workstation **10202** in this context can store, retrieve, process and/or output data and/or communicate with other computers. The computer workstation is used to access the internet websites in **10204** for the following: i) Intergovernmental Panel on Climate Change (IPCC; <URL: http://www.ipcc.ch/>); ii) United Nations (UN) Clean Development Mechanism (CDM; <URL: http://cdm.unfccc.int/index.html/>); iii) the Voluntary Carbon Standard (VCS; <URL: http://www.v-c-s-org/>) and Climate, Community and Biodiversity Alliance (CCBA; <URL: http://www.climate-standards.org/>). Material related to guidance on monitoring and reporting found through **10204** is downloaded to the computer workstation in **10206** and saved on Database 1 in **10208.** Examples of the documents stored on the Database 1 are: i) The Kyoto Protocol; ii) The Revised 1996 IPCC Guidelines (IPCC, 1997); iii) IPCC "Good Practice Guidance for Land Use, Land-Use Change and Forestry" (GPG-LULUCF; IPCC, 2003a); iv) The IPCC "Definitions and Methodological Options to Inventory Emissions from Direct Human-induced Degradation of Forests and Devegetation of Other Vegetation Types" (IPCC, 2003b); v) 2006 IPCC Guidelines for National Greenhouse Gas Inventories" (IPCC, 2006); vi) UNFCCC Clean Development Mechanism (CDM) Methodologies for Afforestation & Reforestation; vii) The Voluntary Carbon Standard's "Guidance for Agriculture, Forestry and Other Land Use Projects" (VCS, 2008); viii) "The Climate, Community and Biodiversity Standards" (CCBA, Second Edition December, 2008; ix) national GHG reporting documentation such as the annual reports from the Australia National Carbon Accounting System (NCAS); etc. Once saved, the guidance documents related to monitoring and/or reporting are then accessed in **10210** by the computer workstation. Text retrieval software is accessed in **10212** by the computer workstation. The text retrieval software is used to search the guidance documents for key words and retrieve text off the guidance related to the key words. The text retrieval is used for the following: in **10214** for the Kyoto Protocol and the IPCC Good Practice Guidelines (GPGs) for Land Use Land Use Change and Forestry (LULUCF) and Agriculture, Forestry and Other Land Use (AFOLU); in **10216** the CDM methods for afforestation and reforestation (a/r); and in **10218** the VCS guidance on AFOLU and the CCBA standard guidance document. Each of the summaries/reviews from the text retrieval are saved and stored on Database 1 in **10220** and accessed with the computer workstation in **10222.** A structured analysis is completed in **10230** by linking the Kyoto Protocol and the IPCC GPGs in **10224** and the CDM a/r methods in **10226** to the VCS guidance on AFOLU and CCBA standards guidance document (s) in **10228.** The output in **10232** for the structured analysis is a summary that is assembled from all monitoring and reporting guidance, which is then saved to Database 1 in **10234.** The outputs of **10232** are accessed in **10236,** and defined as Copyright 1 in **10238** that is printed out in either a Portable Document Format (i.e, pdf and/or similar file format) digital file and/or in hard copy with a printer in **10240.**

The following is an example of a parameterized summary as well as written summary of guidance documents for monitoring and/or reporting for a project site that a client can use for submission to a governing body and/or a trading mechanism, for example, the Voluntary Carbon Standard and the Climate, Community and Biodiversity Alliance Standard and is defined as Copyright 1 in **10238** from **FIG. 2D.** The parameters in the summary are organized by the retrieved information from policy documents into multiple tiers. The parameters are definitions to be used to in monitoring a vegetation attribute. The parameters are used to tie the compliance guidance for monitoring target vegetation attribute to the methodology used to monitoring the target vegetation with remote sensing imagery that is developed in the science plan. The parameters are organized into different tiers of policy documents where each tier has a legal priority over another tier. The different tiers are linked according to legal priority. The linking is completed by pulling text for policy documents with less legal priority with key words from documents with greater legal priority. The text that is pulled from the documents with less legal priority explicitly states how the document with less legal priority is related to policy documents with greater legal priority. The linking can be user assisted and/or automated. In this example, 28 legal/policy information items are complied as parameters including metadata that describe the parameters and/or the legal/policy information and which can be stored and managed via a data structure (e.g., a database) representing review of the target legal/policy information.

Parameter 1. Target greenhouse gas to be monitored under an international multi-lateral policy agreement, for example, $CO_2$, $CH_4$, $N_2O$, HFCs, PFCs and SHF and the following key words: "emission", "removals" "monitoring" and "reporting". Parameter 1 is an example of one of the retrieved outputs in 10214 from FIG. 2D that are condensed in 10224 from FIG. 2D. For example, the Kyoto Protocol required Annex 1 parties to the Convention to reduce emissions of Green House Gases (GHGs, which Annex A defines as $CO_2$, $CH_4$, $N_2O$, HFCs, PFCs and SHF) to the percentage of 1990 emissions set out in Annex B to the Protocol. The Protocol assigned each Annex 1 party a maximum amount of emissions ("the assigned amount") which it might emit during the first commitment period (2008 to 2012). The Protocol stated that parties might offset removals of GHGs that are a result from Land-Use Change and Forestry ("LULUCF") against emissions from LULUCF sources. The Protocol mentioned that the monitoring and reporting of changes in carbon stocks for emissions and removals should be in accordance with the IPCC's Guidance on Good Practice for LULUCF in Decision 15 of the Conference of Parties ("COP/MOP") 1 on the preparation of information required under Article 7 of the Protocol.

Parameter 2. Method(s) used to describe requirements to calculate monitoring of GHG activities derived from retrieved text from the earliest dated and accessible IPCC Guidance on Good Practice (GPG) document for LULUCF referenced in Parameter 1 for an international multi-lateral policy agreement. Thus, input from Parameter 1 is used to identify the document, if any, to use to develop Parameter 2. The text retrieval was derived from the chapter titles of the Revised 1996 IPCC Guidelines (IPCC, 1997) and the following key words: "biomass" and "increments". Parameter 2 is an example of one of the retrieved outputs in 10214 from FIG. 2D that are condensed in 10224 from FIG. 2D. For example, the Revised 1996 IPCC Guidelines (IPCC, 1997) was the first document that instructed countries on how to establish monitoring activities in the following sectors: Energy, Industrial processes, Solvent and other product use, Agriculture, Land use change and forestry, and Waste. Equation 1 and Table 5.2 of the Reference Manual (IPCC, 1997, p. 5.19-5.20) specifically used the biomass growth increments to calculate annual above-ground biomass for reporting values. The Good Practice Guidance and Uncertainty Management in National Greenhouse Gas Inventories (IPCC, 2000) provided no supplementary guidance for monitoring the LULUCF sector developed in the Revised 1996 IPCC Guidelines.

Parameter 3. Method(s) used to describe requirements to calculate monitoring of GHG activities derived from retrieved text from the next earliest dated IPCC Guidance on Good Practice (GPG) document for LULUCF referenced in Parameter 1 for an international multi-lateral policy agreement. Thus, input from Parameter 1 is used to identify the document to develop Parameter 3. Parameter 3 is an example of one of the retrieved outputs in 10214 from FIG. 2D that are condensed in 10224 from FIG. 2D. The text retrieval as derived from text of "Good Practice Guidance for Land Use, Land-Use Change and Forestry" (GPG-LULUCF; IPCC 2003a) and the following key words: "biomass", "increments", "monitoring", "land use", "carbon", "verification" and "remote sensing". For example, the IPCC "Good Practice Guidance for Land Use, Land-Use Change and Forestry" (GPG-LULUCF; IPCC, 2003a), made two primary advances in defining and monitoring the LULUCF sector. First, GPG-LULUCF defined six land use categories necessary for monitoring the LULUCF sector. These categories were: 1) forested land, 2) cropland, 3) grassland, 4) wetland, 5) settled land and 6) other land. The second advance was that it defined five carbon pools necessary for monitoring each of the six land use categories. The five pools were: 1) above-ground biomass, 2) below-ground biomass, 3) dead wood, 4) litter and 5) soil organic matter. Chapter 5.7 of GPG-LULUCF discussed approaches to the verification of GHG inventories. Chapter 5.7 showed that remote sensing is applicable to monitoring the six land use categories and above-ground biomass. Chapter 5.7 also discussed ecosystem modeling approaches that were suitable for verification of the five carbon pools and referred to FOREST-BGC (Waring and Running, 1998) and Biome-BGC (Running and Coughlan, 1988; Running and Hunt, 1993; Running, 1994) as the only "well known examples" of ecosystem models that could be used in verification.

Parameters 4-11. Method(s) used to describe requirements to calculate monitoring of GHG activities derived from retrieved text from the recent dated IPCC Guidance on Good Practice (GPG) document for LULUCF referenced in Parameter 1 for an international multi-lateral policy agreement. Thus, input from Parameter 1 is used to identify the document to use to develop Parameter 4-11. Parameters 4-11 are examples of the retrieved outputs in 10214 from FIG. 2D that are condensed in 10224 from FIG. 2D. The text retrieval was derived from text of Volume 4 of the "2006 IPCC Guidelines for National Greenhouse Gas Inventories" (IPCC, 2006) and the following initial key words: "biomass", "increments", "monitoring", "emission", "removals", "land use", "carbon", "verification" and "remote sensing". Parameters 5-12 were then retrieved with the following key words: "dead wood", "litter", "soil", "respiration", "decomposition", "production", "stocks", "land cover", "forest land"; "grassland"; "tier"; "process"; "ecosystem"; "model"; "Δ"; "+"; "-"; "=" etc.

Parameter 4. Definitions for target greenhouse gas(s) in an eco-region. For example, the Volume 4 of the "2006 IPCC Guidelines for National Greenhouse Gas Inventories" (to be termed "GPG-2006"; IPCC, 2006) dealt with the Land Use, Land Use Change and Forestry (LULUCF), which was redefined by Volume 4 as the Agriculture, Forestry and Other Land Use (AFOLU) sector. GPG-2006 incorporated clear definitions of the carbon cycle processes in the scientific background. These definitions were (IPCC, 2006, Volume 4, Chapter 1, p. 1.6-1.8): "Gross Primary Production (GPP) is the uptake of $CO_2$ through photosynthesis;" "About half of the Gross Primary Production is respired by plants, and returned to the atmosphere, with the remainder constituting Net Primary Production (NPP), which is the total production of biomass and dead organic matter in a year;" "Net Primary Production minus losses from heterotrophic respiration (decomposition of organic matter in litter, dead wood and soils) is equal to the net carbon stock change in an ecosystem and, in the absence of disturbance losses, is referred to as Net Ecosystem Production (NEP);" "Net ecosystem production minus additional carbon losses from disturbance (e.g., fire), harvesting and land clearing during land-use change, is often referred to as Net Biome Production (NBP)." GPG-2006 stated that the "carbon stock change that is reported in national greenhouse gas inventories for land-use categories is equal to net biome production" (IPCC, 2006, Volume 4, Chapter 1, p.1.7).

Parameter 5. Classification of uses of an area of land within an eco-region. For example, the generic equations to be used for annual monitoring and reporting of the Agriculture, Forestry and Other Land Use (AFOLU) sector under the IPCC Good Practice Guidance (IPCC, 2006, Volume 4, Chapter 2, Equations 2.1 and 2.3) are:

For example, Six land use classes:

$$\Delta C_{AFOLU} = \Delta C_{FL} + \Delta C_{CL} + \Delta C_{GL} + \Delta C_{WL} + \Delta C_{SL} + \Delta C_{OL} \quad \text{Eq. 1}$$

Where: $\Delta C$ is the carbon stock change amount; indices denote the following land-use categories: *AFOLU is* Agriculture, Forestry and Other Land Use; *FL* is Forest Land; *CL* is Cropland; *GL* is Grassland; *WL* is Wetlands; SL is Settlements; and *OL* is Other Land.

Parameter 6. Target greenhouse gas pool monitored in each class of area use within an eco-region. For example, Five carbon pools are monitored for each land use class:

$$\Delta C_{LUi} = \Delta C_{AB} + \Delta C_{BB} + \Delta C_{DW} + \Delta C_{LI} + \Delta C_{SO} \quad \text{Eq. 2}$$

Where: $\Delta C_{LUi}$ is the carbon stock changes for a stratum of land-use category; subscripts denote the following carbon pools: *AB* is above-ground biomass; BB is below-ground biomass; *DW* is deadwood; *LI* is litter; and SO is soil organic matter. $\Delta C_{HWP}$ is the carbon stock change for Harvested Wood Products (HWP). HWP is included in the IPCC GPG 2006 for AFOLU, but it is dealt with separately in GPG-2006 when calculating the generic equations for carbon pools in Equation 2.

Parameter 7. Categories of area uses within an eco-region. For example, the IPCC Good Practice Guidance defined AFOLU land use categories required for reporting in the AFOLU sector as the following (IPCC, 2006, Volume 4, Chapter 3, p. 3.6-3.7):

Forested land-This category includes all land with woody vegetation consistent with thresholds used to define Forest Land in the national greenhouse gas inventory. It also includes systems with a vegetation structure that currently fall below, but *in situ* could potentially reach the threshold values used by a country to define the Forest Land category.

Cropland-This category includes cropped land, including rice fields, and agro-forestry systems where the vegetation structure falls below the thresholds used for the Forest Land category.

Grassland-This category includes rangelands and pasture land that are not considered Cropland. It also includes systems with woody vegetation and other non-grass vegetation such as herbs and brushes that fall below the threshold values used in the Forest Land category. The category also includes all grassland from wild lands to recreational areas as well as agricultural and silvi-pastural systems, consistent with national definitions.

Wetlands-This category includes areas of peat extraction and land that is covered or saturated by water for all or part of the year (e.g., peatlands) and that does not fall into the Forest Land, Cropland, Grassland or Settlements categories. It includes reservoirs as a managed sub-division and natural rivers and lakes as unmanaged sub-divisions.

Settlements-This category includes all developed land, including transportation infrastructure and human settlements of any size, unless they are already included under other categories. This should be consistent with national definitions.

Other Land-This category includes bare soil, rock, ice, and all land areas that do not fall into any of the other five categories. It allows the total of identified land areas to match the national area, where data are available. If data are available, countries are encouraged to classify unmanaged lands by the above land-use categories (e.g., into Unmanaged Forest Land, Unmanaged Grassland, and Unmanaged Wetlands). This will improve transparency and enhance the ability to track land-use conversions from specific types of unmanaged lands into the categories above.

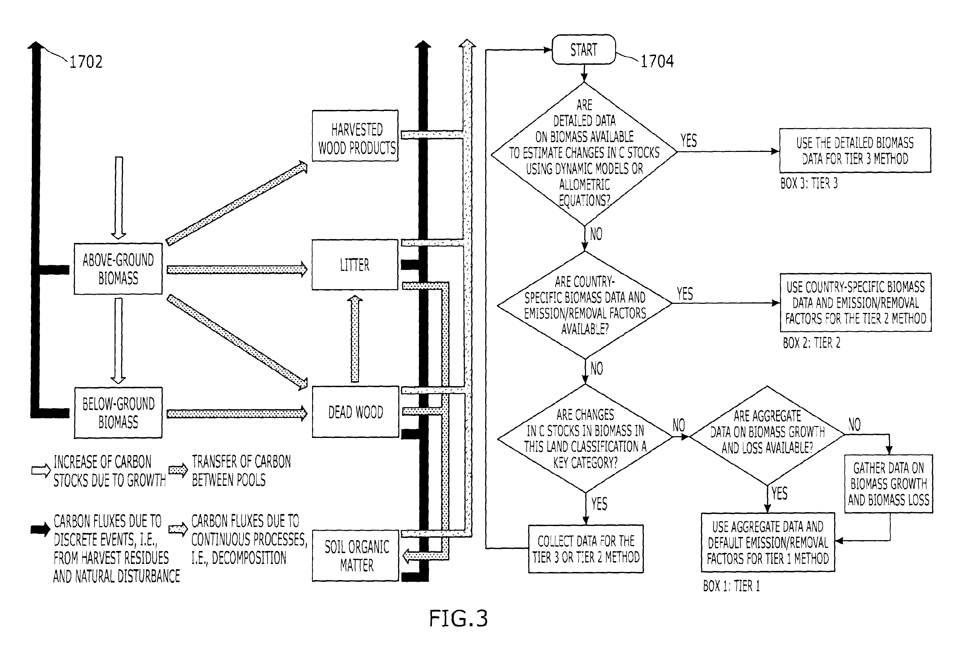

Parameter 8. Target greenhouse gas cycle definition. For example, FIG. 3 shows in 1702 a generalized flowchart of the carbon cycle defined by the IPCC (retrieved from IPCC, 2006, Volume 4, Chapter 2, p. 2.8) and in **1704** (see FIG. 3) shows the generic decision tree for identification of appropriate tier to estimate changes in different carbon pools in each land use category (retrieved from IPCC, 2006, Volume 2, Chapter 2, p. 2.14). The generalized IPCC flowchart of the carbon cycle (in **1702,** see **FIG**. **3)** shows all five pools and associated annual fluxes including inputs to and outputs from the system, as well as all possible transfers between the pools (IPCC, 2006, Vol. 4, Ch 2, p. 2.8). Overall, carbon stock changes within each AFOLU land use stratum are estimated by adding up changes in all carbon pools by AFOLU Generic Equation 2.3 (IPCC, 2006, Volume 4, Chapter 2). The carbon cycle includes changes in carbon stocks due to both continuous processes (i.e., growth, decay) and discrete events (i.e., disturbances like harvest, fire, insect outbreaks, land-use change and other events). Continuous processes can affect carbon stocks in all areas in each year, while discrete events (i.e., disturbances) cause emissions and redistribute ecosystem carbon in specific areas (i.e., where the disturbance occurs) and in the year of the event.

Parameter 9. Definitions of target greenhouse gas pools. For example, the IPCC definition for each carbon pool is as follows (IPCC, 2006, Volume 4, Chapter 1, p. 1.9):

Biomass:

Above-ground biomass-All biomass of living vegetation, both woody and herbaceous, above the soil including stems, stumps, branches, bark, seeds and foliage.

Below-ground biomass-All biomass of live roots. Fine roots of less than 2mm diameter are often excluded because these often cannot be distinguished empirically from soil organic matter or litter.

Dead Organic Matter (DOM): Dead Wood-Includes all non-living woody biomass not contained in the litter, either standing, lying on the ground, or in the soil.

Dead wood includes wood lying on the surface, dead roots, and stumps, larger than or equal to 10 cm in diameter (or the diameter specified by the country).

Litter-Includes all non-living biomass with a size greater than the limit for soil organic matter and less than the minimum diameter chosen for dead wood, lying dead, in various states of decomposition above or within the mineral or organic soil. This includes the litter layer as usually defined in soil typologies. Live fine roots above the mineral or organic soil are included in litter where they cannot be distinguished from empirically.

Soils: Soil Organic Matter-Includes organic carbon in mineral soils to a specified depth chosen by the country and applied consistently through the time series. Live and dead fine roots and DOM within the soil, that are less than the minimum diameter limit (suggested 2 mm) for roots and DOM, are included with soil organic matter where they cannot be distinguished from it empirically.

Parameter 10. Methods used for estimating changes in a target greenhouse gas. For example, the IPCC GPG-2006 developed a decision tree for identification of appropriate tier to estimate changes in carbon stocks. 1704 (see **FIG. 3)** is the three-tiered system developed for decision-making. The decision tree begins by asking whether data on biomass is available to estimate changes in carbon stocks using dynamic models or Allometric Equations? If the answer is "yes" the decision tree indicates that the use dynamic models or Allometric Equations are the preferred methodology quantifying detailed biomass data, which is entitled a "Tier 3" approach. The Tier 2 approach is the use of country-specific biomass data and emissions / removal factors. The bottomed tiered approach, Tier 1, used aggregate data and default emission / removal factors for biomass found in the IPCC GPG-2006. The Tier 3 approach, therefore, supersedes Tier 1 and Tier 2 approaches if a Tier 3 approach is available. Further direction on development of Tier 3 approaches is found at the end of IPCC GPG-2006, Volume 4, Chapter 2, pages 2.50-2.53.

Parameter 11. For example, the IPCC 2006-GPG (Chapter 4, Section 4.2, p.10-27) dealt with the estimation of forest carbon pools and described how this should be completed for each of the three tiered approaches for GHG reporting in AFOLU (see Section 4.4.5 of this document). GPG 2006 (Vol. 4, Ch. 4, p.4.34, Box 4.3) identified The Australia National Carbon Accounting System (NCAS) as an example of a good practice approach in AFOLU sector monitoring.

Method(s) used to describe requirements to calculate monitoring of GHG activities derived from retrieved text from the The Australia National Carbon Accounting System (NCAS) in the year 2006 (<URL: http://www.climatechange.gov.au>) for LULUCF referenced in Parameter 11 from GPG-2006. Thus, information from Parameter 12 is used to identify the document to use to develop Parameter 12. Parameter 12 is an example of one of the retrieved outputs in **10214** from **FIG. 2D** that are condensed in **10224** from **FIG. 2D**. Since NCAS is also an example of a national monitoring system, a more in-depth review of parameters from NCAS could be developed as an example of a national monitoring system in **216** and of **FIG. 2A.** The following key words were used in the retrieval search: "biomass", "increments", "remote sensing", "carbon", and "model.

Parameter 12. For example, the NCAS LULUCF/AFOLU sector model, *CamFor* integrated the *Roth-C* soil carbon model (Jenkinson et. al., 1987, Jenkinson et. al., 1991), the 3-PG forest growth model (Landsberg and Waring 1997) and the *GENDEC* litter decomposition model (Moorhead and Reynolds 1991; Moorhead et. al., 1999). *3-PG* forest growth model was used with NOAA-AVHRR remote sensing data and climate data to model NPP.

At this point, a paragraph is constructed and/or assembled in the word processing software explaining what the retrieved text means in simple language interpretable to anyone skilled in the art. This is an example of a linked summary the documents in 10232 for documents in 10224 from **FIG. 2D.** For example, this means that Net Biome Production (NBP) is the sum of all five carbon pools within all six land use categories (IPCC, 2006, Volume 4, Chapter 2, Equations 2.1 and 2.3). Thus, remote sensing-derived measurements of NPP can be used to monitor biomass and incorporated with ecosystem modeling procedures, such as with the process-based dynamic ecosystem model Biome-BGC, to monitor, report and/or verify all five carbon pools defined as Net Biome Production. Remote sensing of Gross Primary Production and Net Primary Production automatically accounts HWP removals lost throughout a year because the remote sensing images of vegetation growth are at increments of every 8 to 10 days. In other words, remote sensing-derived GPP and NPP is vegetation growth after disturbance and Biome-BGC derived GPP and NPP is vegetation growth before disturbance. Furthermore, IPCC GPG-2006 separates HWP from NBP and also deals with reporting HWP separately. Heterotrophic respiration is the annual carbon flux amount loss to the atmosphere due to decomposition. The IPCC GPGs (2006) define Net Ecosystem Production (NEP) as the numerical difference between heterotrophic respiration and Net Primary Production (NPP). Heterotrophic respiration is not formally accounted for in GPG-2006 other than the reference to NEP and the use of average decomposition rates in relevant carbon pools (i.e., litter and soil out flux). *CamFor* is a Tier 3 approach for national GHG reporting in AFOLU. The *CamFor* model complied with the IPCC Revised 1996 Guidelines for National Greenhouse Gas Inventories (IPCC, 1997) and the IPCC Good Practice Guidance for Land Use, Land Use Change and Forestry (IPCCa, 2003) while taking into account Australian conditions. The *CamFor* model also benefited from Australia's uniqueness of having Landsat and NOAAAVHRR satellite sensor receiving stations. This allowed NCAS to develop a long-term Landsat and NOAAAVHRR imagery archive. These long-term imagery archives for Australia make application of the full *CamFor* model with Landsat and NOAAAVHRR imagery nearly impossible for countries that do not have such recieving stations. Finally, the NCAS methods have not been updated to comply with the IPCC GPG-2006 for AFOLU, as is stated on the NCAS website.

Parameters 13-16. Degradation specification or identification for/within the target eco-region for monitoring GHG activities within the target eco-region. For example, method(s) used to describe requirements to calculate monitoring of GHG activities derived from retrieved text from the recent dated IPCC Guidance on Good Practice (GPG) document for LULUCF referenced in Parameter 1 for an international multi-lateral policy agreement. Thus, input from Parameter 1 is used to identify the document to use to develop Parameters 13-16. Parameters 13-16 are an example of the retrieved outputs in **10214** from **FIG. 2D** that are condensed in **10224** from **FIG. 2D.** The text retrieval was derived from text of the IPCC "Definitions and Methodological Options to Inventory Emissions from Direct Human-induced Degradation of Forests and Devegetation of Other Vegetation Types" (IPCC, 2003b).. This document is not in order by publication date with the other documents due to the specific nature of the document. This document was consistent with the monitoring procedures set forth by GPG-LULUCF (IPCC, 2003a), which was later superseded by GPG-2006. The following initial key words: "biomass", "monitoring", "emission", "removals", "land use", "carbon", "verification", "remote sensing", "definition", "dead wood", "litter", "soil", "respiration", "decomposition", "production", "stocks", "land cover", "forest land"; "grassland"; "tier"; "process"; "ecosystem"; "model"; "$\Delta$"; "+"; "-"; "=" etc.

Parameter 13. General definition of degradation. For example, the IPCC "Definitions and Methodological Options to Inventory Emissions from Direct Human-induced Degradation of Forests and Devegetation of Other Vegetation Types" (IPCC, 2003b); to be referred to hereafter as the "IPCC Degradation Report"). The IPCC Degradation Report specifically defined the term "degradation" to be associated with forested land and "devegetation" specifically associated with cropland, grassland and wetland land use categories.

Parameter 14. Definition of forest degradation. For example, the IPCC Degradation Report reviewed 50 definitions for forest degradation, but "none of [them was] found to be suitable for operational use in the context of the Kyoto Protocol (IPCC, 2003b, p. 11)". The IPCC Degradation Report defined forest degradation as: "A direct human-induced long-term loss (persisting for $X$ years or more) of at least $Y\%$ of forest carbon stocks [and forest values] since time $T$ and not qualifying as deforestation or an elected activity under Article 3.4 of the Kyoto Protocol (IPCC, 2003b, p. 16)."

Parameter 15. Definition of forest degradation. For example, the IPCC Degradation Report mentioned that the land use area, time and carbon loss thresholds were "unspecified" because of operational differences between countries. In terms of monitoring forest degradation with remote sensing, the IPCC degradation report stated that "remote sensing remains one of the most efficient means of detecting activities across broad spatial extents that impact forests" (IPCC, 2003b, p. 19). The IPCC Degradation Report linked forest degradation to the carbon pools discussed in GPG-LULUCF (IPCC, 2003a), and specifically stated that "defining forest degradation based on changes in biomass may be the most straightforward to implement and can be directly related to estimates of all relevant forest carbon pools (IPCC, 2003b, p. 16)."

Parameter 16. Definition of devegetation. For example, the IPCC Degradation Report stated that they found "very few published definitions of devegetation and they are essentially the corollaries of deforestation" (IPCC, 2003b, p. 17) in other land use types. The IPCC Degradation Report stated, "The Marrakesh Accords do not define devegetation. The authors state that the Accords do define revegetation as 'a direct human-induced activity to increase carbon stocks on sites through the establishment of vegetation that covers a minimum of 0.05 hectares and does not meet the definitions of afforestation and reforestation...' (IPCC, 2003b, p. 17)." The IPCC Degradation Report stated that the following definition met the operational necessities for monitoring devegetation of other vegetation types in the context of the Kyoto Protocol:

"A direct human-induced long-term loss (persisting for $X$ years or more) of at least $Y\%$ of vegetation [characterized by cover / volume / carbon stocks] since time Ton vegetation types other than forest and not subject to an elected activity under Article 3.4 of the Kyoto Protocol. Vegetation types consist of a minimum area of land of Zhectares with foliar cover of $W\%$ (IPCC, 2003b, p. 19)."

At this point, a paragraph is constructed and/or assembled in the word processing software explaining what the retrieved text means in simple language interpretable to anyone skilled in the art. This is an example of a linked summary the documents in **10232** for documents in **10224** from **FIG. 2D.** For forest degradation, this means that remote sensing-derived measurements of standing biomass stored in wood and vegetation growth values of Net Primary Production and Net Biome Production are applicable to the monitoring of forest degradation of biomass in the context of The Degradation Report and GPG-2006. With regard to monitoring devegetation of other vegetation types, The IPCC Degradation Report implied that remote sensing had the same monitoring capabilities as with forest degradation. This means that remote sensing-derived measurements for vegetation growth of Net Primary Production and Net Biome Production can be used to monitor biomass devegetation of other vegetation types in the context of non-forested land under the IPCC Degradation Report and GPG-2006.

Parameters 17-23. Voluntary greenhouse gas trading mechanisms. Method(s) used to describe requirements to calculate monitoring of GHG activities derived from retrieved text from the Voluntary Carbon Standard's "Guidance for Agriculture, Forestry and Other Land Use Projects" (VCS, 2008) document is used as an example of guidance from a voluntary mechanism. Parameters 17-23 are an example of the retrieved outputs in **10218** from **FIG. 2D** that are condensed in **10228** from **FIG. 2D.** The VCS document is relevant to parameters 17-23, except for parameter 19. Parameter 19 is for the UN CDM Approved Consolidated Methodologies for Afforestation & Reforestation and was informed of for use by the Voluntary Carbon Standard guidance. Parameter 19 is an example of the retrieved outputs in **10216** from **FIG. 2D** that are condensed in **10226** from **FIG. 2D.** The CDM Guidance is an example of a regulated mechanism. The reason this is a brief review of the CDM methods is because the intent of this section is an example of the VCS guidance, which in turn references the CDM methods. The text retrieval for both VCS and CDM documents used the following as examples of key words: "IPCC", "GPG", "biomass", "monitoring", "land use", "carbon", "verification", "remote sensing", "definition", "dead wood", "litter", "soil", "respiration", "decomposition", "production", "stocks", "land cover", "forest land"; "grassland"; "tier"; "process"; "ecosystem"; "model"; "deforestation, "degradation" and the following symbols "Δ"; "+"; "-"; "=" etc.

Parameter 17. General definition for monitoring requirements of offset activities under the VCS mechanism. For example, the Voluntary Carbon Standard's "Guidance for Agriculture, Forestry and Other Land Use Projects" (VCS, 2008; and to be referred to hereafter as "The VCS AFOLU Document") provided guidance for Voluntary Carbon Units (VCUs) in the AFOLU sector. The general VCS guidance on estimating GHG removals stated on page 28: "VCS AFOLU methodologies provide guidance for estimating net GHG benefits from project activities against the baseline scenario following the methodologies outlined in the IPCC Guidelines 2006 for AFOLU." The VCS Document also stated the following for monitoring net emissions reductions and GHG removals for all AFOLU projects: "To be eligible under the VCS, AFOLU projects must have robust and credible monitoring protocols as defined in the approved methodologies. Monitoring and ex-post quantification of the project scenario (including off-site climate impacts) must follow the applicable guidance available in approved A/R CDM methodologies and/or IPCC documents (VCS, 2008, p. 31)."

The VCS AFOLU project activities were grouped into four categories. The following subsections are a brief review of standards required for monitoring and verification of the four VCS AFOLU categories.

Parameter 18. Definition of vegetation attribute monitoring requirements for an offset project activity related to Afforestation, Reforestation and Revegetation (ARR) under the VCS mechanism. For example, the VCS AFOLU Document stated that "eligible activities in the Afforestation, Reforestation and Revegetation (ARR) project category consist of establishing, increasing or restoring vegetative cover through the planting, sowing or human-assisted natural regeneration of woody vegetation to increase carbon stocks in woody biomass and, in certain cases, soils. Examples of envisaged VCS ARR activities included: reforestation of forest reserves; reforestation or revegetation of protected areas and other high priority sites; reforestation or revegetation of degraded lands; and rotation forestry with long harvesting cycles (VCS, 2008, p.9)." Eligible carbon pools for VCS credits are above-ground biomass, below-ground biomass, dead wood, litter, soil organic matter and harvested wood products. Page 29 of The VCS AFOLU Document directed carbon pool monitoring for ARR to follow "the guidance provided by the IPCC or approved Afforestation and Reforestation (A/R) CDM methodologies." Furthermore, on page 6, The VCS AFOLU Document stated that Validators & Verifiers are considered "accredited" [for all four VCS ALOLU sectors] under the VCS if they are accredited for scope 14 (Afforestation & Reforestation) of the UNFCCC Clean Development Mechanism (CDM). Two meanings for compliance are interpreted from this statement: 1) that VCS ARR is linked to CDM compliance on carbon pool monitoring and verification for Afforestation & Reforestation and 2) rules established for CDM compliance on carbon pool monitoring and verification supersede those of the VCS, because accreditation as a project verifier under CDM methods preempts a verifier gaining accreditation under the VCS.

Parameter 19. Definition of vegetation attribute monitoring requirements for an offset project activity related to Afforestation and Reforestation under the CDM mechanism. For example, the Approved Consolidated Methodologies for Afforestation & Reforestation" provided guidance for carbon pool monitoring and verification under the CDM. On page 3, the document states that above-ground and below-ground biomass are required for monitoring and verification. Dead wood, litter and soil organic matter are required if the data is available, or alternatively, are not required if the data is not available.

Parameter 20. Definition of vegetation attribute monitoring requirements for an offset project activity related to Agricultural Land Management (ALM) under the VCS mechanism. For example, the VCS AFOLU Document stated that "land use and management activities that have been demonstrated to reduce net greenhouse gas (GHG) emissions on cropland and grassland (see IPCC 2006 GL for AFOLU) by increasing carbon (C) stocks (in soils and woody biomass) and/or decreasing $CO_2$, $N_2O$ and/or $CH_4$ emissions from soils are eligible for certification under the VCS as Agricultural Land Management (ALM) projects (VCS, 2008, p. 10)." The VCS AFOLU Document mentioned three categories for ALM activities are: (A) improved cropland management; (B) improved grassland management (C) cropland and grassland land-use conversions. The VCS AFOLU Document also stated on page 18 that "the primary carbon pool of concern for ALM is soil carbon. Since the definition of ALM included reference to reporting GHG emissions from IPCC GPG-2006, it is straightforward that monitoring should be in line with the IPCC GPG-2006 for AFOLU. Page 30 of The VCS Document linked monitoring of ALM to the IPCC GPG-2006 in AFOLU and reviewed the 3 Tiered approach found in GPG-2006 (see Section 4.4.5 of this document).

Parameter 21. Definition of vegetation attribute monitoring requirements for an offset project activity related to Improved Forest Management (IFM) under the VCS mechanism. For example, VCS AFOLU Document stated that Improved Forest Management (IMF) activities are implemented on forest lands managed for wood products. These areas are designated, sanctioned or approved for such activities (e.g., such as logging concessions or plantations) by the national or local regulatory bodies and are eligible for crediting under the VCS IFM category. IFM activities are intended to increase carbon stocks and reduce GHGs. IFM activities included reduced impact logging, conversion of logged forests to protected areas, extending the rotation age of evenly managed forests and conversion of low-productive forests to high productive forests. All carbon pools are required for monitoring and verification of IFM under the VCS (VCS, 2008, p.18). The VCS AFOLU Document also stated on page 30 that: "To date, no approved methodologies exist for IFM project activities under the UNFCCC. Guidance for estimating carbon stocks and changes in them is provided in the IPCC GPG-2006 (Chapter 4, Section 4.2, p.10-27)."

Parameter 22. Definition of vegetation attribute monitoring requirements for an offset project activity related to Reduced Emissions for Deforestation and forest Degradation (REDD) under the VCS mechanism. For example, the VCS AFOLU Document stated that: "activities that reduce the conversion of native or natural forests to non-forest land, which are often coupled with activities that reduce forest degradation and enhance carbon stocks of degraded and/ or secondary forests that would be deforested in absence of the Reduced Emission from Deforestation and Forest Degradation (REDD) project activity, are creditable as REDD section under the VCS (VCS, 2008, p. 13)." Deforestation can be planned (designated and sanctioned) or unplanned (unsanctioned) activities within a country. The VCS AFOLU Document defined planned deforestation activities as the following: "national resettlement programs from non-forested to forested regions; national land plans to reduce the forest estate and convert it to industrial-scale production of commodities such as soybeans, pulpwood, and oil palm; plans to convert well-managed community-owned forests to other non-forest uses; or planned forest conversion for urban, rural, and infrastructure development (VCS, 2008, p. 13)." Unplanned deforestation activities are defined as: " activities that occur as a result of socio-economic forces that promote alternative uses of forested land, and the inability of institutions to control these activities; such as population growth and the expansion of roads and other infrastructure leading to subsistence food production and fuelwood gathering taking place on lands not designated for such activities (VCS, 2008, p. 13)." The VCS AFOLU Document stated that the following REDD practices are eligible activities under the VCS (VCS, 2008, p. 14):

1) Avoiding planned deforestation (APD): Reduces GHG emissions by stopping deforestation on forest lands that are legally authorized and documented to be converted to non-forest land.

2) Avoiding unplanned frontier deforestation and degradation (AUFDD): Reduces GHG emissions by stopping deforestation/degradation of degraded to mature forests at the forest frontier that has been expanding historically, or will expand in the future, as a result of improved forest access, often through construction of roads.

3) Avoiding unplanned mosaic deforestation and degradation (AUMDD): Reduces GHG emissions by stopping deforestation/degradation of degraded to mature forests occurring in a mosaic pattern.

Parameter 23. Definition of monitoring requirements for Reduced Emissions for Deforestation and forest Degradation (REDD) under the VCS mechanism. For example, the VCS Document stated on page 19 that all carbon pools are required for REDD monitoring activities. The VCS Document directly linked VCS REDD monitoring and reporting to the IPCC Good Practice Guidance for AFOLU, and stated the following (VCS, 2008, p. 31): "the IPCC 2006 Guidelines provide[d] guidance for estimating forest regrowth (carbon accumulation) if degradation is reduced, and for estimating reductions in forest carbon stocks caused by removals of biomass exceeding regrowth. Monitoring and estimation methods currently must be based on the IPCC Guidelines."

At this point, a paragraph is constructed and/or assembled in the word processing software explaining what the retrieved text means in simple language interpretable to anyone skilled in the art. This is an example of a linked summary the documents in **10232** for documents in **10228** from FIG. **2D** when the review is for the VCS mechanism. This is an example of a linked summary the documents in **10232** for documents in **10226** from FIG. **2D** when the review is for the CDM mechanism. The basic point of the retrieved information is that all VCS AFOLU projects must comply with the monitoring methodologies for GHG reporting in the IPCC Good Practice Guidance and UNFCCC CDM methodologies. Furthermore, the retrieved text means the following for the four VCS categories: 1) ARR projects must comply with the monitoring methodologies for GHG reporting in the IPCC Good Practice Guidance UNFCCC CDM methodologies and can draw upon methods from peer-reviewed literature; 2) ALM projects must comply with the monitoring methodologies for GHG reporting in the IPCC Good Practice Guidance and can draw upon methods from peer-reviewed literature; 3) IFM projects must comply with the monitoring methodologies for GHG reporting in the IPCC GPG-2006, IPCC GPG-LULUCF and can draw upon methods from peer-reviewed literature and 4) REDD projects must comply with the monitoring methodologies for GHG reporting in the IPCC Good Practice Guidance and can draw upon methods from peer-reviewed literature. For CDM methods relevant to VCS ARR, all methodological equations stem from the IPCC "Good Practice Guidance for Land Use, Land-Use Change and Forestry" (GPG-LULUCF; IPCC, 2003a, see section 4.3 of this document) and peer-reviewed literature. This means that CDM methods for monitoring and verification follow the IPCC Good Practice Guidance.

Parameters 24-28. Method(s) used to describe requirements to calculate monitoring of GHG activities derived from retrieved text from the "The Climate, Community and Biodiversity Standards" (CCBA, Second Edition December, 2008) document as an example of guidance of a voluntary mechanism. The CCBA document is relevant to parameters 24-28. Parameters 24-28 are an example of the retrieved outputs in **10218** from **FIG. 2D** that are condensed in **10228** from **FIG. 2D** The text retrieval for the CCBA Standard documents used the following as examples of key words: "IPCC", "GPG", "biomass", "monitoring", "land use", "carbon", "verification", "remote sensing", "definition", "dead wood", "litter", "soil", "respiration", "decomposition", "production", "stocks", "land cover", "forest land"; "grassland"; "tier"; "process"; "ecosystem"; "model"; "deforestation, "degradation" and the following symbols "Δ"; "+"; "-"; "=" etc.. In the example provided, the retrieval only focuses on the climate component for compliance with The CCBA Standards related to carbon monitoring and verification for CCBA accredited land-based carbon projects.

Parameter 24. General definition for monitoring requirements of offset activities under the CCBA mechanism. For example, the CCBA Standards are intended for any land-based project including Reduce Emissions through avoided Deforeststion and forest Degradation (REDD) as well as those that remove carbon dioxide through sequestration.

Parameter 25. Definition for monitoring requirements of the original site condition under the CCBA mechanism. For example, Section G.1 on describing the original project site conditions: when describing the original site conditions for climate, The CCBA Standards stated that: "current carbon stocks [must be accounted for] within the project area(s), using stratification by land-use or vegetation type and methods of carbon calculation (such as biomass plots, formulae, default values) from the IPCC's 2006 Guidelines for AFOLU or a more robust and detailed methodology."

Parameter 26. Definition for monitoring requirements of the baseline projections under the CCBA mechanism. For example, Section G.2 on baseline projections: the project baseline projections are intended to provide a "without" project reference scenario. Or in other words, what would happen at the project site if the CCBA accredited project did not occur. Points 1 and 3 related to GHG monitoring and are linked the IPCC Good Practice Guidance. Point 1 stated that the "land-use scenario in the absence of the project" should be described "following IPCC 2006 GL for AFOLU or a more robust and detailed methodology (CCBA, 2008, p. 14)." Point 3 stated that carbon stock changes should be calculated for the absence of project scenario. Estimation of carbon stocks is required for all land-use classes and carbon pools required in the IPCC GPG 2006 for AFOLU (CCBA, 2008, p. 14; and see section 4.4.1 of this document for further detail on these GHG reporting requirements). Project accounting is required for the timeframe of either the project lifetime or accounting period. GHG emissions from $CH_4$ and $N_2O$ are also required for reporting the without project reference scenario.

Parameter 27. Definition for monitoring requirements of project impacts under the CCBA mechanism. For example, Section CL.1 on net positive climate impacts: CCBA accredited projects must generate a positive impact on atmospheric GHG concentrations from land use change within the project site and during the project's lifetime. "Net changes in carbon stocks due to project activities must be estimated using the methods of calculation, formulae and default values of the IPCC Guidelines for AFOLU or a more robust and detailed methodology (CCBA, 2008, p. 22)." Emissions of $CH_4$ and $N_2O$ must be estimated "with" the project activity. GHG emissions resulting from the following project activities must also be quantified: biomass burning, fossil fuel combustion, synthetic fertilizers and decomposition of nitrogen fixing species.

Parameter 28. Definition for monitoring requirements of project impacts under the CCBA mechanism. For example, Section CL.3 on climate impact monitoring: before a project is initiated, the project developers must have a monitoring plan in place to quantify and document changes (within and around the project boundaries) in project-related carbon pools, GHG emissions, and non-$CO_2$ GHG emissions if appropriate (CCBA, 2008, p. 24). Potential carbon pools to be included are: above-ground biomass, below-ground biomass, litter, dead wood, harvested wood products, soil carbon and peat. Carbon pools expected to decrease "must" be monitored. A full monitoring plan must be developed within six months of the project start date.

At this point, a paragraph is constructed and/or assembled in the word processing software explaining what the retrieved text means in simple language interpretable to anyone skilled in the art. This is an example of a linked summary the documents in **10232** for documents in **10228** from FIG. **2D** when the review is for the CCBA mechanism. For example, this means that The CCBA Standards required methods that described the original conditions of a project site and monitoring methodologies during project implementation period to comply with the IPCC Good Practice Guidance and can draw upon methods from peer-reviewed literature.

A science plan is developed from the outputs of the legal / policy analysis. The reason a science plan is developed is to provide an intellectual bridge between what is required by the trading mechanism and the methods and techniques that are used to monitor the vegetation attribute at a project site for a client. With no legal/policy analysis, the science has no intellectual link to the requirements for monitoring and/or reporting of a vegetation attribute for the trading mechanism(s) relevant to the client. The legal/policy analysis shows that two types of vegetation attribute information are required by the client to comply with annual monitoring and/or reporting. The two types of vegetation attribute information are 1) a numerical biophysical element and 2) a land classification element indicating a specific land use. The primary spatial and temporal information used to monitor a vegetation attribute at a client's project site is with remote sensing imagery. Remote sensing imagery means digital images from satellite-borne active and/or passive sensors with measurement application to monitoring vegetation. Examples of remote sensing imagery from active sensors include Light Detection and Ranging (LiDAR) sensors and Synthetic-aperture radar (SAR) sensors. Passive sensors collect reflectance of electromagnetic radiation from the earth's surface. Remote sensing imagery may also be obtained from an unmanned aerial vehicle (i.e., an unmanned aircraft system). There are many satellites in orbit around the earth that can be used to develop a science plan and a database, so that a client's vegetation attribute can be monitored. The key initial component of the science plan is a strategic assessment of the capabilities of sensing instruments onboard current and future planned satellite missions, so that a sensor can be chosen that best implements the desired monitoring and/or reporting required by a client.

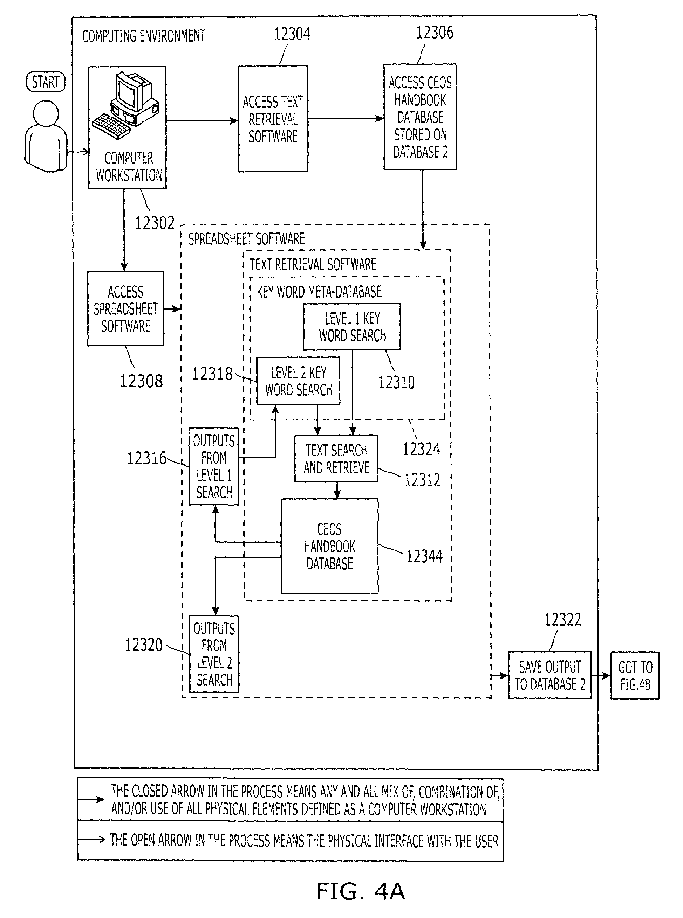

FIG. 4A shows the process used to retrieve text from the database for current and planned satellite missions. A database on current and future planned satellite missions and instrument sensors is developed on Database 2. The Committee on Earth Observation Satellites (CEOS) Handbook provides a regularly updated database of nearly all current and future planned satellite missions and sensing instruments <URL: http://www.eohandbook.com/>. The CEOS Handbook databases are downloaded and stored on Database 2. The information available on the satellite instruments (i.e., the sensor) includes: the satellite mission; the status of the mission; the type of sensor imagery; measurement applications; resolution; swath; accuracy; and other technical characteristics of the instrument. Text retrieval software is used to retrieve information from the CEOS databases and the process is exemplified in FIG. 4A. A user accesses a computer workstation in 12302 that includes screen display(s), processor(s), hard drive(s), a keyboard, a mouse, a router connected to the internet and other physical elements related to a computer workstation, etc. A computer workstation 12302 in this context can store, retrieve, process and/or output data and/or communicate with other computers. The computer workstation is used to access text retrieval software in 12304 and spreadsheet software in 12308. In 12306, the text retrieval software is used to access the CEOS Handbook database. The Level 1 key word search in 12310 for the CEOS Handbook database uses any and/or all the following key words as examples: "vegetation", "global", "forest", "crop", "land", etc. The key words are entered into the text retrieval software in 12312, which is processed with the CEOS Handbook database in 12314. The text is retrieved in a new table in 12316 for the sensor name, status, type; measurement applications; resolution; swath; and accuracy. The CEOS Handbook database also includes information on mission launch date, mission end of life date; orbit details, etc. The names of the satellite missions from the newly retrieved table in the Level 1 key word search are used as key words in a new Level 2 key word in 12318. The search and retrieval in 12312 and 12314 are run again for the Level 2 key words. The mission launch data and end of life date is retrieved to the output of the table in 12320, and is combined with the Level 1 outputs. Other information related to the cost of image acquisition and planned mission continuity is retrieved by the user from the website of the sponsoring Earth Observation Agency. This additional information is retrieved via the internet and added to the satellite instrument table with the results of the Level 1 and Level 2 text retrieval from the CEOS Handbook and as other relevant information become available online. The instrument table is saved in electronic format to Database 2 in 12322 and updated with new releases for the CEOS Handbook. In 12324, the Level 1 and Level 2 key words are stored on a meta-database on Database 2. The instrument table is scored and ranked for a client's monitoring requirements, such as: 1) whether the instrument has global reach for annual monitoring, 2) has at least 5 years of annual historical data to develop a baseline for vegetation attribute(s) at a project site, 3) has data continuity for at least the next 10 years for post-validation monitoring of a vegetation attribute to be consistent with the baseline, and 4) the cost of image acquisition.

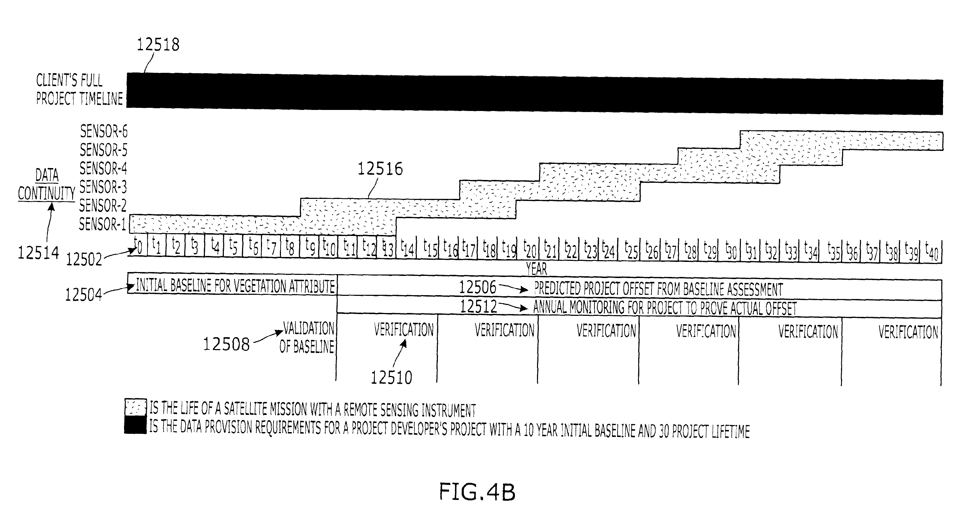

FIG. 4B shows an illustrative output for the strategic assessment of current and planned satellite missions and instrument senor capabilities based on data continuity and data provision for a client's project offset activity. In 12502, $t_0$-$t_{40}$ an example of 41 years in time is shown. This amount of years is only given as an example and the amount of years is variable and dependent on the client's project lifetime. In 12504, an initial baseline period is shown during which a client will need to obtain measurements of a vegetation attribute. The example of the initial baseline in 12504 is for 10 years (i.e., $t_0$-$t_9$) and is used to develop the initial baseline for the vegetation attribute. Normally, the initial baseline can be developed for a period of 5 to 10 years prior to the project start date, because the initial baseline needs to assess the status of current vegetation attributes under the current land use regime prior to project implementation. A longer baseline might be considered inappropriate because it could possibly represent a more historic land use that is no longer reality on the ground. It is important to note here that the project developer client cannot monetize credits for the initial baseline, because this period represents time before the project implementation period. In 12506, a predicted project offset period is shown. 12506 is the offset crediting period for the clients project. The predicted offset is calculated by the client from the baseline assessment with fractional reductions of GHG emissions derived from the project activity. The example in 12506 shows a predicted offset for a project activity lasting 40 years (i.e., between $t_{11}$ and $t_{40}$). In 12508, the project validation year is shown in year $t_{10}$. 12508 is the year that the client intends to get the project accredited through a trading mechanism, or if the project was already accredited, is the year in which the project was accredited. This also means that the baseline in 12504 will normally be developed from a historical database prior to the date of validation. The project may need to be re-validated later in the project life to re-assess a future baseline, but an example of this re-assessment is not provided here. In 12510, project verification for real offsets is shown at 5 year intervals. The 5 year interval is only used as an example here and is dependent on the client. In 12512, a period is shown that matches 12506, where 12512 is the actual annual monitoring used to assess a vegetation attribute after the baseline period during the project implementation. The point is that the actual annual monitoring in 12512 is completed to show that the project activity is actually removing the GHGs that were predicted in 12506. The information contained in 12502 through 12512 is fully dependent on the client's project activity specifications. The point of 15202 through 12512 is to show how many years of continuous monitoring data the client needs to make a project work in reality as a tradable commodity. In 12518, the full period of data provision requirements is shown for the period $t_0$ through $t_{40}$. The point of 12518 is that data provision for a project site should be consistent for the entire period shown in 12518. In 12514, multiple satellite sensors for current and future satellite missions are shown that will be used to suggest the most appropriate data continuity to monitor a client's project site. The period going forward in time is limited to what is known about future planned satellite missions. In **12514,** an example of 6 current and future planned sensors is used as an example. The 6 sensors all have very similar sensors (i.e., resolution, swath, and is either active and/or passive in information collection). In **12516,** the satellite mission project life is shown for each of the 6 sensors. **12516** shows that all years need to be accounted for matching the full period shown in **12518.** For data continuity, there may be overlaps in time between sensors, but typically there would not be substantial missing periods in time. If the project lifetime goes beyond the available information for current and planned sensors, the longest possible data continuity is shown. Data continuity can be more important than any other instrument sensor characteristic (i.e., resolution and/or whether the sensor is active or passive) for effective project monitoring and/or reporting, because a satellite might only have a mission life of between 5-10 years when the client's project life can be in excess of 30 years. If a sensor is chosen that has no long-term data continuity, the client's investment for initial project development for the baseline vegetation attribute will be wasted. This is because the data/information source used to develop the baseline and the predicted offset will no longer be available to the client to show that the predicted offset actually happened once the satellite mission life has ended.

Based on the results from the ranking of information from the instrument table, a standard science product and database is chosen to be initially developed with the NASA Earth Observing System (EOS) Moderate Resolution Imaging Spectroradiometer (MODIS) instrument (i.e., sensor). The MODIS imagery is used to monitor all elements of a vegetation attribute (i.e., biophysical and land classification), because the MODIS class sensors can provide global annual temporal replication. The legal/policy review found that the use of allometric equations and processed-based ecosystem models are defined as Tier 3 significance (i.e., the highest ordered Tier) for monitoring biophysical elements of vegetation attributes. The science plan will formulate directions for allometric equations to be implemented with MODIS sensor imagery and/or a similar sensor class to the MODIS imagery in operation or planned (i.e., MERIS on the ENVISAT mission, VIIRS on the NPOESS mission and/or Sentinel-3). The biophysical elements of a vegetation attribute are developed for annual vegetation flux amount (i.e., vegetation growth) and annual storage in a vegetation stock amount (i.e., woody biomass). A secondary database is developed to provide higher resolution imagery support to a client, but at temporal replication of about once every 5-10 years. The secondary database is initially developed with Landsat imagery. The Landsat imagery is used to monitor land classification elements for a vegetation attribute only, because there is generally only one useful high quality image snapshot available on a global basis about once every 5-10 years with the Landsat class sensor. Other Landsat resolution class remote sensing imagery (i.e., from active and/or passive sensors) that is not free, can be obtained and a database developed at the request of a client with the costs associated to the acquisition of the imagery passed on to the client.

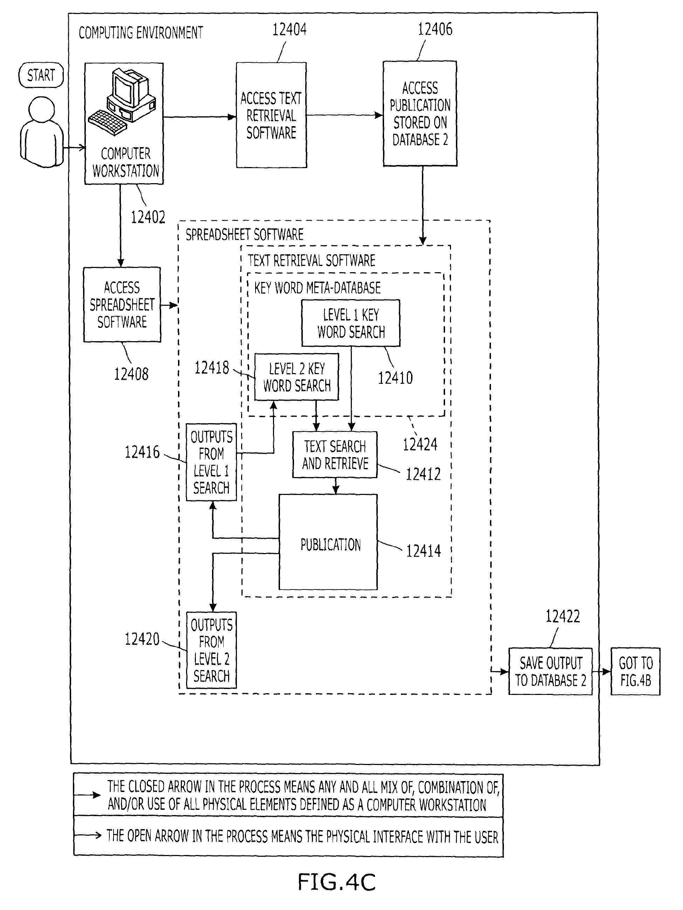

FIG. 4C shows the process used to retrieve text from publications for relevant science on monitoring vegetation attribute(s) with the identified satellite instrument. An intelligence assessment is gathered on the current knowledge base related to the targeted instrument sensor (i.e., the MODIS science base). The websites for publishers of peer-review journal articles are accessed with a computer work station. Examples of these websites are ScienceDirect and Wiley InterScience. The websites for the publishers of peer-review journal articles have internal key word search engines. The website key word search engines are used to search for the identified instrument (i.e., "MODIS"). All journal articles are downloaded and stored to Database 2. Text retrieval software is used to retrieve information from the peer-reviewed journal articles and the process is exemplified in FIG. 4C. A user accesses a computer workstation in 12402 that includes screen display(s), processor(s), hard drive(s), a keyboard, a mouse, a router connected to the internet and other physical elements related to a computer workstation, etc. A computer workstation 12402 in this context can store, retrieve, process and/or output data and/or communicate with other computers. The computer workstation is used to access text retrieval software in 12404 and word processing software in 12408. In 12406, the text retrieval software is used to access the meta-database for the key words develop by the legal policy analysis. In 12410, the key word search developed from Legal / Policy Analysis in 12234 and 12242 stored on a meta-database on Database 1 is used as a Level 1 key word search in 12410. The key words are entered into the text retrieval software in 12412, which is then processed for the target journal article in 12414. The retrieved outputs are sentences from the targeted journal article inputted to word processing software in 12416. If any new key words are identified from the text retrieval in 12416 by a user, the identified words are used as Level 2 key words in 12418. The Level 2 key words are then entered into the text retrieval software in 12412 and processed for the journal article. Tables and figures are also retrieved in any of the retrieval searches when the tables and figures are described by a key word. The retrieved outputs are sentences from the targeted journal article inputted to word processing software in 12420. The retrieved information in the word processing software is saved in electronic format to Database 2 in 12422 and updated with the availability of new journal articles. In 12424, the Level 1 and Level 2 key words are stored on a meta-database on Database 2.

The NASA MODIS sensor's land team has developed many very high quality standard products relevant to monitoring the earth's surface, but not one of these standard products meets the specific legal requirements that are specified by the legal/policy analysis. For example, MODIS MOD 17 was developed to monitor global carbon sequestration for Gross Primary Production (GPP) and Net Primary Production (NPP), but neither the GPP nor NPP cycle measurements fulfill the annual reporting requirements from the legal/policy analysis. The standard MODIS products were developed to meet research needs for an academic audience and user group. The MODIS standard products are, however, very useful as a science-base for which new extensions can be developed that specifically meet compliance guidance for monitoring and/or reporting from the legal/policy analysis. Methods described as Allometric Equations 1 are used to extend standard remote sensing products, such as MODIS MOD 17, with a processed-based dynamic ecosystem, such as Biome-BGC, to a new vegetation attribute amount that complies with the required amount for monitoring and/or reporting of a vegetation attribute noted in the legal/policy review.

IPCC GPG-LULUCF (IPCC, 2003a) stated Biome-BGC as "an example" of a dynamic ecosystem process model (i.e., a biogeochemical model) that can be used in independent verification of vegetation attributes. Biome-BGC is an ecosystem process model that estimates storage and flux of carbon, nitrogen and water. Theory and applications of Biome-BGC and its predecessor, FOREST-BGC, are widely available (e.g., Hunt et al. 1996; Kimball et al. 1997a; Kimball et al. 1997b; Running 1994; Running and Coughlan 1988; Running and Gower 1991; Running and Hunt 1993; Running and Nemani 1991; White et al. 1999; White et al. 2000, Mu et al. 2008). Biome-BGC is available online for download here: <URL: http://www.ntsg.umt.edu/models/bgc/>.

NASA MODIS MOD 17 product for Gross Primary Production (GPP) and Net Primary Production (NPP) was the first continuous satellite-driven dataset monitoring global vegetation productivity (See MODIS MOD 17 user guide, a copy of which is incorporated herein by reference). The MODIS MOD 17 user guide can also be retrieved from: <URL: http://www.ntsg.umt.edu/modis/MOD17UsersGuide.pdf>. The modeling architecture for MOD 17 was developed around Biome-BGC. MOD 17 outputs are validated with FLUXNET data [available online at: <URL: http://www.fluxnet.ornl.gov/fluxnet/index.cfm>]. The difference between Biome-BGC modeled GPP/NPP and MOD 17 modeled GPP/NPP is that the MOD 17 outputs represent real-world growth rates after disturbance and the Biome-BGC outputs are theoretical growth rates before disturbance. Thus, a combination of MODIS GPP/NPP and newly developed Allometric Equations 1 from Biome-BGC modeled parameters extended to MOD 17 are used to quantify all carbon flux (i.e., sequestration or vegetation growth) and storage required for annual GHG reporting in the IPCC GPGs.

Allometric Equations 1 are used to develop annual carbon flux vegetation attribute by extending the modeling architecture used to develop MOD 17 from Biome-BGC. The new extensions use the outputs from the legal/policy review that define the following: the carbon cycle; the carbon pools within the carbon cycle; and the equations used to calculate the annual carbon pools within the carbon cycle. Allometric Equations 1 start at the carbon cycle variable for Net Primary Production (NPP), where MODIS MOD 17 ends. The IPCC's definitions state the amount from NPP after disturbance and respiration is the required annual carbon stock change amount reported in five carbon pools: above-ground biomass, below ground biomass, dead wood, litter and soil organic matter. The IPCC also states that the flux in the carbon pool lost to harvested wood products (i.e., carbon stocks lost to deforestation/degradation) is reported separately. Thus, NPP is 100% of the total amount of carbon that can be allocated to the five carbon pools and lost to respiration. Carbon sequestration from vegetation growth after disturbance is the difference between remote sensing-derived NPP and a process-based dynamic ecosystem model-derived NPP. The average percentages of Biome-BGC modeled NPP allocated annually to the five carbon pools and lost to respiration is used to develop Allometric Equations 1 for annual carbon flux (i.e., carbon sequestration or vegetation growth rates). Allometric Equations 1 meet the same specifications of the MOD 17 architecture (i.e., the fractions change by land cover type). Allometric Equations 1 for annual carbon flux are then used with MOD 17 NPP to partition the total remote sensing NPP to the five carbon pools after carbon is lost to respiration.

Allometric Equations 1 are developed for the annual woody biomass/carbon stock storage in a vegetation attribute by extending the modeling architecture used to develop MOD 17 from Biome-BGC. The new Biome-BGC extensions for annual woody carbon stocks are based on the theoretical architecture of MOD 17 available online at: <URL: http://www.fluxnet.ornl.gov/fluxnet/index.cfm>. One element in the MOD 17 process for calculating NPP includes a calculation for the vegetation attribute of annual live-wood biomass from a vegetation index (i.e., Leaf-Area Index [LAI] in standard publically disclosed MOD 17 architecture). The term live-wood biomass means the outer bark, inner bark, cambium and sapwood portions of a tree's physiology. The dead wood biomass element of the tree's physiology is still needed to calculate annual woody biomass storage. The term dead wood means the heartwood of a tree's physiology. In theory, total woody biomass storage is 100%. Live wood biomass and dead wood biomass equals a percentage allocation of the total woody biomass at 100%. Biome-BGC is used to model annual woody biomass, annual live wood biomass and annual dead wood biomass. Allometric Equations 1 for woody biomass are then developed for the fractional relationship between annual woody biomass stocks, annual live wood biomass and annual dead wood biomass. Allometric Equations 1 meet the same specifications of the MOD 17 architecture (i.e., the fractions change by land cover type). Allometric Equations 1 for annual woody biomass storage are then used with MOD 17-derived live wood biomass to equate annual dead wood biomass that is in turn used with live wood biomass to equate annual total woody biomass storage.

Data mining software uses input data and machine learning algorithms to extract patterns from the input data that are used to develop a predictor model. The methods described for Allometrics Equations 2 use data mining software to develop a regression and/or classification predictor model between a targeted sample(s) of a vegetation attribute and remote sensing imagery. The input data is stored on the database and comprises of 1) samples of target data for geospatial vegetation attributes and 2) remote sensing imagery (i.e., the standard MODIS database, the secondary Landsat database and/or other remote sensing imagery that a client prefers to use). A standard data mining target database is developed for samples of vegetation attribute(s) from any and/or all of the following sources: 1) geospatial data recorded as a publication in peer-review literature (i.e., this means the text of the publication provides a vegetation attribute amount with an associated longitude and latitude in the text of the publication and/or when there is a referenced publication to a publically available gridded electronic geospatial data file of a vegetation attribute); 2) geospatial data of the vegetation attribute reported publically as an official estimate from a government body; 3) geospatial data of the vegetation attribute reported publically that is stored on the database of a regulated and/or voluntary trading mechanism; 4) the standard remote sensing modeled product(s) for a vegetation attribute, such as MODIS MOD 17 and 5) any and/or all other publically available geospatial data for a vegetation attribute that is relevant to a client for monitoring and/or reporting under a trading mechanism. A client can also provide confidential project specific geospatial data for a vegetation attribute(s) that is stored in a database. The data mining software mines the target samples of vegetation attributes with the remote sensing imagery stored on the database. The mining identifies whether a pattern exists between the samples of the vegetation attribute and the remote sensing imagery. A pattern that exists between the samples for the vegetation attribute and remote sensing imagery is used to train a predictor model. Model validation and verification options are standard in data mining software, so the percentage of samples reserved for validation and/or verification is decided by the client. After an acceptable predictive model is developed, the data mining software is used to score the full image of input remote sensing data for a predicted vegetation attribute.

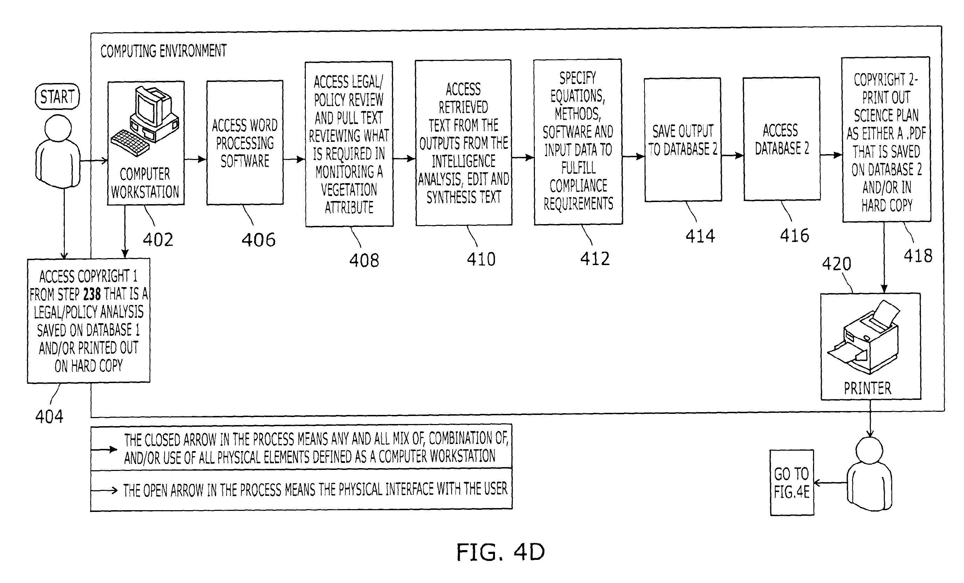

FIG. 4D shows the generic process used to develop a science plan. A computer workstation in 402 includes screen display(s), processor(s), hard drive(s), a keyboard, a mouse, a router connected to the internet and other physical elements related to a computer workstation, etc. A computer workstation for 402 in this context can store, retrieve, process and/or output data and/or communicate with other computers. Copyright 1 from 238 is viewed in 404 as either a printed out hard copy and/or accessed in Portable Document Format (i.e, :pdf and/or similar file format) by the computer workstation. A word processing software (i.e., MS Word) is accessed in 406 by the computer workstation. The text from the legal / policy analysis is pulled by the user in 408 related to what is required for monitoring the vegetation attribute. The retrieved text from the intelligence analysis from steps 12416 and 12420 is accessed in 410, edited and synthesized by a user to summarize the current knowledge base. In 412, the directions that will be used to develop and implement the monitoring procedures for a vegetation attribute are drafted by the user. The directions in 412 include specific equations, methods, software and input data that will be used to monitor the vegetation attribute. The full document is saved on Database 2 in 414. The outputs of 412 are accessed in 416, defined as Copyright 2 in 418 that is printed out in either a Portable Document Format (i.e, .pdf and/or similar file document format) digital file and/or in hard copy with a printer in 420.

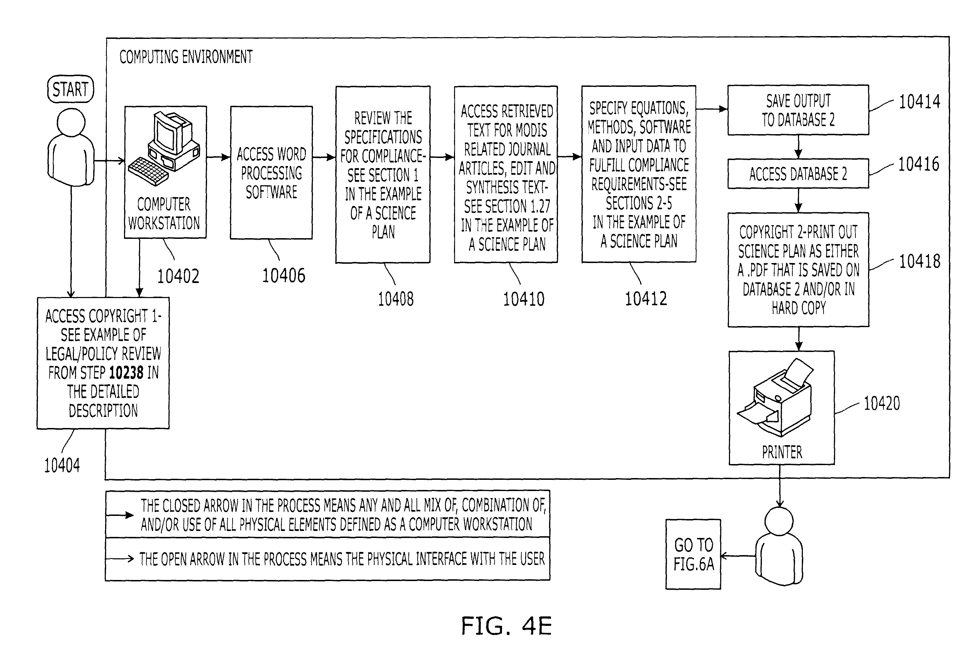

FIG. 4E shows an example for the generic process used to develop a science plan applied to the outputs of 10238. Science plans are developed to match the technical requirements specified in the Legal / Policy Analysis for monitoring and/or reporting the relevant vegetation attributes-carbon flux and storage-that are in line with the Voluntary Carbon Standard's (VCS) guidance in AFOLU and the Climate, Community, and Biodiversity Alliance's Standards. A computer workstation in 10402 includes screen display(s), processor(s), hard drive(s), a keyboard, a mouse, a router connected to the internet and other physical elements related to a computer workstation, etc. A computer workstation at 10402 in this context can store, retrieve, process and/or output data and/or communicate with other computers. Copyright 1 from 10238 is stored and managed as a data structure, and, for example, can be viewed in 10404 as either a printed out hard copy and/or accessed in Portable Document Format (i.e., .pdf and/or similar file format) by the computer workstation. A word processing software (i.e., MS Word) is accessed in 10406 by the computer workstation. The text from the legal/policy analysis is pulled by the user in 10408 related to what is required for monitoring the vegetation attribute. The retrieved text from the intelligence analysis from steps 12416 and 12420 is accessed in 10410, edited and synthesized by a user to summarize the current knowledge base. In 10412, the directions that will be used to develop and implement the monitoring procedures for a vegetation attribute are drafted by the user. The directions in 10412 include specific equations, methods, software and input data that will be used to monitor the vegetation attribute. The full document is saved on Database 2 in 10414. The outputs of 10412 are accessed in 10416, defined as Copyright 2 in 10418 that is printed out in either a Portable Document Format (i.e., .pdf and/or similar file document format) digital file and/or in hard copy with a printer in 10420.

The following from points 1-5 are examples of science plans and defined as Copyright 2 in 10418 from FIG. 4E, which is/are generated for monitoring the target vegetation attribute for the target eco-region, based upon the compiled policy parameters.

1.0 Policy background (the text in section 1 is retrieved text referenced in **10408** from **FIG. 4E** and is similar as the legal/policy analysis, except the material in **Table 1.** Policy guidance in section one is only provided as a justification for land use in Table 1 justified):

1.1 Use of standardized international land cover data sets:

1.2 The use of international land cover date sets can be used to monitor the 6 land use categories required in the AFOLU sector in the following capacity (IPCC, 2006, Vol. 4, Ch 3, p. 3.25):

1.3 Estimating spatial distribution of land-use categories: Conventional inventories usually provide only the total sum of land-use area by classes. Spatial distribution can be reconstructed using international land-use and land cover data as auxiliary data where national data are not available.

1.4 Reliability assessment of the existing land-use datasets: Comparison between independent national and international datasets can indicate apparent discrepancies, and understanding these may increase confidence in national data and/or improve the usability of the international data, if required for purposes such as extrapolation.

1.5 When using an international dataset, inventory compilers should consider the following:

1.6 The classification scheme (e.g., definition of land-use classes and their relations) may differ from that in the national system.

1.7 Spatial resolution (typically 1 km nominally but sometimes an order of magnitude more in practice) may be coarse, so national data may need aggregating to improve comparability.

1.8 Classification accuracy and errors in geo-referencing may exist, though several accuracy tests are usually conducted at sample sites. The agencies responsible should have details on classification issues and tests undertaken.

1.9 As with national data, interpolation or extrapolation will probably be needed to develop estimates for the time periods to match the dates required for reporting.

1.10 The IPCC Good Practice Guidance suggested that international land cover maps can be used to monitor the six land use categories required in the AFOLU sector (IPCC, 2006, Vol. 4, Ch 3, p. 3.25). The IPCC GPG-2006 referred to the International Geosphere-Biosphere Program's (IGBP) Global 1 km x 1 km land cover map as one example of international land cover maps applicable for comparison with national datasets (IPCC, 2006, Vol. 4, Ch 3, p. 3.25). Carbon Auditors used IGBP land cover classification maps developed from the NASA EOS MODIS satellite sensor to reclassify and map the six AFOLU categories. **Table 1** lists the IGBP land cover classes in the left column. The right column lists which of the IGBP land cover classes were reclassified to AFOLU classes for the following: forested land, grassland, cropland, wetland, settled land and other land.

Table 1

| IGBP Definitions | AFOLU definitions |
| --- | --- |
| 0) Water | No Data |
| 1) Evergreen needleleaf forest | 1) Forested land |
| 2) Evergreen broadleaf forest | 1) Forested land |
| 3) Deciduous needleleaf forest | 1) Forested land |
| 4) Deciduous broadleaf forest | 1) Forested land |
| 5) Mixed forests | 1) Forested land |
| 6) Closed shrublands | 2) Grassland |
| 7) Open shrublands | 2) Grassland |
| 8) Woody savannas | 2) Grassland |
| 9) Savannas | 2) Grassland |
| 10) Grasslands | 2) Grassland |
| 11) Permanent wetlands | 4) Wetland |
| 12) Croplands | 3) Cropland |
| 13) Urban and built-up | 5) Settled land |
| 14) Cropland/natural vegetation mosaic | 3) Cropland |
| 15) Permanent snow and ice | 6) Other land |
| 16) Barren or sparsely vegetated | 6) Other land |
| 17) UNCLASSIFIED | 6) Other land |

1.11 Carbon Model Theory & Design (the text in section 1.11 is retrieved from the intelligence assessment from **FIG. 4C)** :

Biome-BGC

Biome-BGC is an ecosystem process model that estimates storage and flux of carbon, nitrogen and water. Using prescribed site conditions, meteorology, and parameter values, Biome-BGC simulates daily fluxes and states of carbon, water, and nitrogen for coarsely defined biomes at areas ranging from 1 $m^2$ to the entire globe. Plant physiological processes respond to diurnal environmental variation (Geiger and Servaites 1994), but Biome-BGC uses a daily time in order to take advantage of widely available daily temperature and precipitation data from which daylight averages of short wave radiation, vapor pressure deficit, and temperature are estimated (Thornton et al. 1997; Thornton and Running 1999).

Biome-BGC simulates the development of soil and plant carbon and nitrogen pools; no input of soil carbon information or leaf area index (LAI, $m^2$ leaf area per $m^2$ ground area) is required. LAI controls canopy radiation absorption, water interception, photosynthesis, and litter inputs to detrital pools and is thus central to Biome-BGC. Model structure is discussed by Thornton (Thornton, 1998), and will not be presented here. Briefly, though, NPP is based on gross primary production simulated with the Farquhar photosynthesis model (Farquhar et al. 1980) minus maintenance respiration [calculated as a function of tissue nitrogen concentration (Ryan 1991)] and growth respiration (a constant fraction of gross primary production). Theory and applications of Biome-BGC and its predecessor, FOREST-BGC, are widely available (e.g., Hunt et al. 1996; Kimball et al. 1997a; Kimball et al. 1997b; Running 1994; Running and Coughlan 1988; Running and Gower 1991; Running and Hunt 1993; Running and Nemani 1991; White et al. 1999; White et al. 2000, Mu et al. 2008).

NASA MODIS MOD 17

NASA MODIS MOD 17 product for Gross Primary Production (GPP) and Net Primary Production (NPP) is the first continuous satellite-driven dataset monitoring global vegetation productivity. The algorithm is based on the original logic of Monteith (1972, 1977) suggesting that NPP under non-stressed conditions is linearly related to the amount of absorbed Photosynthetically Active Radiation (PAR) during the growing season. In reality, vegetation growth is subject to a variety of stresses that tend to reduce the potential growth rate, especially stresses resulting from climate (temperature, radiation, and water) or the interaction of these primary abiotic controls, which impose complex and varying limitations on vegetation activity in different parts of the world (Churkina & Running,1998; Nemani et al., 2003; Running et al., 2004). Combining the logic of Monteith, climate controls, and some principles of modeling NPP learned from a general process-based ecosystem model, Biome-BGC (Running & Hunt, 1993), the MODIS GPP/NPP algorithm was developed using satellite-derived land cover, the fraction of photosynthetically active radiation absorbed by vegetation (FPAR), and leaf area index (LAI) as input surface vegetation information (Running et al., 2000), while the necessary climate information is obtained from a global climatic data assimilation system developed by the NASA Goddard Global Modeling and Assimilation Office (GMAO).

2.0 Science Plan-Directions 1: Application of a dynamic ecosystem model to develop Allometric Equations 1 for carbon flux variables. (Section 2 through Section 5 in this example of a science plan are referenced in **10412** from **FIG. 4D)**

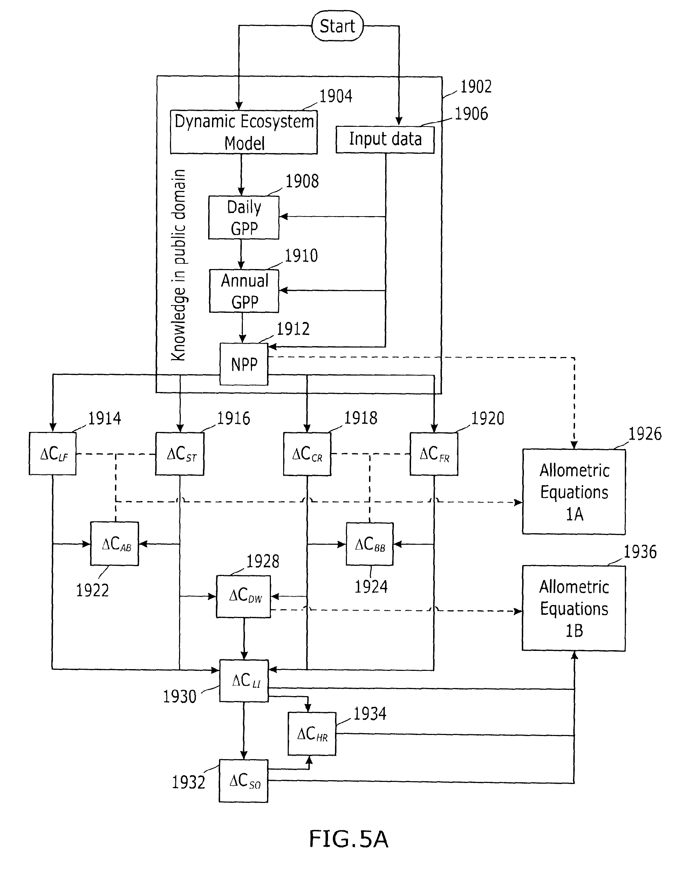

2.1 FIG. 5A shows an example of the process used to develop Allometric Equations 1. FIG. 5A is used with Science Plan-Directions 1 in section 2 in the example of a science plan. Directions 1 develop allometrics from a processed-based dynamic ecosystem model for carbon sequestration. Straight lines show the pathway or route to calculate carbon flux and dotted lines indicate route to develop Allometric Equations 1. More particularly, the following are methods for implementing a dynamic ecosystem model with a land cover map to develop Allometric Equations for monitoring the legally required carbon pools identified for carbon flux. The allometrics that are developed are, in theory, an extension to modeling gross primary production (GPP) and net primary production (NPP) (i.e., a light use efficiency model) with remote sensing imagery. The allometrics can therefore be implemented with any remote sensing-derived model of NPP. The allometrics were specifically developed here as an extension to the NASA Moderate Resolution Imaging Spectroradiometer (MODIS) sensor standardized global GPP/NPP algorithm called MOD 17. In this example, the dynamic ecosystem model BIOME-BGC is run for thirty individual years with global input data and the mean carbon pool variable for all years will be used for development Allometric Equations 1. **FIG. 5A** shows the process used to develop carbon flux variables. The box **1902** defines the knowledge in public space. The steps that fall outside **1902** are new to science. The process begins by using input data in **1906** and a dynamic ecosystem model in **1904** to calculate daily GPP in **1908,** annual GPP in **1910** and NPP in **1912.**

2.2 Develop Allometric Equations 1 A in **1926** (see **FIG. 5A)** for above-ground biomass ($\Delta C_{AB}$) in **1922** is calculated as the sum of leaf organic matter ($\Delta C_{LF}$) in **1914** and the sum of organic matter diverted to the stem ($\Delta C_{ST}$) in **1916** as the following ($AB_1$ and $AB_2$):

$$LC_i\_AB_1\_LF\_ratio = LC_i(\overline{\chi}\Delta C_{LF}[gCm^{-2}]/\overline{\chi}NPP[gCm^{-2}]) \quad \text{Eq.3}$$

$$LC_i\_AB_2\_ST\_ratio = LC_i(\overline{\chi}\Delta C_{ST}[gCm^{-2}]/\overline{\chi}NPP[gCm^{-2}]) \quad \text{Eq.4}$$

Where $LC_i$ is the specific land cover category (BGC land cover was used here), $\overline{\chi}\Delta C_{LF}$ is the mean carbon flux (g) in annual leaf organic matter for an individual land cover class (but is interchangeable with any land cover classification system), $\overline{\chi}\Delta C_{ST}$ is the mean carbon flux (g) in annual leaf organic matter for an individual land cover class and $\overline{\chi}NPP$ is the annual carbon flux (g) amount for mean Net Primary Production (NPP) in the same land cover category.

2.3 Develop Allometric Equations 1 A (see **FIG. 5A)** for below-ground biomass ($\Delta C_{BB}$) in **1924** will be calculated as the sum of fine root carbon ($\Delta C_{FR}$) in **1920** and the sum of coarse root carbon ($\Delta C_{CR}$) in **1918** as the following ($BB_1$ and $BB_2$):

$$LC_i\_BB_1\_FR\_ratio = LC_i(\overline{\chi}\Delta C_{FR}[gCm^{-2}]/\overline{\chi}NPP[gCm^{-2}]) \quad \text{Eq.5}$$

$$LC_i\_BB_2\_CR\_ratio = LC_i(\overline{\chi}\Delta C_{CR}[gCm^{-2}]/\overline{\chi}NPP[gCm^{-2}]) \quad \text{Eq.6}$$

Where $LC_i$ is the specific land cover category, $\overline{\chi}\Delta C_{FR}$ is the mean fine root carbon accumulation (g) amount for an individual land cover category, $\overline{\chi}\Delta C_{CR}$ is the mean coarse root carbon accumulation (g) amount for an individual land cover category and $\overline{\chi}NPP$ is the annual carbon flux (g) amount for mean Net Primary Production (NPP) in the same land cover category.

2.4 Develop Allometric Equations **1 B** in **1936** (see **FIG. 5A)** based on the combined relationship of biomass carbon pools allocated to Dead Orgainic Matter and Soil carbon pools defined in the generalized IPCC flowchart of the carbon cycle (see **FIG. 5A).** The allometric equations are based on the mean carbon flux values for all years of the model run.
Annual dead wood ($\Delta C_{DW}$) accumulation in **1928** for each category is calculated by the following two calculations ($DW_1$ and $DW_2$):

$$LC_i\_DW_1\_ST\_ratio = LC_i(\overline{\chi}\Delta C_{DW}[gCm^{-2}]/\overline{\chi}\Delta C_{ST}[gCm^{-2}]) \quad \text{Eq.7}$$

$$LC_i\_DW_2\_CR\_ratio = LC_i(\overline{\chi}\Delta C_{DW}[gCm^{-2}]/\overline{\chi}\Delta C_{CR}[gCm^{-2}]) \quad \text{Eq.8}$$

Where $LC_i$ is the specific land cover category, $\overline{\chi}\Delta C_{DW}$ is the mean annual dead wood accumulation in the associated land cover category, $\overline{\chi}\Delta C_{ST}$ is the mean carbon flux (g) in annual leaf organic matter for an individual land cover class, and $\overline{\chi}\Delta C_{CR}$ is the mean coarse root carbon accumulation (g) amount for an individual land cover class.

2.5 Annual litter ($\Delta C_{LI}$) accumulation in **1930** for each category is calculated by the following five calculations ($LI_1$ - $LI_5$):

$$LC_i\_LI_1\_LF\_ratio = LC_i(\overline{\chi}\Delta C_{LI}[kgCm^{-2}]/\overline{\chi}\Delta C_{LF}[kgCm^{-2}]) \quad \text{Eq.9}$$

$$LC_i\_LI_2\_ST\_ratio = LC_i(\overline{\chi}\Delta C_{LI}[kgCm^{-2}]/\overline{\chi}\Delta C_{ST}[kgCm^{-2}]) \quad \text{Eq.10}$$

$$LC_i\_LI_3\_FR\_ratio = LC_i(\overline{\chi}\Delta C_{LI}[kgCm^{-2}]/\overline{\chi}\Delta C_{FR}[kgCm^{-2}]) \quad \text{Eq.11}$$

$$LC_i\_LI_4\_CR\_ratio = LC_i(\overline{\chi}\Delta C_{LI}[kgCm^{-2}]/\overline{\chi}\Delta C_{CR}[kgCm^{-2}]) \quad \text{Eq.12}$$

$$LC_i\_LI_5\_DW\_ratio = LC_i(\overline{\chi}\Delta C_{LI}[kgCm^{-2}]/\overline{\chi}\Delta C_{DW}[kgCm^{-2}]) \quad \text{Eq.13}$$

Where $LC_i$ is the specific land cover category, $\overline{\chi}\Delta C_{LI}$ is the mean annual litter accumulation in the specific $LC_i$ category, $\overline{\chi}\Delta C_{LF}$ is the mean annual leaf carbon transferred to litter in the specific $LC_i$ category, $\overline{\chi}\Delta C_{ST}$ is the mean annual stem carbon transferred to litter in the specific $LC_i$ category, $\overline{\chi}\Delta C_{FR}$ is the mean annual fine root transferred to litter in the specific $LC_i$ category, $\overline{\chi}\Delta C_{CR}$ is the mean annual coarse root transferred to litter in the specific $LC_i$ category and $\overline{\chi}\Delta C_{DW}$ is the mean annual dead wood accumulation in the associated $LC_i$ category.

2.6 Annual soil organic matter ($\Delta C_{SO}$) accumulation in **1932** for each category is calculated by the following equation ($SO_1$):

$$LC_i\_SO_1\_LI\_ratio = LC_i(\overline{\chi}\Delta C_{SO}[kgCm^{-2}]/\overline{\chi}\Delta C_{LI}[kgCm^{-2}]) \quad \text{Eq.14}$$

Where $LC_i$ is the specific land cover category, $\overline{\chi}\Delta C_{SO}$ is the mean annual accumulation in soil organic matter in the individual $LC_i$ category and $\overline{\chi}\Delta C_{LI}$ is the mean annual litter accumulation in the specific $LC_i$ category.

2.7 Annual heterotrophic respiration ($\Delta C_{HR}$):
Heterotrophic respiration is the carbon flux amount loss to the atmosphere due to decomposition. The IPCC GPGs defines Net Ecosystem Production (NEP) as the numerical difference between heterotrophic respiration and Net Primary Production (NPP) (section 1.5 above). The IPCC GPGs only suggest using the mean rate of regional decay and do provide direction for calculating heterotrophic respiration set forth by the generic equations in the GPG-2006 (Equation 2 above). Two methods for measuring heterotrophic respiration can be used. The first is directly using the annual heterotrophic respiration measurements from a dynamic process model. This method is useful for monitoring dynamic annual heterotrophic respiration. The second is to develop an average heterotrophic respiration amount, so that heterotrophic respiration can be spatially assessed at the same resolution as the satellite imagery. Section 2.8 describes the second method to equate mean heterotrophic respiration.

2.8 Annual heterotrophic respiration ($\Delta C_{HR}$) accumulation in **1934** for each land cover category is calculated by the following two calculations ($HR_1$ and $HR_2$):

$$LC_i\_HR_1\_LI\_ratio = LC_i(\overline{\chi}\Delta C_{HR}[kgCm^{-2}]/\overline{\chi}\Delta C_{LI}[kgCm^{-2}]) \quad \text{Eq.15}$$

$$LC_i\_HR_2\_SO\_ratio = LC_i(\overline{\chi}\Delta C_{HR}[kgCm^{-2}]/\overline{\chi}\Delta C_{SO}[kgCm^{-2}]) \quad \text{Eq.16}$$

Where $LC_i$ is the specific land cover category, $\overline{\chi}\Delta C_{HR}$ is the mean annual dead wood accumulation in the associated land cover category, $\overline{\chi}\Delta C_{LI}$ is the mean carbon flux in annual litter for an individual land cover class, and $\overline{\chi}\Delta C_{SO}$ is

the mean soil organic matter carbon accumulation amount for an individual land cover category.

2.9 Application of MOD 17 to AFOLU requirements:

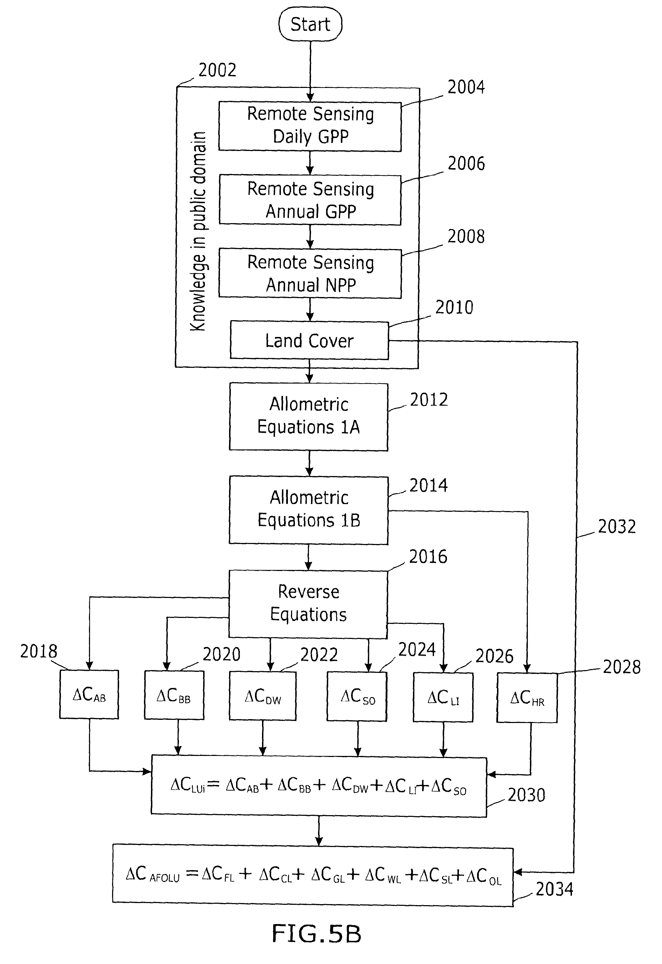

**FIG. 5B** shows an example of the process used to implement Allometric Equations 1 with remote sensing imagery. **FIG. 5B** is used with Science Plan-Directions 1 in section 2 in the example of a science plan. Directions 1 implement the allometrics from developed in **FIG. 5A** with remote sensing imagery. More particularly, the following methods are used to implement allometic Equations A and B with a satellite remote sensing dynamic model of Gross Primary Production (GPP) and Net Primary Production (NPP). **FIG. 5B** shows the process used to implement Allometric Equations 1 A and 1B with remote sensing derived GPP/NPP and a standardized land cover map(s). Biome-BGC and MODIS MOD 17 were used as the dynamic ecosystem model from sections 2.1-2.8, but the process is applicable to all dynamic process models. Furthermore, the process in FIG. 20 can be implemented with any remote sensing measurement of GPP/NPP and/or any light use efficiency model. MODIS MOD 17 NPP algorithm provides real time and real world estimates of global carbon flux in biomass. The MOD 17 algorithm architecture is built around the BIOME-BGC model, which was cited in GPG-LULUCF as an example of well known ecosystem model that could be used for verification in the LULUCF sector under IPCC Good Practice Guidelines. Thus, a combination of MOD 17 NPP plus BIOME-BGC parameters for carbon pools fulfils the good practice guidance for LULUCF sector verification within the same dynamic ecosystem modeling architecture.

2.10 The box referred to in **2002** (see **FIG. 5B**) defines the current knowledge space in public domain. MOD 17 is run to calculate daily GPP in **2004 ,** annual GPP in **2006** and NPP in 2008.

2.11 A standardized land cover map in **2010** is overlaid over NPP.

2.12 Implement Allometric Equations 1 A in **2012** (see **FIG. 5B**):

2.13 The results for Equations 3 and 4 are processed with satellite derived NPP to determine total annual above-ground biomass flux ($\Delta C_{AB}$):

$$\Delta C_{AB}\_LF\_1[gCm^{-2}] = LC_i\_AB_1\_LF\_ratio * MOD17\_NPP[gCm^{-2}] \quad \text{Eq.17}$$

$$\Delta C_{AB}\_ST\_2[gCm^{-2}] = LC_i\_AB_2\_ST\_ratio * MOD17\_NPP[gCm^{-2}] \quad \text{Eq.18}$$

$$\Delta C_{AB}\_IN\_3[gCm^{-2}] = \Delta C_{AB}\_LF\_1[gCm^{-2}] + \Delta C_{AB}\_ST\_2[gCm^{-2}] \quad \text{Eq.19}$$

Where $\Delta C_{AB}\_IN\_3[gCm^{-2}]$ is equal to the total carbon flux in $\Delta C_{AB}$, but not inclusive of carbon flux transferred to other carbon pools.

2.14 The results for Equations 5 and 6 are processed with satellite derived NPP to determine total annual below-ground biomass flux ($\Delta C_{BB}$):

$$\Delta C_{BB}\_FR\_1[gCm^{-2}] = LC_i\_BB_1\_FR\_ratio * MOD17\_NPP[gCm^{-2}] \quad \text{Eq.20}$$

$$\Delta C_{BB}\_CR\_2[gCm^{-2}] = LC_i\_BB_2\_CR\_ratio * MOD17\_NPP[gCm^{-2}] \quad \text{Eq.21}$$

$$\Delta C_{BB}\_IN\_3[gCm^{-2}] = \Delta C_{BB}\_FR\_1[gCm^{-2}] + \Delta C_{BB}\_CR\_2[gCm^{-2}] \quad \text{Eq.22}$$

Where $\Delta C_{BB\_}IN\_3[gCm^{-2}]$ is equal to the total carbon flux amount in annual below ground biomass, but not inclusive of carbon flux transferred to other carbon pools.

2.15 Implement Allometric Equations 1 B in 2014 (see **FIG. 5B**):

2.16 The results for Equations 7 and 8 are processed with Equations 3 and 4 to determine the annual accumulation of dead wood ($\Delta C_{DW}$) per $LC_i$ categories:

$$\Delta C_{DW}\_ST\_1[gCm^{-2}] = LC_i\_DW_1\_ST\_ratio * \Delta C_{AB}\_ST\_2[gCm^{-2}] \quad \text{Eq.23}$$

$$\Delta C_{DW}\_CR\_2[gCm^{-2}] = LC_i\_DW_2\_CR\_ratio * \Delta C_{BB}\_CR\_2[gCm^{-2}] \quad \text{Eq.24}$$

$$\Delta C_{DW}\_IN\_3[gCm^{-2}] = \Delta C_{DW}\_ST\_1[gCm^{-2}] + \Delta C_{DW}\_CR\_2[gCm^{-2}] \quad \text{Eq.25}$$

Where $\Delta C_{DW\_}IN\text{-}3[gCm^{-2}]$ is equal to the total carbon flux amount in annual dead wood debris, but not inclusive of carbon flux transferred to annual litter flux.

2.17 The result for Equations 9 to 13, 17 and 18, 20 and 21, and 23 and 24 are processed together to determine total annual litter carbon flux ($\Delta C_{LI}$) per $LC_i$ categories:

$$\Delta C_{LI}\_LF\_1[gCm^{-2}] = LC_i\_LI_1\_LF\_ratio * \Delta C_{AB}\_LF\_1[gCm^{-2}] \quad \text{Eq.26}$$

$$\Delta C_{LI}\_ST\_2[gCm^{-2}] = LC_i\_LI_2\_ST\_ratio * \Delta C_{AB}\_ST\_2[gCm^{-2}] \quad \text{Eq.27}$$

$$\Delta C_{LI}\_FR\_3[gCm^{-2}] = LC_i\_LI_3\_FR\_ratio * \Delta C_{BB}\_FR\_1[gCm^{-2}] \quad \text{Eq.28}$$

$$\Delta C_{LI}\_CR\_4[gCm^{-2}] = LC_i\_LI_4\_CR\_ratio * \Delta C_{BB}\_CR\_2[gCm^{-2}] \quad \text{Eq.29}$$

$$\Delta C_{LI}\_DW\_5[gCm^{-2}] = LC_i\_LI_5\_DW\_ratio * \Delta C_{DW}\_IN\_3[gCm^{-2}] \quad \text{Eq.30}$$

$$\Delta C_{LI}\_IN\_6[gCm^{-2}] = \Delta C_{LI}\_LF\_1[gCm^{-2}] + \Delta C_{LI}\_ST\_2[gCm^{-2}]$$
$$+ \Delta C_{LI}\_FR\_3[gCm^{-2}] + \Delta C_{LI}\_CR\_4[gCm^{-2}]$$
$$+ \Delta C_{LI}\_DW\_5[gCm^{-2}] \quad \text{Eq.31}$$

Where $\Delta C_{LI\_}IN\_6[gCm^{-2}]$ is equal to the total carbon flux amount in annual litter, but not inclusive of carbon flux flux transferred from litter to soil organic matter and lost to the atmosphere through heterotrophic respiration.

2.18 The results for Equations 14 and 31 are processed together to determine total annual soil organic matter carbon flux ($\Delta C_{SO}$) per *LCi* categories :

$$\Delta C_{SO}\_IN\_1[gCm^{-2}] = LC_i\_SO_1\_LI\_ratio*\Delta C_{LI}\_IN\_6[gCm^{-2}] \quad \text{Eq.32}$$

Where $\Delta C_{SO\_}IN\_1[gCm^{-2}]$ is equal to the total carbon flux amount in soil organic matter under IPCC GPGs, but not inclusive of the carbon flux lost to the atmosphere during heterotrophic respiration.

2.19 The results for Equations 15 and 16 are processed with Equations 31 and 32 to determine annual heterotrophic respiration ($\Delta C_{HR}$) in **2028** per $LC_i$ category (see **FIG. 5b):**

$$\Delta C_{HR}\_LI\_1[gCm^{-2}] = LC_i\_HR_1\_LI\_ratio*\Delta C_{LI}\_IN\_6[gCm^{-2}] \quad \text{Eq.33}$$

$$\Delta C_{HR}\_SO\_2[gCm^{-2}] = LC_i\_HR_2\_SO\_ratio*\Delta C_{SO}\_IN\_1[gCm^{-2}] \quad \text{Eq.34}$$

$$\Delta C_{HR}\_IN\_3[gCm^{-2}] = \Delta C_{HR}\_LI\_1[gCm^{-2}]+\Delta C_{HR}\_SO\_2[gCm^{-2}] \quad \text{Eq.35}$$

Where $\Delta C_{HR\_}IN\_3[gCm^{-2}]$ is equal to the total carbon flux lost to the atmosphere through heterotrophic respiration.

2.20 Implement Reverse Equations in **2016** (see **FIG. 5B**):

2.21 Reverse equations are used to calculate total annual carbon flux for Net Biome Production under IPCC GPGs per land use type ($\Delta C_{LUi}$):

2.22 Equation 2 in **2030** was described in the IPCC Good Practice Guideline to calculate Net Biome Production per land use type.

2.23 Annual carbon flux in soil organic matter ($\Delta C_{SO}$) in **2024** per land use category (*LUi*) is calculated by the following:

$$\Delta C_{SO}\_LUi[gCm^{-2}] = \Delta C_{SO}\_IN\_1[gCm^{-2}]-\Delta C_{HR}\_SO\_2[gCm^{-2}] \quad \text{Eq.36}$$

2.24 Annual carbon flux in litter ($\Delta C_{LI}$) in **2026** per land use category (*LUi*) is calculated by the following:

$$\Delta C_{LI}\_LUi[gCm^{-2}] = \Delta C_{LI}\_IN\_6[gCm^{-2}]-\Delta C_{HR}\_LI\_1[gCm^{-2}]$$
$$-\Delta C_{SO}\_IN\_1[gCm^{-2}] \quad \text{Eq. 37}$$

2.25 Annual carbon flux in dead wood ($\Delta C_{DW}$) in **2022** per land use category (*LUi*) is calculated by the following:

$$\Delta C_{DW}\_LUi[gCm^{-2}] = \Delta C_{DW}\_IN\_3[gCm^{-2}]-\Delta C_{LI}\_DW\_5[gCm^{-2}] \quad \text{Eq. 38}$$

2.26 Annual carbon flux in below ground biomass ($\Delta C_{BB}$) in **2020** per land use category (*LUi*) is calculated by the following:

$$\Delta C_{BB}\_LUi[gCm^{-2}] = \Delta C_{BB}\_IN\_3[gCm^{-2}] - \Delta C_{LI}\_FR\_3[gCm^{-2}]$$
$$- \Delta C_{LI}\_CR\_4[gCm^{-2}] - \Delta C_{DW}\_CR\_2[gCm^{-2}] \quad \text{Eq. 39}$$

2.27 Annual carbon flux in below ground biomass ($\Delta C_{AB}$) in **2018** per land use category (*LUi*) is calculated by the following:

$$\Delta C_{AB}\_LUi[gCm^{-2}] = \Delta C_{AB}\_IN\_3[gCm^{-2}] - \Delta C_{LI}\_LF\_1[gCm^{-2}]$$
$$- \Delta C_{LI}\_ST\_2[gCm^{-2}] - \Delta C_{DW}\_ST\_1[gCm^{-2}] \quad \text{Eq. 40}$$

2.28 Reclassify the land cover map in **2032** to the LULUCF/AFOLU definitions described in **Table 1.**

2.29 Implement Equation 2 in **2030.**

2.30 Total carbon flux for all land use types in all AFOLU in **2034** is calculated by Equation 1.

3.0 Science Plan-Directions 2: Application of a dynamic ecosystem model to develop Allometric Equations 1C for wood carbon (i.e., biomass stocks) variables.

3.1 This is a general method to incorporate dynamic process modeling with remote sensing techniques to quantify wood carbon stocks. The newly developed wood carbon storage allometrics are implemented to model remote sensing-derived woody biomass stocks and/or carbon storage in wood. Carbon stored in wood is used to quantify $CO_2$ storage in wood.

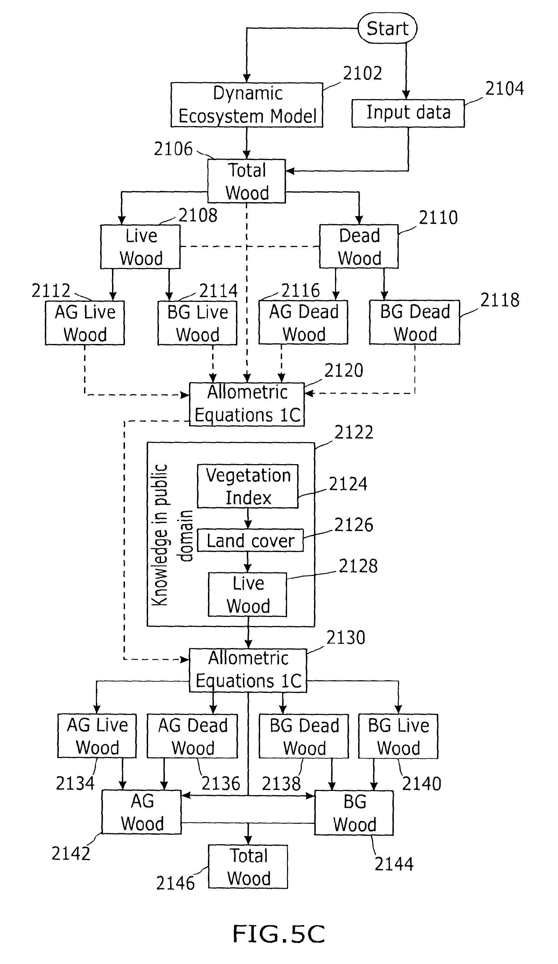

3.2 **FIG. 5C** shows an example of the process used to develop Allometric Equations 1. **FIG. 5C** is used with Science Plan-Directions 2 in section 3 in the example of a science plan. **FIG. 5C** is an example of a flow chart used to develop Allometric Equations 1 for woody biomass stocks and implement the allometrics with remote sensing imagery. More particularly, **FIG. 5C** is a flow chart of the process used to measure standing biomass stocks (i.e., total wood). The steps taken in our carbon model are the following:

3.3 A dynamic model is used to calculate annual total wood biomass storage in dry matter in **2106.**

$$LC_i\_TotalDM\_C\_ratio = LC_i(\overline{\chi}TotalWood[tDMha] / \overline{\chi}TotalWood[tCha]) \quad \text{Eq. 41}$$

Where $LC_i$ is each land cover class (i); $\overline{\chi}TotalWood[tDMha]$ is the total wood storage in tonnes of dry matter per hectare for each land cover class; $\overline{\chi}TotalWood[tCha]$ is the total wood storage in tonnes of carbon per hectare for each land cover class; and *TotalDM_C_ratio* is the ratio between total wood storage in tonnes of dry matter per hectare to total wood storage in tonnes of carbon per hectare.

3.4 Next a dynamic ecosystem model (see **FIG. 5C)** is used to calculate annual above-ground live wood biomass storage in dry matter in **2112,** annual below-ground live wood biomass storage in dry matter in **2114,** annual above-ground dead wood biomass storage in dry matter in **2116** and annual below-ground wood dead biomass storage in dry matter in **2118.** Total live wood in **2108** and dead wood in **2110** are summed for above and below ground partitions of total wood. The term live wood means the outer bark, inner bark, cambium and sapwood portions of a tree's physiology. The term dead wood means the heartwood of a tree's physiology.

3.5 Allometric Equations 1C are developed to quantify the proportional relationship between total wood biomass in **2106 ,** total above-ground live wood biomass in dry matter in **2112,** below-ground live wood biomass in dry matter in **2114,** above-ground dead wood biomass in dry matter in **2116** and below ground dead wood biomass in dry matter in **2118.**

$$LC_{i\_}AG\_LW\_ratio = LC_i(\overline{\chi}AG\_LW[tDMha]/\overline{\chi}TotalLW[tDMha]) \qquad \text{Eq. 42}$$

$$LC_{i\_}BG\_LW\_ratio = LC_i(\overline{\chi}BG\_LW[tDMha]/\overline{\chi}TotalLW[tDMha]) \qquad \text{Eq. 43}$$

Where $LC_i$ is each land cover class (i); $\overline{\chi}AG\_LW[tDMha]$ is the mean above ground live wood storage in tonnes of dry matter per hectare for each BGC land cover class; $\overline{\chi}TotalLW[tDMha]$ is the mean total (above and below ground) live wood storage in tonnes of dry matter per hectare for each land cover class; $\overline{\chi}BG\_LW[tDMha]$ is the mean below ground live wood storage in tonnes of dry matter per hectare for each land cover class;
$LC_{i\_}AG\_LW\_ratio$ is the ratio between above ground live wood storage in tonnes of dry matter per hectare to total live wood storage in tonnes of dry matter per hectare; and
$LC_{i\_}BG\_LW\_ratio$ is the ratio between below ground live wood storage in tonnes of dry matter per hectare to total live wood storage in tonnes of dry matter per hectare.

$$LC_{i\_}AG\_DW\_ratio = LC_i(\overline{\chi}AG\_DW[tDMha]/\overline{\chi}AG\_LW[tDMha]) \qquad \text{Eq. 44}$$

$$LC_{i\_}BG\_DW\_ratio = LC_i(\overline{\chi}BG\_DW[tDMha]/\overline{\chi}BG\_LW[tDMha]) \qquad \text{Eq. 45}$$

Where $LC_i$ is each land cover class (i); $\overline{\chi}AG\_LW[tDMha]$ is the mean above ground live wood storage in tonnes of dry matter per hectare for each BGC land cover class;
$\overline{\chi}AG\_DW[tDMha]$ is the mean above ground dead wood storage in tonnes of dry matter per hectare for each BGC land cover class; $\overline{\chi}BG\_LW[tDMha]$ is the mean below ground live wood storage in tonnes of dry matter per hectare for each BGC land cover class;
$\overline{\chi}BG\_DW[tDMha]$ is the mean below ground dead wood storage in tonnes of dry matter per hectare for each BGC land cover class; $LC_{i\_}AG\_DW\_ratio$ is the ratio between above ground dead wood storage in tonnes of dry matter per hectare to above ground live wood storage in tonnes of dry matter per hectare; and $LC_{i\_}BG\_DW\_ratio$ is the ratio between below ground dead wood storage in tonnes of dry matter per hectare to below ground live wood storage in tonnes of dry matter per hectare.

3.6 A remote sensing modeled vegetation index (i.e., leaf area index (LAI), enhanced vegetation index (EVI), and/or normalized difference vegetation index (NDVI)) is used calculate annual live wood mass (see **FIG. 5C).** The method to calculate live wood mass is found in the MODIS MOD 17 algorithm. The grey box in **2122** (see **FIG. 21)** defines the current knowledge in public domain. The dynamic ecosystem model derived allometric equations used to quantify total wood biomass for the following: annual above-ground live wood biomass storage in dry matter in **2134,** annual below-ground live wood biomass storage in dry matter in **2138,** annual above-ground dead wood biomass storage in dry matter in **2136** and annual below-ground wood dead biomass storage in dry matter in **2140.**

$$AG\_LW[tDMha] = LC_{i\_}AG\_LW\_ratio*Tot\_LW[tDMha] \qquad \text{Eq. 46}$$

$$BG\_LW[tDMha] = LC_{i\_}BG\_LW\_ratio*Tot\_LW[tDMha] \qquad \text{Eq. 47}$$

Where $LC_i$ is each land cover class (i); $LC_{i\_}AG\_LW\_ratio$ is the result for Equation 42; $Tot\_LW[tDMha]$ is derived from leaf area index or enhanced vegetation index for total live wood in tonnes of dry matter per hectare; $LC_{i\_}BG\_LW\_ratio$ is the result to Equation 43; $AG\_LW[tDMha]$ is the total above ground live wood storage in tonnes of dry matter per hectare, and $BG\_LW[tDMha]$ is the total below ground live wood storage in tonnes of dry matter per hectare.

$$AG\_DW[tDMha] = LC_i\_AG\_DW\_ratio * AG\_LW[tDMha] \qquad \text{Eq. 48}$$

$$BG\_DW[tDMha] = LC_i\_BG\_DW\_ratio * BG\_LW[tDMha] \qquad \text{Eq. 49}$$

Where $LC_i$ is each land cover class (i); $LC_i\_AG\_DW\_ratio$ is the result for Equation 44; $AG\_LW[tDMha]$ is the total above ground live wood storage in tonnes of dry matter per hectare; $LC_i\_BG\_DW\_ratio$ is the result to Equation 45; $BG\_LW[tDMha]$ is the total below ground live wood storage in tonnes of dry matter per hectare; $AG\_DW[tDMha]$ is the total above ground dead wood storage in tonnes of dry matter per hectare, and $BG\_DW[tDMha]$ is the total below ground dead wood storage in tonnes of dry matter per hectare.

3.7 Annual total above-ground wood biomass storage in dry matter in **2142** and annual total below ground wood biomass storage in dry matter in **2144** are calculated next.

$$AG\_Total[tDMha] = AG\_LW[tDMha] + AG\_DW[tDMha] \qquad \text{Eq. 50}$$

$$BG\_Total[tDMha] = BG\_LW[tDMha] + BG\_DW[tDMha] \qquad \text{Eq. 51}$$

Where $AG\_LW[tDMha]$ is the result to Equation 46; $AG\_DW[tDMha]$ is the result to Equation 48, $BG\_LW[tDMha]$ is the result to Equation 47; $BG\_DW[tDMha]$ is the result to Equation 49; $AG\_Total[tDMha]$ is the total above ground wood storage in tonnes of dry matter hectare, and $BG\_Total[tDMha]$ is the total below ground wood storage in tonnes of dry matter hectare.

3.8 Annual total wood biomass storage in tonnes of dry matter in **2146** is calculated.

$$Total\_wood[tDMha] = AG\_Total[tDMha] + BG\_Total[tDMha] \qquad \text{Eq. 52}$$

Where $AG\_Total[tDMha]$ is the result of Equation 50; $BG\_Total[tDMha]$ is the result of Equation 51; and $Total\_wood[tDMha]$ is the total (above and below ground) wood storage in tonnes of dry matter per hectare.

3.9 Annual total wood carbon storage was calculated.

$$Total\_wood\_C[tCha] = Total\_wood[tDMha] * TotalDM\_C\_ratio \qquad \text{Eq. 53}$$

Where $Total\_wood[tDMha]$ is the result of Equation 52, $TotalDM\_C\_ratio$ is the result of Equation 41; and $Total\_wood\_C[tCha]$ is the total wood carbon storage per hectare.

4.0 Science Plan-Directions 3: Remote sensing processing and statistical analysis of remote sensing imagery prior to modeling vegetation stock attributes with data mining software.

4.1 Science Justification for MODIS imagery and other remote sensing imagery of vegetation:
Above-ground biomass stocks and/or wood carbon storage (i.e., the trunk, branches and other woody elements of a tree) in theory will change very little between years in undisturbed conditions, except by increasing from vegetation growth and/or annual re-growth. Substantial change in biomass stocks, especially decreases in biomass stocks, is due to disturbance and human impact (i.e., deforestation and degradation). After disturbance, green-up ensues in natural regeneration. The IPCC Degradation Report defined forest degradation as: "A direct human-induced long-term loss (persisting for $X$ years or more) of at least $Y\%$ of forest carbon stocks [and forest values] since time $T$ and not qualifying as deforestation or an elected activity under Article 3.4 of the Kyoto Protocol (IPCC, 2003b, p. 16)."

The IPCC definition for forest degradation requires a regression for annual carbon stocks on the y-axis and time in years along the x-axis. Inter-annual vegetation growth (i.e., NPP) over time can be highly variable and dependent on a number of factors, such as climate, natural disturbance such as grazing by wild herbivores and human impact (i.e., deforestation and forest degradation). However, when assessing inter-annual change for woody biomass stocks (.i.e., biomass and/or carbon storage), the remote sensing measurements between years should have a low percentage (i.e., in the single digits) for coefficient variation when there is no human impact. When variability is high, the vegetation attribute most likely captured by the variability is growth rather than stocks. Therefore, assessing for the least amount of coefficient of variation for remote sensing measurements, prior modeling vegetation stock variables, should be indicative of the best practical remote sensing variable to measure above ground biomass stocks. This also relates to the IPCC definition for forest degradation, because a low percentage for coefficient of variation reverse engineers a regression line between years with little to no change in carbon stocks. This means that the best estimate for inter-annual remote sensing estimates of vegetation woody biomass stocks will be determined by the remote sensing variable with the lowest inter-annual coefficient of variation. Fundamentally, the theoretical approach for monitoring a vegetation attribute found herein is rooted in ecological theory related to assessment of multiple stable states for a target biophysical element over time and space. Applied to remote sensing techniques, each pixel in the remote sensing image represents a unique state (i.e., a quantitative amount) of the target biophysical element (i.e., biomass stocks) at one point in time. The unique state of the biophysical element should be relatively stable over time except for the cycling between disturbance and growth/re-growth. This also means a very small coefficient of variation should be found for the average stable state measurement of the target biophysical element across ecosystems between years when there is no and/or little disturbance. When there is a negative annual trend for the vegetation attribute over time, a disturbance hotspot is indicated by a negative annual trend in the pixel over time. This will also indicate degradation of the target bio-physical element for the monitoring period.

4.2 Justification of MODIS class imagery:

MODIS imagery is used as an example because the raw data is freely available at multiple spatial resolutions and at 8-day temporal replication with long-term planned data continuity. The reason MODIS class imagery is preferred over higher resolution imagery, such as Landsat class imagery, is because MODIS imagery better represents fundamental ecological assessment of wall-to-wall vegetation attributes over both time and space with a global reach and temporal replication on an annual basis. Alternatively, Landsat style analyses better represents the geography of map-making related to change in land cover with a classified spatial value and temporal replication with global reach on a decadal or bi-decadal increment. Global reach is necessary to develop a standardized product that can be used by everyone. Since the IPCC's definition of forest degradation require annual monitoring of carbon stocks in a regression against time, Landsat class imagery cannot be used to monitor forest degradation on a global basis.

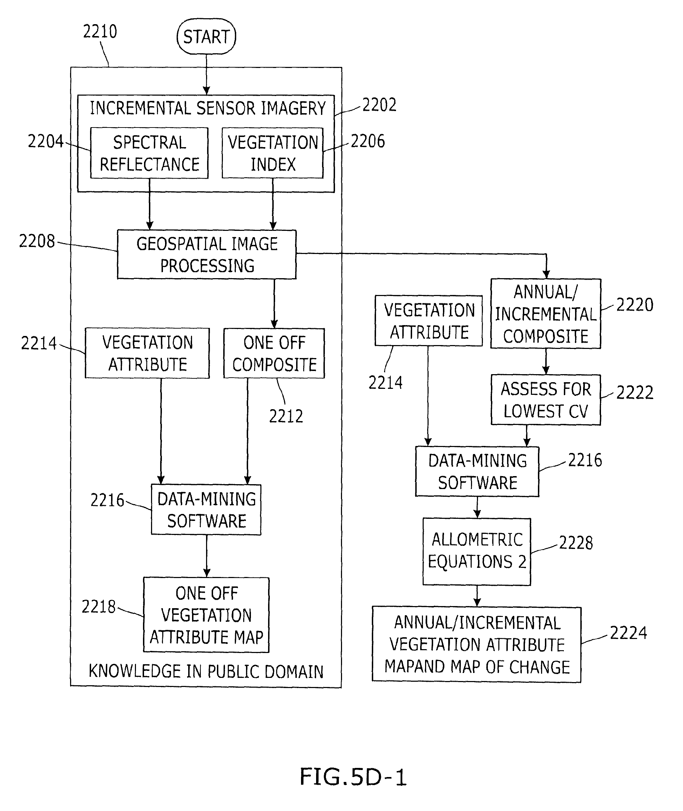

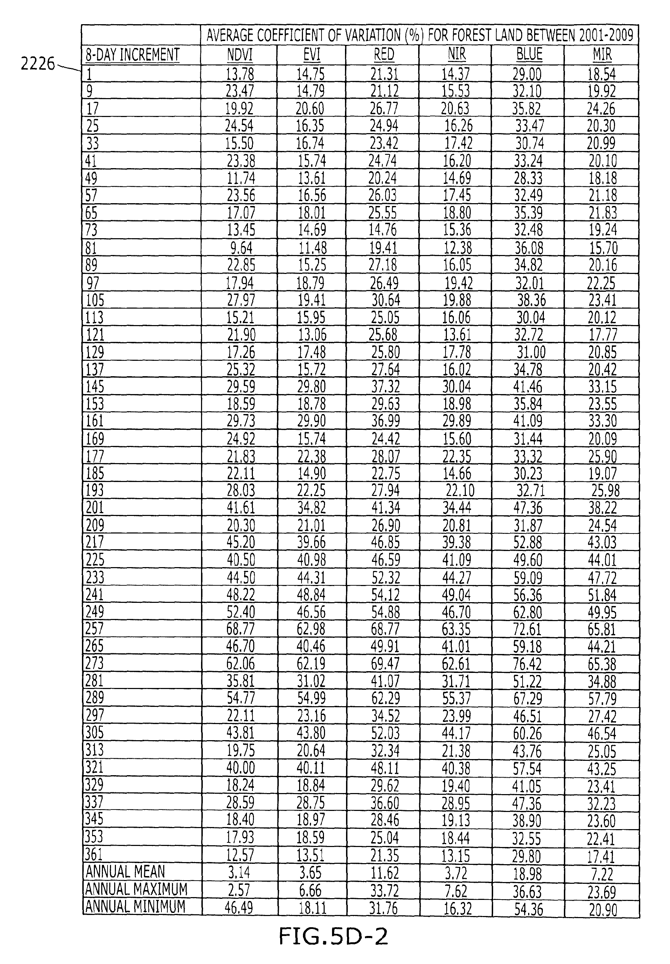

4.3 **FIG. 5D** shows an example of the process used to develop Allometric Equations 2. **FIG. 5D** is used with Science Plan-Directions 3 in section 4 in the example of a science plan. **FIG. 5D** is an example of a flow chart used to develop Allometric Equations 2 for stocks of vegetation attributes and develop allometrics with data mining software. More particularly, **FIG. 5D** provides a schematic used for image processing to determine stocks of vegetation attributes:

4.3.1 In **2202,** a variety of incremental (meaning more than 1 imager per year) sensor imagery is used, such as from MODIS sensor imagery at various spatial resolutions, such as at 1 km x 1 km, 500m x 500m and 250m x 250m resolutions. The primary imagery used is either spectral reflectance in **2204** or a vegetation index in **2206,** but any of following products can also be used: 1) MOD/MYD/MCD 15 8-day fraction of Absorbed Photosynthically Active Radiation (fPAR), 2) MOD/MYD/MCD 15 8-day Leaf Area Index (LAI) and 3) MOD/MYD 13 8-day Enhanced Vegetation Index (EVI), and 4) MOD/MYD 13 8-day Normalized Difference Vegetation Index (NDVI), and 5) MOD/MYD 13 8-day Red reflectance, 6) MOD/MYD 13 8-day near-infrared reflectance, 7) MOD/MYD 13 8-day mid-infrared reflectance, MOD/MYD 13 8-day blue reflectance and 8) MCD 43 8-day Nadir BRDF-Adjusted Reflectance (NBAR) for all 7 bands. All MOD MODIS imagery is for the period 2001 - present and all MODIS MYD imagery is from about mid-2000.

4.3.2 Images are first processed in **2208** for pixel reliability and quality so as to determine highest quality pixels in the image and to remove low quality pixels (i.e., cloud cover). Raw MODIS imagery has a sub-set with a quality control band(s). For example, MOD/MYD 13 has two quality control sub-sets (band 3 and 12) that are used to assess pixel quality and remove low quality pixels. The gray box in **2210** shows the current knowledge in public domain. Stocks are processed to create a one off composite in **2212.** The one off composite is then used in data mining software in **2216** with vegetation attribute data in **2214** to model a one off biomass map in **2218.** "One off" meaning a map of stocks that does not have the capability of reproducing stock maps on an incremental basis, such as an annual stock map for multiple years required to fulfill the IPCC definitions of forest degradation and devegetation to determine Y% change over a period of time.

4.3.3 The imagery processed in **2208** (see **FIG.** 5C) are next processed in **2220** for the following qualitative statists per pixel in the image: 1) annual mean, 2) annual maximum, 3) annual minimum, 4) annual medium, 5) annual standard deviation.

4.3.4 The inter-annual coefficient of variation is used in **2222** to assess for both daily and annual qualitative statistics for the following examples: 1) MOD/MYD/MCD 15 8-day fraction of Absorbed Photosynthically Active Radiation (fPAR), 2) MOD/MYD/MCD 15 8-day Leaf Area Index (LAI) and 3) MOD/MYD 13 8-day Enhanced Vegetation Index (EVI), and 4) MOD/MYD 13 8-day Normalized Difference Vegetation Index (NDVI), and 5) MOD/MYD 13 8-day Red reflectance, 6) MOD/MYD 13 8-day near-infrared reflectance(NIR), 7) MOD/MYD 13 8-day mid-infrared reflectance (MIR), MOD/MYD 13 8-day blue reflectance and 8) MCD 43 8-day Nadir BRDF-Adjusted Reflectance (NBAR) for all 7 bands.

4.3.5 In **2222** (see **FIG. 5C),** the coefficient of variation is also sampled for either 1) a land cover map showing a minimum of AFOLU defined land cover classes and/or 2) a point file for georeferenced field plots of known biomass and/or a land classification. In **2226,** the results for the analysis of average coefficient of variation between 2001-2009 are provided for forest land at MODIS grid tile h19v09 for MOD/MYD 13 8-day, annual mean, annual max and annual min NDVI, EVI, RED, NIR, BLUE and MIR band composites. Note that the 8-day coefficient of variation is highly variable. The least variability is in annual mean and max NDVI and mean EVI. The annual mean and max NDVI and mean EVI will be used in the example of modeling annual biomass stocks with data mining software.

4.4 After 4.3 has been completed to determine the most appropriate band(s) to use to model biomass stocks in **2212,** a point file for georeferenced field plot will be overlaid on the bands and sampled for the pixel in which the point falls. The data will then be uploaded into a data-mining software in **2216.** The data-mining software is used to generate Allometric Equations 2 in 2228 and annual maps of carbon stocks in **2224.**

5.0 Science Plan-Directions 4: Modeling vegetation attributes with a Random Forests model in a data mining software:

5.1 Chapter 5.7 of IPCC GPG-LULUCF (2003a) described the use of remote sensing data and image products as an Approach 4b (p. 5.67) to measure above-ground biomass stored in wood. The chapter stated (p 5.67): "Satellite remote sensing and its image products may also be appropriate for assessing biomass and biomass changes at the major ecosystem level (e.g., grassland vs. forest). Carbon stocks in forests can be estimated using correlations between spectral image data and biomass, provided that adequate data (not used for inventory estimates) are available to represent the range in forest biomes and management regimes for which estimates are required. Correlation equations, may be affected by several parameters (canopy and understorey type, season, illumination, satellite-viewing geometry), and must in general be developed for each forest type. In addition, vegetation indices (e.g., the Normalised Difference Vegetation Index, NDVI) have also been used for the estimation of above ground biomass. The correlation equations referenced above between a measurement from a remote sensing image and a physical measurement of biomass is an allometric equation.

5.2 Justification for MODIS imagery:
Baccini et al (2008) used 7 reflectance bands from MODIS MCD 43 NBAR imagery at 1 km x 1 km resolution to develop a Random Forest model with above ground biomass sampled from field observations. The biomass map they developed was a mosaic of best quality observations over a multi-year period. The field observations also came from different years, some outside the period observed with the NBAR imagery, so there may be a temporal mismatch when regressing the field observations with remote sensing imagery. The ultimate result of Baccini et al's work is a one-off biomass map. The issue with this one-off map is that it has no practical application to meeting the annual monitoring and reporting requirements for either the IPCC GPGs or any of the carbon trading mechanisms. This is because annual maps are needed to 1) develop a baseline assessment of a project site and 2) repeat monitoring annual after a project is validated to show that the carbon amount is still there. Hence, the results of Science Plan-Directions 3 are used in Science Plan-Directions 4 to determine which remote sensing variables would be best applied to monitoring inter-annual above ground biomass stocks and their annual change.

5.3 Sampling remote sensing imagery with the point file of field observation coordinates is completed for annual composites of qualitative statistics (annual mean, maximum, minimum, and medium) with the following imagery: 1) MOD/MYD/MCD 15 8-day fraction of Absorbed Photosynthically Active Radiation (fPAR), 2) MOD/MYD/MCD 15 8-day Leaf Area Index (LAI) and 3) MOD/MYD 13 8-day Enhanced Vegetation Index (EVI), and 4) MOD/MYD 13 8-day Normalized Difference Vegetation Index (NDVI), and 5) MOD/MYD 13 8-day Red reflectance, 6) MOD/MYD 13 8-day near-infrared reflectance, 7) MOD/MYD 13 8-day mid-infrared reflectance, MOD/MYD 13 8-day blue reflectance and 8) MCD 43 8-day Nadir BRDF-Adjusted Reflectance (NBAR) for all 7 bands.

5.4 The composites with the lowest coefficient of variation (i.e., at least in single percentages) are used, such as in section 4.3.5.

5.5 A two tiered approach is used to determine which annual composite is used to develop the Random Forests model:

Tier 1: If the field observation(s) are in a year that corresponds directly with the remote sensing composites (i.e., the field observation(s) were sampled in 2009) than the corresponding annual composite(s) from that year is used (i.e., mean annual EVI for 2009).

Tier 2: If the field observation are not in a year that corresponds directly with the remote sensing composites (i.e., the field observation(s) were sampled in 1995), from a period of time that represents a period of multiple years (i.e., one image for the period 2000-2005), and/or unknown, than the mean of the corresponding annual composite(s) is used (i.e., mean annual EVI between 2001-present).

Tier 1 supersedes Tier 2 in this context.

5.6 The data from the remote sensing composite and the field observations are then used as input data for the Random Forest model.

5.7 The Random Forest model is used to model a minimum of 200 decision trees that are in turn used as predictors in the model. 200 decisions trees is normally the smallest suggested amount of decisions trees to grow for the Random Forest model. Any amount of decision trees over 200 can also be run. The model is validated and verified with a percentage of geospatial data withheld from the training model.

5.8 The full remote sensing annual composites are used as input data in the Random Forest model. The decision tree predictors developed in 5.6 are used to score/model vegetation attributes for the full annual remote sensing composites for each temporal replicate in the incremental time series (i.e., for each year).

5.9 Regression analysis is completed for the annual maps of vegetation attribute(s) over time to assess temporal change in the vegetation attribute over space as a map.

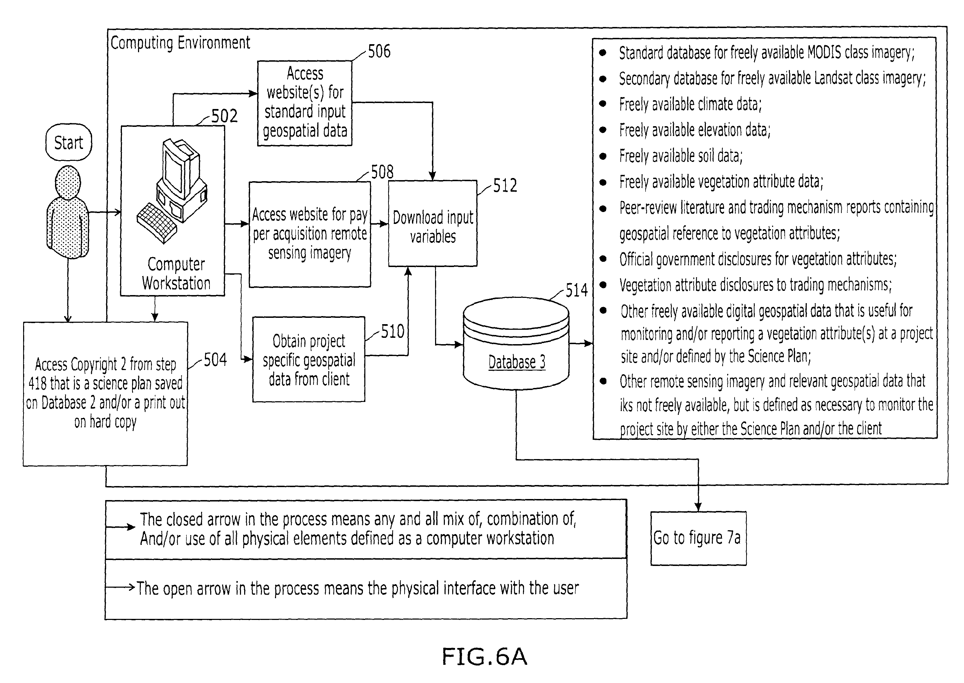

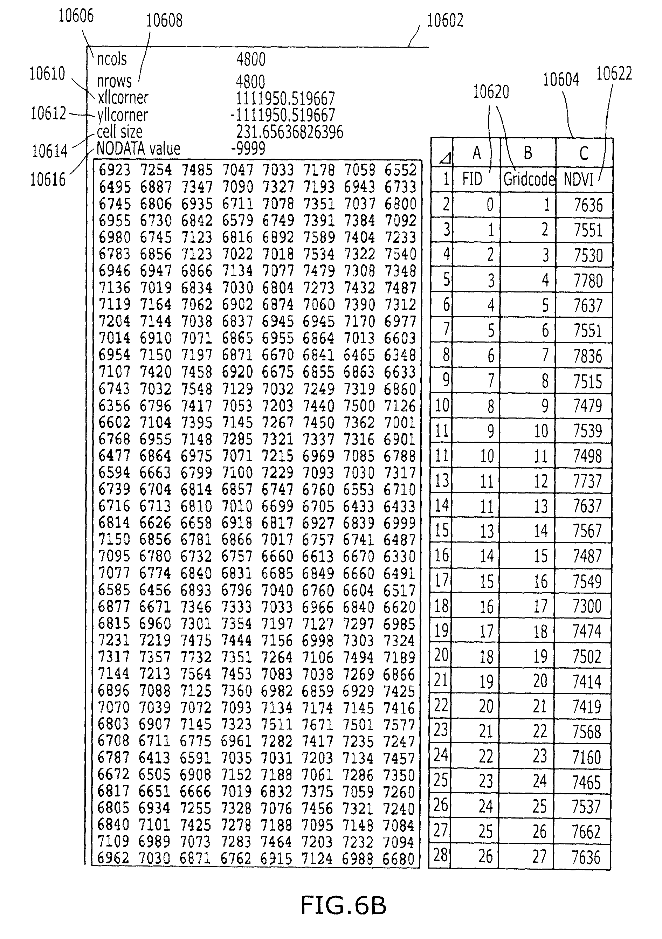

FIG. 6A shows an example for the generic process used to obtain the primary database for input data relevant to the science plan with an example of obtaining the MODerate Resolution Imaging Spectroradiometer (MODIS) remote sensing imagery data referred to in the example of the science plan from **10418.** A user accesses a computer workstation in **502** that includes a screen display(s), processor(s), hard drive(s), a keyboard, a mouse, a router connected to the internet and other physical elements related to a computer workstation, etc. A computer workstation **502** in this context can store, retrieve, process and/or output data and/or communicate with other computers. Copyright 2 in **10418** is viewed by the user in **504** as either a printed out hard copy and/or accessed in Portable Document Format (i.e, .pdf and/or similar file format) by a computer workstation in **502.** The computer workstation is used to access the websites of relevant standard input geospatial data in **506.** Examples of web-sites where MODIS and/or Landsat imagery can be downloaded are the following: <URLs: https://lpdaac.usgs.gov/lpdaac/get_data; https://wist.echo.nasa.gov/wistbin/api/ims.cgi?mode=MAINSRCH&JS=1; https://lpdaac.usgs.gov/lpdaac/get_data/data pool>. Examples of standard MODIS products that are downloaded and stored on Database 3 include any and/or all of the following: MCD45A1, MOD09GA, MYD09GA, MOD09GQ, MYD09GQ MOD09CMG, MYD09CMG, MOD09A1, MYD09A1, MOD09Q1, MYD09Q1, MOD13A1, MYD13A1, MOD13A2, MYD13A2, MOD13Q1, MYD13Q1, MOD13A3, MYD13A3, MOD13C1, MYD13C1, MOD13C2, MYD13C2, MOD44W, MOD11_L2, MYD11_L2, MOD11 A1, MYD11 A1, MOD11 A2, MYD11 A2, MOD11 B1, MYD11 B1, MOD11 C1, MYD11 C1, MOD11 C2, MYD11 C2, MOD11C3, MYD11C3, MOD14, MYD14, MOD14A1, MYD14A1, MOD14A2, MYD14A2, MCD15A2, MOD15A2, MYD15A2, MOD17A2, MYD17A2, MCD43A3, MCD43B3, MCD43C3, MCD43A1, MCD43B1, MCD43C1, MCD43A2, MCD43B2, MCD43C2, MCD43A4, MCD43B4, MCD43C4, MOD12Q1, MCD12Q1, MOD12Q2, MCD12Q2, MOD12C1, MCD12C1, MOD44B. The websites for freely available climate data are accessed in **506.** Examples of freely available climate data (past, present and/or future) that is stored on Database 3 include the World Meteorological Organization's geospatial data, the University of East Angelia Climate Research Unit's publically available gridded climate data, and the NCEP-NCAR publically available gridded climate data. The websites for freely available elevation data are accessed in **506.** An example of elevation data that is stored on Database 3 is from the Shuttle Radar Topography Mission (SRTM). The websites for freely available soil data is accessed in **506.** An example of soil data that is stored on Database 3 is the ISRIC-WISE revised soil property estimates for soil types of the world. The websites for freely available vegetation attributes is accessed in **506.** An example of vegetation attribute data that is stored on Database 3 is FLUXNET data and associated sites. The websites for publishers of peer-review journal articles are accessed in **506.** Examples of these website's are ScienceDirect and Wiley InterScience. The websites for the publishers of peer-review journal articles have internal key word search engines. The website key word search engines are used to search for the specific vegetation attribute (i.e., "biomass") and key word that reference a geographical coordinate (i.e., "latitude" and "longitude"). Any publications related to any input data and/or the use of any input data and/or the use of any computer implemented software to fulfill the science and/or methods disclosed herein are also downloaded. The retrieved files are downloaded and stored on Database 3. The websites of regulated and voluntary trading mechanisms are accessed in **506.** Project developers that develop a project site for an offset related to a vegetation attribute must supply documents that report vegetation attributes to the trading mechanism. The reporting documents are normally in the form of Project Design Documents, Project Validation Documents and Project Verification Documents and are publically disclosed on the trading mechanism website. The reporting documents from trading mechanisms are downloaded and stored on Database 3. The downloaded peer-review articles and/or reporting documents from trading mechanisms are then accessed with a text retrieval software and a key word search is performed similar to the process of described in **FIG. 4B**. The Level 1 key words used in the text retrieval software are for a vegetation attribute (i.e., "biomass"), a numerical amount for all referenced vegetation attributes, the measurement system (i.e., "tC ha") and the latitude and longitude coordinates. Any additional key words deemed useful by the user are added to a Level 2 key word search. Tables are also retrieved in any of the search levels when the tables are described by a key word. This retrieved information is outputted to a spreadsheet that is stored on Database 3. The Level 1 and Level 2 key words are stored on a meta-database. The aforementioned process is only used on documents when there is no disclaimer limiting the use of the document and/or use of the contents of the document in certain ways described herein. The websites for freely available official governmental disclosures for vegetation attributes are accessed in **506.** An example of such a website is the USDA Forest Service Geodata Clearinghouse. Examples of government disclosures for geospatial data of a vegetation attribute stored on Database 3 is Forest Service's biomass maps for the Contiguous US, Alaska and Puerto Rico. Other freely available digital geospatial information that is internet accessible and useful for monitoring and/or reporting vegetation attribute(s) at a project site and/or defined by future science plans are stored on Database 3. In **508,** the websites for remote sensing images that are not free are accessed after direction from the client and access to the remote sensing imagery is acquired, downloaded in **512** and stored on Database 3 in **514.** Geospatial data for vegetation attributes specifically related to the client's project site are obtained in **510** through an internet interface (i.e., email and/or a website) and downloaded in **512** by the computer workstation and stored on Database 3 in **514.** The geospatial data is obtained electronically from the client and/or an agent acting on behalf of the client, either: electronically transmitting, sending, emailing and/or uploading the geospatial data through an internet interface (i.e., email and/or a web-site interface). All geospatial data stored on Database 3 in **514** is stored in standardized raster files (i.e., .hdf, .tif, .img, etc. files) and/or a spreadsheet (i.e., .txt, .dbf, .html, .csv, etc. files) that can be georeferenced and/or assigned a grid code coordinate and/or as a vector file (i.e., a shapefile with associated files). **FIG. 6B** shows an example of a georeferenced spreadsheet in 10602 and a spreadsheet with an assigned grid code coordinate in **10604.** In **10602,** the arrows point to the following: columns in **10606;** rows in **10608;** x and y corners in **10610** and **10612,** respectively; pixel cell size in **10614,** no data value in **10616** and the box in **10618** shows the raw data. In **10604,** a grid code is pointed to in either of the examples in **10620,** where a unique gridded value is assigned to the individual pixel information in **10622** for the whole remote sensing image.

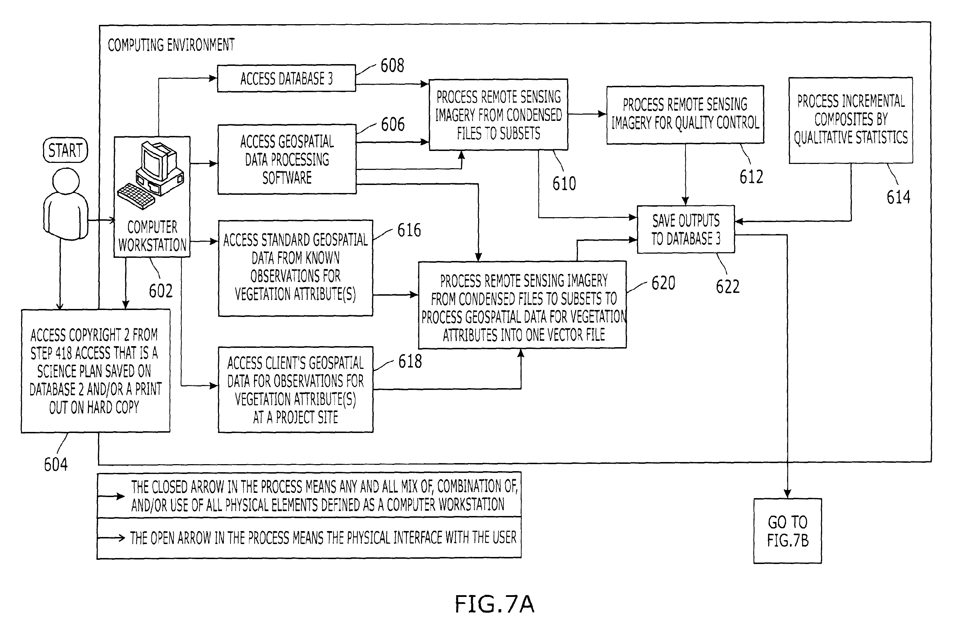

After the standard MODIS products are downloaded, the raw download imagery files are not directly useful for monitoring and/or reporting a project site, because multiple image bands are condensed on one Hierarchical Data Format file (i.e., .hdf file), the individual pixels in an image may be degraded in quality by cloud cover and other data distortions, and the imagery is in 8-day temporal replicates that need to be processed, for example, to an annual temporal replicates for a certain qualitative statistical amount. Hence, the raw downloaded data needs to be preprocessed to prepare the data for use with the directions in the Science Plan. **FIG. 7A** shows the generic process used to pre-process remote sensing imagery in Database 3. A user accesses a computer workstation in **602** that includes a screen display(s), processor(s), hard drive (s), a keyboard, a mouse, a router connected to the internet and other physical elements related to a computer workstation, etc. A computer workstation **602** in this context can store, retrieve, process and/or output data and/or communicate with other computers. Copyright 2 in **418** is viewed in **604** as either a printed out hard copy and/or accessed in Portable Document Format (i.e, .pdf and/or similar file format) by a computer workstation in **602.**

The computer workstation is used to access a geospatial data processing software in **606** that has the ability to process geospatial data in line with and fulfill the science plan in **418.** Examples of geospatial data processing software are the following: ArcView/GIS, Erdas Imagine, Envi, Idrisi, Quantum GIS, Grass, and Land Change Modeler and/or other relevant image processing software, etc., that is copyrighted and/or copyrightable and/or in open access. The geospatial data processing software is stored on the computer workstation hard drive and installed on the work station. The computer workstation is used with the geospatial data processing software to access Database 3 in **608.** The geospatial data processing software is used to sub-set files stored on Database 3 in **610** that are in a condensed file format (i.e., an image file with more than one band per image on the file). After the files are sub-setted, the geospatial data processing software is used in **612** with the Quality Control sub-set to remove low quality and/or contaminated pixels in the imagery. The geospatial data processing software is next used in **614** to develop qualitative statistical composites (i.e., mean, minimum, maximum, medium, standard deviation, etc) for daily, weekly and/or every 8-day, 10-day, 15-day, monthly, and/or annual increments. After the qualitative statistical composites are processed, the different remote sensing imagery and/or bands may be mixed and/or combined to create an index, such as a photochemical chemical index. In **616,** the standard geospatial data for vegetation attribute(s) is accessed. The standard geospatial data for vegetation attributes can be any data described in Database 3, except data that is obtained under a confidential agreement with a client. In **618,** the client's geospatial data for a know vegetation attribute is accessed from Database 3. The client's data is kept separate from the standard data because of confidentially agreements. In **620,** any and/or all information for the vegetation attribute is processed with geospatial data processing software to create a point vector file for each at the geographical coordinates of the vegetation attribute(s). The outputs from **610, 612, 614** and **620** are stored on Database 3 in **622** in standardized raster files (i.e., .hdf, .tif, .img, etc. files) and/or a spreadsheet (i.e., .txt, .dbf, .html, .csv, etc. files) that can be georeferenced and/or assigned a grid code coordinate and/or a geospatial vector file (i.e., a shapefile with associated files). All raster files stored on Database 3 may be processed, merged and/or placed into a mosaic and/or standardized to the WGS-84 geographical coordinate system.

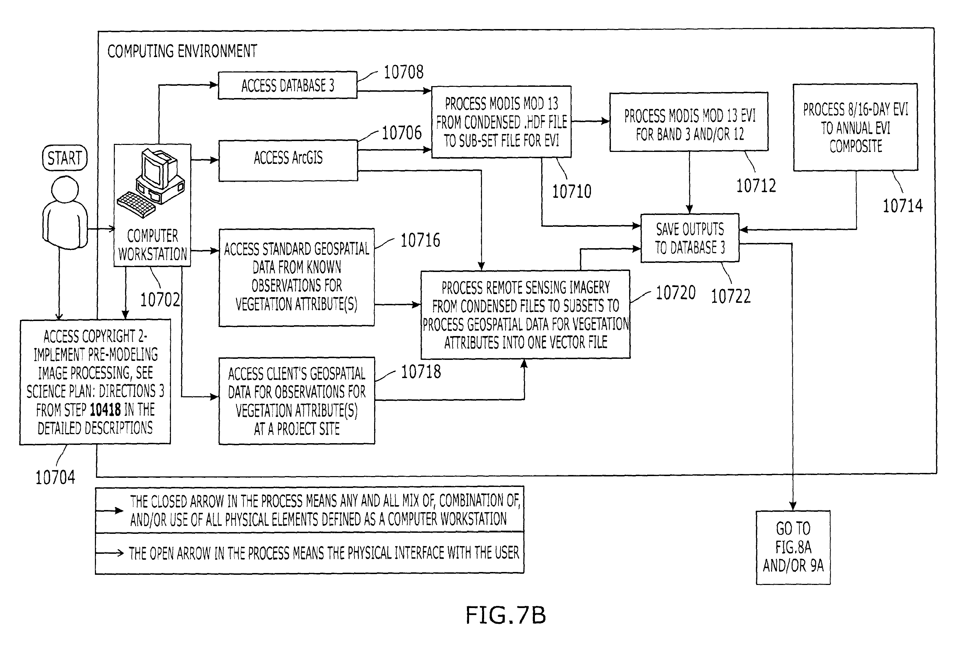

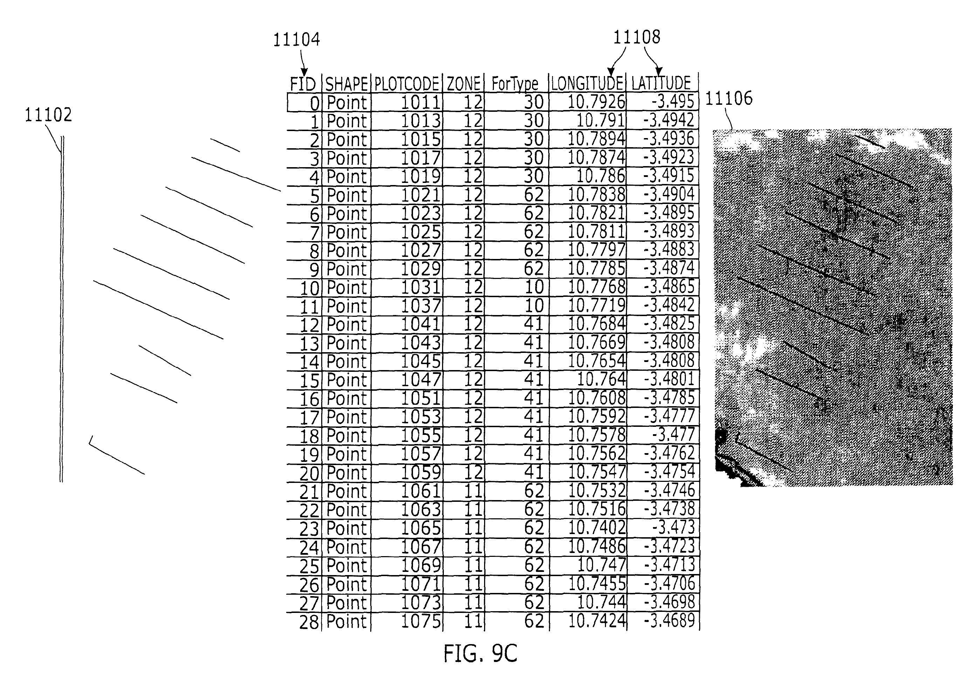

**FIG. 7B** shows an example for the generic process used to pre-process remote sensing imagery in the primary database applied to MODIS MOD/MYD 13 from **FIG. 105.** A user accesses a computer workstation in **10702** that includes a screen display(s), processor(s), hard drive(s), a keyboard, a mouse, a router connected to the internet and other physical elements related to a computer workstation, etc. A computer workstation **10702** in this context can store, retrieve, process and/or output data and/or communicate with other computers. Copyright 2 from **10418** is viewed in **10704** as either a printed out hard copy and/or accessed in Portable Document Format (i.e, .pdf and/or similar file format) by a computer workstation **10702.** The computer workstation is used to access a geospatial data processing software, ArcGIS in **10706.** The computer workstation is used with ArcGIS to access Database 3 in **10708.** The geospatial data processing software is used in 10710 to sub-set files stored on Database 3 that are in a condensed file format. For example, MOD 13 is downloaded in a condensed Hierarchical Data Format (i.e., .hdf format). MOD 13 in hdf format stores a variety of sub-sets, including Normalized Difference Vegetation Index (NDVI), Enhanced Vegetation Index (EVI), Red, Blue, Near-Infrared, Mid-Infrared, VI Quality, Pixel Reliability, etc. After the files are processed for sub-sets, ArcGIS is used in **10712** with the Pixel Reliability sub-set and/or VI Quality sub-set to remove low quality and/or contaminated pixels in the image, such as removing pixels with cloud cover from an EVI image. ArcGIS is next used in **10714** to develop qualitative statistical composites (i.e., mean, minimum, maximum, medium, standard deviation, etc) for daily, weekly and/or every 8-day, monthly, and/or annual increments. For example, all 8-day MODIS MOD/MYD EVI image composites from the year 2009 can be processed into one image for the mean annual EVI per pixel in 2009. In **10716,** standard geospatial data from known observations stored on Database 3 are accessed, such as the Forest Service's biomass maps in a raster file for the Contiguous US, Alaska and Puerto Rico. These raster files are processed with ArcGIS in **10718** to convert the pixels in the raster file to a point in a vector file. In **10718,** a clients geospatial data is accessed from Database 3. The table file with the vegetation attributes is processed in ArcGIS in **10720** to a vector file with a point at the geographical location (i.e., longitude and latitude) for each field observation of a vegetation attribute. An example of the vector file output in **10720** is shown in **11102** from **FIG. 9C.** This is an example of over 500 field plots obtained from SFM-Africa. Each point in **11102** is indicative of a field observation for a vegetation attribute and a geographical coordinate. In **11104** from FIG. 9C, the arrows in 11108 show the longitude and latitude for each point in **11102.** The outputs of **10710, 10712, 10714,** and **10720** are stored on Database **3** in **10722** in standardized raster files (i.e., .hdf, .tif, .img, etc. files) and/or a spreadsheet (i.e., .txt, .dbf, .html, .csv, etc. files) that can be georeferenced and/or assigned a grid code coordinate and/or a geospatial vector file (i.e., a shapefile with associated files). All raster files stored on Database 3 may be processed, merged and/or placed into a mosaic and/or standardized to the WGS-84 geographical coordinate system.

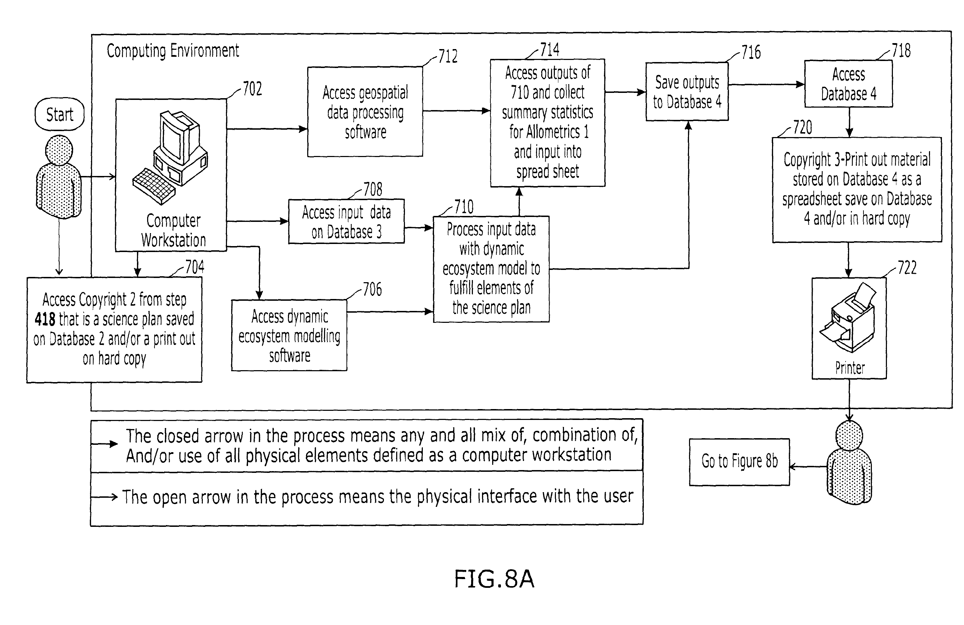

**FIG. 8A** shows the generic process used to develop Allometric Equations 1. A user accesses a computer workstation in **702** that includes a screen display(s), processor(s), hard drive(s), a keyboard, a mouse, a router connected to the internet and other physical elements related to a computer workstation, etc. A computer workstation **702** in this context can store, retrieve, process and/or output data and/or communicate with other computers. The science plan from **418** is viewed in **704** as either a printed out hard copy and/or accessed in Portable Document Format (i.e., .pdf and/or similar file format) by a computer workstation in **702.** The computer workstation is used to access a dynamic ecosystem modeling software in **706.** A dynamic ecosystem modeling software means a copyrighted and/or copyrightable software that can be used to quantify GHGs emissions, removals and/or other vegetation attributes defined by the science plan in **418.** A dynamic ecosystem model (i.e., a biogeochemical model) may also include any mix and/or combination and/or coupling of a dynamic ecosystem modeling software to one or more other dynamic ecosystem modeling software and/or jointly coupling one or more models to a soil and/or hydraulic model and/or the added coupling to a climate model. The dynamic ecosystem model is stored on the computer workstation hard drive and installed on the work station. The computer workstation is used in **710** to process the dynamic ecosystem model in **706** with input data from **708** stored on Database 3 to fulfill elements of **418.** The outputs of the processing in **710** are stored on Database 4 in **716.** The computer workstation is next used to access a geospatial data processing software in **712.** Examples of geospatial data processing software are the following: ArcView/GIS, Erdas Imagine, Envi, Idrisi, Quantum GIS, Grass, and Land Change Modeler and/or other relevant image processing software, etc., that is copyrighted and/or copyrightable and/or in open access. The image processing software is used to access the outputs of **710** and develop summary statistics that are uploaded into a spreadsheet (i.e., MS Excel) in **714.** The summary statistics are used in **714** to reclassify land cover maps for the summary statistics. The outputs of **714** are stored on Database 4 in **716.** The outputs from **714,** are stored on Database 4 in standardized raster files (i.e., .hdf, .tif, .img, etc. files) and/or a spreadsheet/table and/or a spreadsheet (i.e., .txt, .dbf, .html, .csv, etc. files) that can be georeferenced and/or assigned a grid code coordinate and/or a geospatial vector file (i.e., a shapefile with associated files). The material stored on **716** is accessed in **718** with spreadsheet software (i.e. MS Excel), printed out as a spreadsheet and/or a table and/or an atlas/illustration and defined as Copyright 3 in **720** in either a Portable Document Format (i.e, .pdf and/or similar file format) digital file and/or in hard copy with a printer in 722.

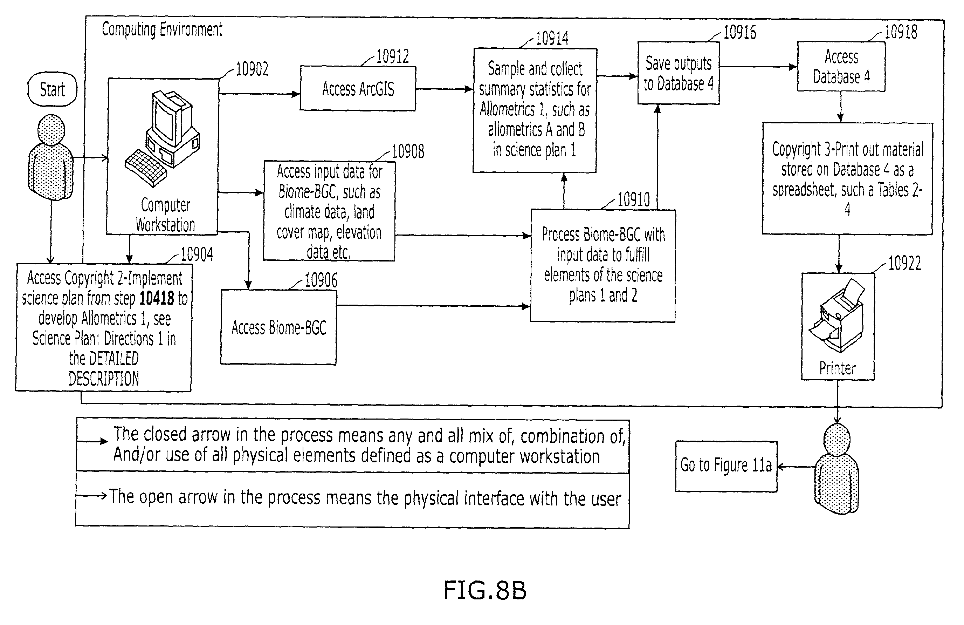

**FIG. 8B** shows an example for the generic process used to develop Allometric Equations 1 applied to dynamic ecosystem model Biome-BGC. A user accesses a computer workstation in **10902** that includes a screen display(s), processor(s), hard drive(s), a keyboard, a mouse, a router connected to the internet and other physical elements related to a computer workstation, etc. A computer workstation **10902** in this context can store, retrieve, process and/or output data and/or communicate with other computers. The elements of Science Plan-Directions 1 in **FIG. 5A from 10418** are viewed in **10904** as either a printed out hard copy and/or accessed in Portable Document Format (i.e, .pdf and/or similar file format) by a computer workstation in **10902.** The computer workstation is used to access Biome-BGC in **10906.** Biome-BGC may also be mixed and/or combined and/or coupled with to one or more other dynamic ecosystem modeling software and/or jointly coupled to a soil and/or hydraulic model (such as Century) and/or coupled to a climate model (such as ANU-Spline). The computer workstation is used in **10910** to process the Biome-BGC in **10906** with input data from **10908** stored on Database 3 (such as various climate data, soil data, elevation data, etc.) to fulfill elements of Science Plan-Directions 1 in **FIG. 5A** from **10418.** The outputs of the processing in **10910** are stored on Database 4 in **10916.** The computer workstation is next used to access ArcGIS in **10912.** ArcGIS is used to access the outputs of **10910** and develop summary statistics for land cover that are uploaded into a spreadsheet (i.e., MS Excel) in **10914.** The summary statistics are used in **10914** to reclassify land cover maps for the summary statistics. The outputs of **10914** are stored on Database 4 in **10916.** The outputs from **10914,** are stored on Database 4 in standardized raster files (i.e., .hdf, .tif, .img, etc. files) and/or a spreadsheet/table and/or a spreadsheet (i.e., .txt, .dbf, .html, .csv, etc. files) that can be georeferenced and/or assigned a grid code coordinate and/or a geospatial vector file (i.e., a shapefile with associated files). The material stored on **10916** is accessed in **10918** with spreadsheet software (i.e. MS Excel), printed out as a spreadsheet and/or a table and/or an atlas/illustration and defined as Copyright 3 in **10920** in either a Portable Document Format (i.e, .pdf and/or similar file format) digital file and/or in hard copy with a printer in **10922. Table 2** defines **Tables 3** and **4. Tables 3** and **4** are examples of outputs from **10922.**

Table 2

| Parameter | Units | Description |
|---|---|---|
| $\overline{\chi}\varDelta C_{NPP}$ | (g C m$^{-2}$) | Mean Net Primary Production |
| $\overline{\chi}\varDelta C_{LF}$ | (g C m$^{-2}$) | Mean partitioning to leaf carbon |
| $\overline{\chi}\varDelta C_{ST}$ | (g C m$^{-2}$) | Mean partitioning to stem carbon |
| $AB_1\_LF\_ratio$ | None | Ratio of leaf carbon to net primary production |
| $AB_2\_ST\_ratio$ | None | Ratio of stem carbon to net primary production |
| $\overline{\chi}\varDelta C_{FR}$ | (g C m$^{-2}$) | Mean partitioning to fine root carbon |
| $\overline{\chi}\varDelta C_{CR}$ | (g C m$^{-2}$) | Mean partitioning to coarse root carbon |
| $BB_1\_FR\_ratio$ | None | Ratio of fine root carbon to net primary production |

(continued)

| Parameter | Units | Description |
|---|---|---|
| $BB_2\_CR\_ratio$ | None | Ratio of coarse root carbon to net primary production |
| $\overline{\chi \Delta C_{DW}}$ | (g C m$^{-2}$) | Mean partitioning to dead wood debris carbon |
| $DW_1\_ST\_ratio$ | None | Ratio of dead wood debris carbon to stem carbon |
| $DW_2\_CR\_ratio$ | None | Ratio of dead wood debris carbon to coarse root carbon |
| $\overline{\chi \Delta C_{LI}}$ | (g C m$^{-2}$) | Mean partitioning to litter carbon |
| $LI_1\_LF\_ratio$ | None | Ratio of litter carbon to leaf carbon |
| $LI_2\_ST\_ratio$ | None | Ratio of litter carbon to stem carbon |
| $LI_3\_FR\_ratio$ | None | Ratio of fine litter carbon to fine root carbon |
| $LI_4\_CR\_ratio$ | None | Ratio of litter carbon to coarse root carbon |
| $LI_5\_DW\_ratio$ | None | Ratio of litter carbon to dead wood debris carbon |
| $\overline{\chi \Delta C_{SO}}$ | (g C m$^{-2}$) | Mean partitioning to soil organic matter carbon |
| $SO_1\_LI\_ratio$ | None | Ratio of soil organic matter carbon to litter carbon |
| $\overline{\chi \Delta C_{HR}}$ | (g C m$^{-2}$) | Mean release to heterotrophic respiration carbon |
| $HR_1\_LI\_ratio$ | None | Ratio of heterotrophic respiration carbon to litter carbon |
| $HR_1\_SO\_ratio$ | None | Ration of heterotrophic respiration carbon to soil organic matter carbon |

Table 3

| Parameter | Biome Classification | | | | | |
|---|---|---|---|---|---|---|
| | ENF | EBF | DNF | DBF | MF | Cshrub |
| $\overline{\chi \Delta C}_{NPP}$ (g C m$^{-2}$) | 470.51 | 1083.45 | 312.54 | 592.49 | 528.38 | 426.72 |
| $\overline{\chi \Delta C}_{LF}$ (g C m$^{-2}$) | 96.79 | 328.23 | 66.41 | 125.90 | 119.78 | 187.84 |
| $\overline{\chi \Delta C}_{ST}$ (g C m$^{-2}$) | 212.94 | 328.23 | 146.10 | 276.97 | 223.69 | 41.32 |
| $AB_1\_LF\_ratio$ (g C m$^{-2}$/ g C m$^{-2}$) | 0.21 | 0.30 | 0.21 | 0.21 | 0.23 | 0.44 |
| $AB_2\_ST\_ratio$ (g C m$^{-2}$/ g C m$^{-2}$) | 0.45 | 0.30 | 0.47 | 0.47 | 0.42 | 0.10 |
| $\overline{\chi \Delta C}_{FR}$ (g C m$^{-2}$) | 96.79 | 328.23 | 66.41 | 125.90 | 122.83 | 187.84 |
| $\overline{\chi \Delta C}_{CR}$ (g C m$^{-2}$) | 63.88 | 98.47 | 33.60 | 63.70 | 61.98 | 12.40 |
| $BB_1\_FR\_ratio$ (g C m$^{-2}$/ g C m$^{-2}$) | 0.21 | 0.30 | 0.21 | 0.21 | 0.23 | 0.44 |
| $BB_2\_CR\_ratio$ (g C m$^{-2}$/ g C m$^{-2}$) | 0.13 | 0.09 | 0.11 | 0.11 | 0.12 | 0.03 |
| Corresponding UMD Land Cover Classification | 1 | 2 | 3 | 4 | 5 | 8 |

| Parameter | Biome Classification | | | | |
|---|---|---|---|---|---|
| | Oshrub | WL | Wgrass | Grass | Crop |
| $\overline{\chi \Delta C}_{NPP}$ (g C m$^{-2}$) | 196.77 | 661.65 | 628.77 | 265.25 | 412.61 |
| $\overline{\chi \Delta C}_{LF}$ (g C m$^{-2}$) | 86.32 | 205.89 | 210.11 | 88.41 | 137.53 |
| $\overline{\chi \Delta C}_{ST}$ (g C m$^{-2}$) | 18.99 | 133.57 | 45.93 | 0.00 | 0.00 |
| $AB_1\_LF\_ratio$ (g C m$^{-2}$/ g C m$^{-2}$) | 0.44 | 0.31 | 0.33 | 0.33 | 0.33 |
| $AB_2\_ST\_ratio$ (g C m$^{-2}$/ g C m$^{-2}$) | 0.10 | 0.20 | 0.07 | 0.00 | 0.00 |
| $\overline{\chi \Delta C}_{FR}$ (g C m$^{-2}$) | 86.32 | 285.35 | 360.75 | 176.81 | 275.05 |
| $\overline{\chi \Delta C}_{CR}$ (g C m$^{-2}$) | 5.70 | 37.06 | 12.30 | 0.00 | 0.00 |
| $BB_1\_FR\_ratio$ (g C m$^{-2}$/ g C m$^{-2}$) | 0.44 | 0.43 | 0.57 | 0.67 | 0.67 |
| $BB_2\_CR\_ratio$ (g C m$^{-2}$/ g C m$^{-2}$) | 0.03 | 0.06 | 0.02 | 0.00 | 0.00 |
| Corresponding UMD Land Cover Classification | 9 | 6 | 7 | 10 | 12 |

Table 4

| Parameter | Biome Classification | | | | | |
|---|---|---|---|---|---|---|
| | ENF | EBF | DNF | DBF | MF | Cshrub |
| $\overline{\chi\Delta C}_{DW}$ (g C m$^{-2}$) | 202.74 | 310.48 | 136.65 | 263.84 | 213.48 | 36.67 |
| $DW_1\_ST\_ratio$ (g C m$^{-2}$/ g C m$^{-2}$) | 0.95 | 0.95 | 0.94 | 0.95 | 0.95 | 0.89 |
| $DW_2\_CR\_ratio$ (g C m$^{-2}$/ g C m$^{-2}$) | 0.95 | 0.95 | 0.94 | 0.95 | 0.95 | 0.89 |
| $\overline{\chi\Delta C}_{LI}$ (g C m$^{-2}$) | 438.73 | 1010.88 | 284.84 | 554.01 | 493.95 | 384.12 |
| $LI_1\_LF\_ratio$ (g C m$^{-2}$/ g C m$^{-2}$) | 0.96 | 0.95 | 0.97 | 0.97 | 0.96 | 0.90 |
| $LI_2\_ST\_ratio$ (g C m$^{-2}$/ g C m$^{-2}$) | 0.003 | 0.002 | 0.002 | 0.002 | 0.002 | 0.009 |
| $BGC\_LI_3\_FR\_ratio$ (g C m$^{-2}$/ g C m$^{-2}$) | 0.96 | 0.95 | 0.97 | 0.97 | 0.96 | 0.90 |
| $LI_4\_CR\_ratio$ (g C m$^{-2}$/ g C m$^{-2}$) | 0.003 | 0.002 | 0.003 | 0.002 | 0.002 | 0.01 |
| $LI_5\_DW\_ratio$ (g C m$^{-2}$/ g C m$^{-2}$) | 0.96 | 0.96 | 0.93 | 0.95 | 0.95 | 0.94 |
| $\overline{\chi\Delta C}_{SO}$ (g C m$^{-2}$) | 229.65 | 537.36 | 145.98 | 289.07 | 258.46 | 206.14 |
| $SO_1\_LI\_ratio$ (g C m$^{-2}$/ g C m$^{-2}$) | 0.52 | 0.53 | 0.51 | 0.52 | 0.52 | 0.54 |
| $\overline{\chi\Delta C}_{HR}$ (g C m$^{-2}$) | 199.94 | 450.27 | 127.54 | 252.91 | 223.52 | 163.55 |
| $HR_1\_LI\_ratio$ (g C m$^{-2}$/ g C m$^{-2}$) | 0.46 | 0.45 | 0.45 | 0.46 | 0.45 | 0.43 |
| $HR_1\_SO\_ratio$ (g C m$^{-2}$/ g C m$^{-2}$) | 1.0 | 1.0 | 1.0 | 1.0 | 1.0 | 0.99 |
| Corresponding UMD Land Cover Class | 1 | 2 | 3 | 4 | 5 | 8 |

| Parameter | Biome Classification | | | | |
|---|---|---|---|---|---|
| | Oshrub | WL | Wgrass | Grass | Crop |
| $\overline{\chi\Delta C}_{DW}$ (g C m$^{-2}$) | 17.18 | 125.71 | 42.78 | 0.00 | 0.00 |
| $DW_1\_ST\_ratio$ (g C m$^{-2}$/ g C m$^{-2}$) | 0.90 | 0.94 | 0.93 | n/a | n/a |
| $DW_2\_CR\_ratio$ (g C m$^{-2}$/ g C m$^{-2}$) | 0.90 | 0.94 | 0.93 | n/a | n/a |
| $\overline{\chi\Delta C}_{LI}$ (g C m$^{-2}$) | 179.98 | 625.37 | 605.09 | 259.32 | 408.24 |
| $LI_1\_LF\_ratio$ (kg C m$^{-2}$/ g C m$^{-2}$) | 0.92 | 0.96 | 0.97 | 0.98 | 0.99 |
| $LI_2\_ST\_ratio$ (g C m$^{-2}$/ g C m$^{-2}$) | 0.009 | 0.003 | 0.003 | n/a | n/a |
| $LI_3\_FR\_ratio$ (g C m$^{-2}$/ g C m$^{-2}$) | 0.92 | 0.97 | 0.97 | 0.98 | 0.99 |
| $LI_4\_CR\_ratio$ (g C m$^{-2}$/ g C m$^{-2}$) | 0.009 | 0.003 | 0.003 | n/a | n/a |
| $LI_5\_DW\_ratio$ (g C m$^{-2}$/ g C m$^{-2}$) | 0.95 | 0.95 | 0.95 | n/a | n/a |
| $\overline{\chi\Delta C}_{SO}$ (g C m$^{-2}$) | 96.44 | 335.38 | 327.73 | 140.63 | 224.69 |
| $SO_1\_LI\_ratio$ (g C m$^{-2}$/ g C m$^{-2}$) | 0.54 | 0.54 | 0.54 | 0.54 | 0.55 |
| $\overline{\chi\Delta C}_{HR}$ (g C m$^{-2}$) | 76.58 | 274.95 | 261.24 | 110.61 | 176.52 |
| $HR_1\_LI\_ratio$ (g C m$^{-2}$/ g C m$^{-2}$) | 0.43 | 0.44 | 0.43 | 0.43 | 0.43 |
| $HR_1\_SO\_ratio$ (g C m$^{-2}$/ g C m$^{-2}$) | 1.0 | 1.0 | 1.0 | 1.0 | 1.0 |
| Corresponding UMD Land Cover Class | 9 | 6 | 7 | 10 | 12 |

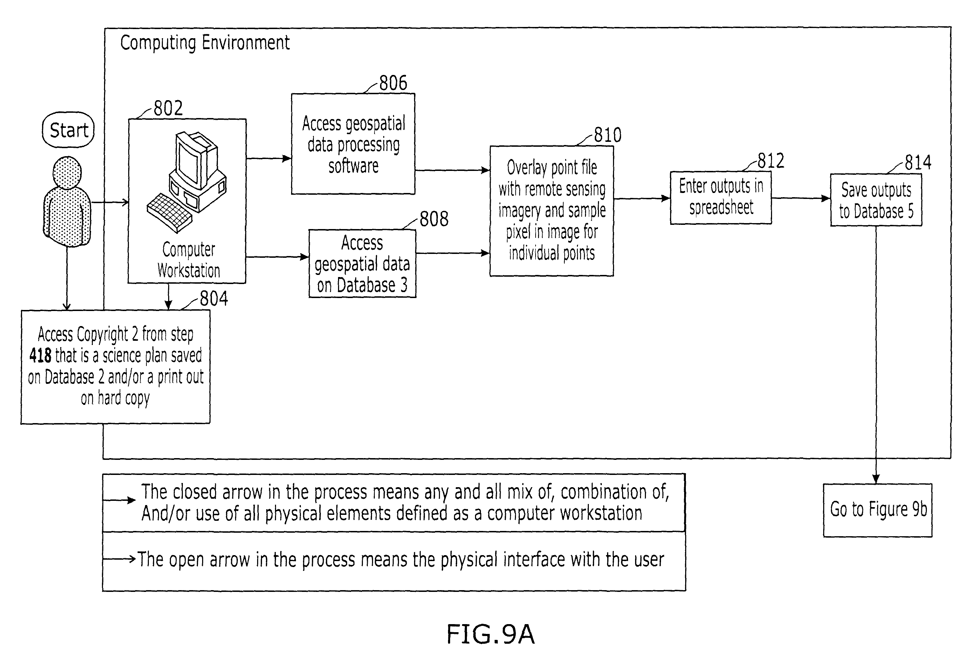

**FIG. 9A** shows the first half of the generic process used to develop Allometric Equations 2. A user accesses a computer workstation in **802** that includes a screen display(s), processor(s), hard drive(s), a keyboard, a mouse, a router connected to the internet and other physical elements related to a computer workstation, etc. A computer workstation **802** in this context can store, retrieve, process and/or output data and/or communicate with other computers. Copyright 2 in **418** is viewed in **804** as either a printed out hard copy and/or accessed in Portable Document Format (i.e., .pdf and/or similar file format) by a computer workstation **802**. The computer workstation is used to access geospatial data processing software in **806**. In **808**, Database 3 is accessed by the computer workstation for the geospatial data pertaining to 1) remote sensing imagery and 2) vegetation attributes in a vector file. In **810,** the geospatial data processing software is used to overlay the vegetation attribute vector file on the remote sensing imagery. The geospatial data processing software is then used to sample the remote sensing imagery per point in the vegetation attribute vector file. The outputs of **810** are entered into a spreadsheet (i.e., MS Excel) in **812** and stored on Database 5 in **814.**

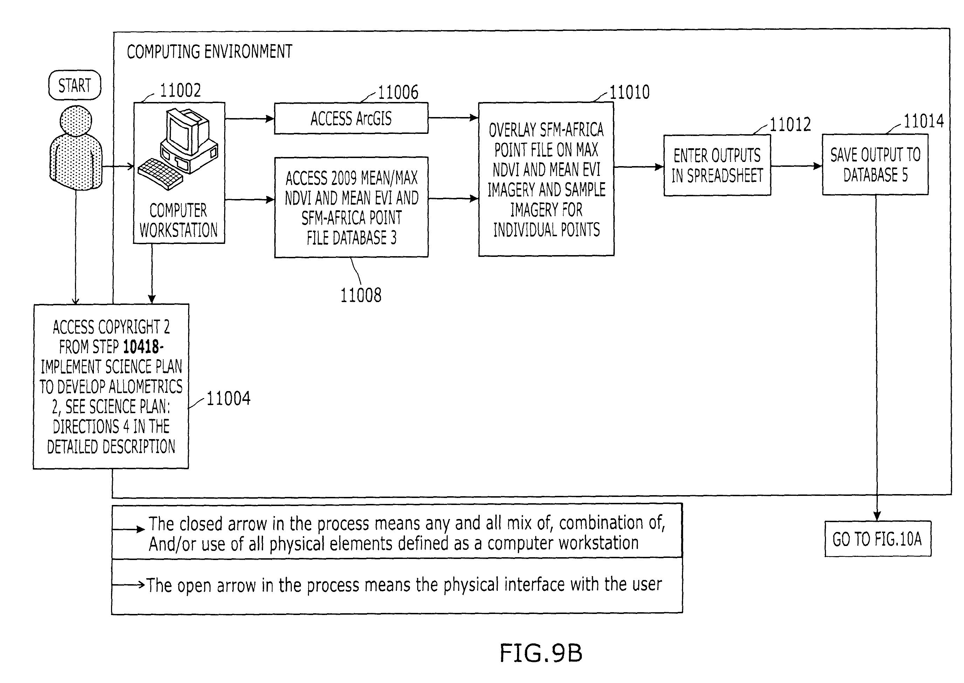

**FIG. 9B** shows the first half of the generic process used to develop Allometric Equations 2 applied with Science Plan-Directions 4 from 10418. A user accesses a computer workstation in **1102** that includes a screen display(s), processor(s), hard drive(s), a keyboard, a mouse, a router connected to the internet and other physical elements related to a computer workstation, etc. A computer workstation **11002** in this context can store, retrieve, process and/or output data and/or communicate with other computers. Copyright 2 in **10418** is viewed in **11004** as either a printed out hard copy and/or accessed in Portable Document Format (i.e., .pdf and/or similar file format) by a computer workstation in **11002**. The computer workstation is used to access ArcGIS in **11006**. In **11008,** the computer workstation is used to access Database 3 for geospatial data 1) for composites of 2009 MODIS mean / max NDVI and mean EVI at 250m x 250m spatial resolution in 2009 and 2) a vector file containing 551 points of geospatial data for vegetation attribute obtained in 2009. The decision to use 2009 mean / max NDVI and mean EVI was determined by Science Plan-Directions 3 and the results exemplified for coefficient of variation in **2226** from **FIG. 5D.** In this instance, the geospatial data for the 551 points in the vector file was obtained from SFM-Africa. The vegetation attributes were collected by SFM-Africa from field observation in 2009 for basal area, which SFM-Africa then used with allometric equations to calculate volume and above-ground biomass per point. In **11010,** ArcGIS is used to overlay the 551 points in the vector file on the 2009 MODIS mean / max NDVI and mean EVI at 250m x 250m spatial resolution. In **11106** from **FIG. 9C,** the image shows a visual representation of the 551 field plots overlaid on 2009 mean EVI at 250m x 250m resolution. ArcGIS is then used to sample the 2009 MODIS mean / max NDVI and mean EVI at 250m x 250m spatial resolution per point in the vector file containing 551 points of geospatial data. The outputs of **11010** are entered into a spreadsheet (i.e., MS Excel) in **11012** and stored on Database 5 in **11014.**

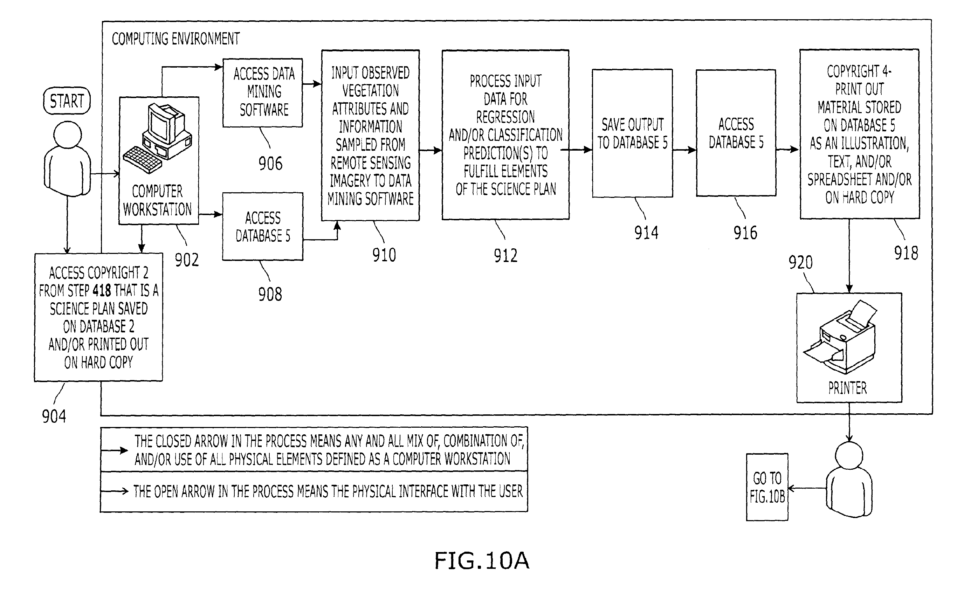

**FIG. 10A** shows the second half of the process used to develop Allometric Equations 2. A user accesses a computer workstation in **902** that includes a screen display(s), processor(s), hard drive (s), a keyboard, a mouse, a router connected to the internet and other physical elements related to a computer workstation, etc.). A computer workstation **902** in this context can store, retrieve, process and/or output data and/or communicate with other computers. Copyright 2 in **418** is viewed in **904** as either a printed out hard copy and/or accessed in Portable Document Format (i.e, .pdf and/or similar file document format) by a computer workstation. The computer work station is used to access data mining software in **906**. Data mining software in this context means a software package (i.e., advanced statistical operations developed by the field of machine learning) that can be used to train a predictive regression and/or classification model between remote sensing imagery of vegetation (i.e., input data) and field observations of vegetation attributes (i.e., target data). Examples of data mining software are Orange, Weka, Rattle and/or any application, variation and/or version of the software R. The spreadsheets from **812** stored on Database 5 are accessed in **908** and entered as input data into the data mining software in **910**. In **912,** the data mining software is processed with the input data to develop a training dataset for a predictive model based on observed vegetation attributes from the predictor input data. The outputs of **912** are stored on Database 5 in **914**. The outputs of **912** are accessed in **916,** printed out as a spreadsheet and/or a graphical illustration and defined as Copyright 4 in **918** in either a Portable Document Format (i.e., .pdf and/or similar file document format) digital file and/or in hard copy with a printer in **920**.

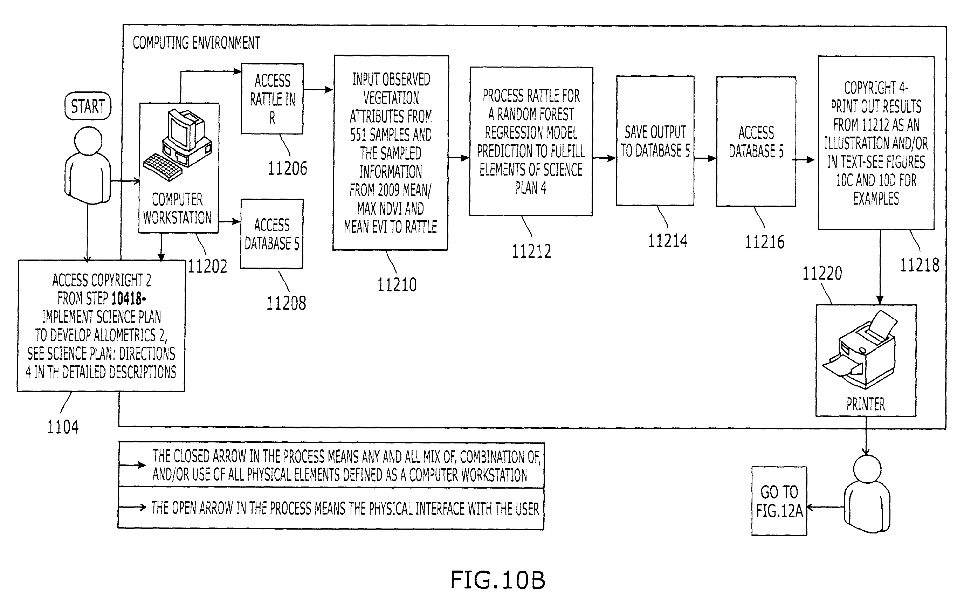

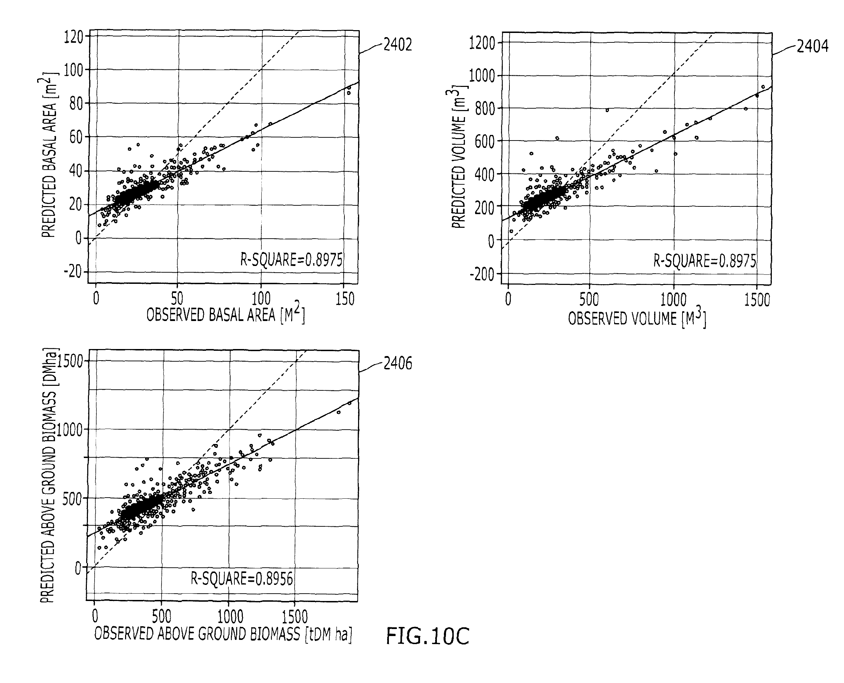

**FIG. 10B** shows an example for the second half of the generic process used to develop Allometric Equations 2 applied to the data mining software Rattle in R. A user accesses a computer workstation in **11202** that includes a screen display(s), processor(s), hard drive(s), a keyboard, a mouse, a router connected to the internet and other physical elements related to a computer workstation, etc. A computer workstation **11202** in this context can store, retrieve, process and/or output data and/or communicate with other computers. Copyright 2 in **10418** is viewed in **11204** as either a printed out hard copy and/or accessed in Portable Document Format (i.e, .pdf and/or similar file document format) by a computer workstation **11202**. The computer workstation is used to access Rattle in R in **11206**. The spreadsheet containing the samples for 2009 MODIS mean / max NDVI and mean EVI and SFM-Africa's 551 field observations from **11012** that are stored on Database 5 are accessed in **11208** and entered into Rattle in **11210**. In **11212,** Rattle is processed with the said spreadsheet from **11208** to develop a training model with a Random Forest regression predictive function (see Science Plan-Directions 4 in **10418).** The outputs of the training model from **11212** are stored on Database 5 in **11214**. The outputs of the training model are **11212** are accessed in **11216,** printed out as a spreadsheet, text and/or a graphical illustration and defined as Copyright 4 in **11218** in either a Portable Document Format (i.e., .pdf and/or similar file format) digital file and/or in hard copy with a printer in **11220. FIG. 10C** shows an example of an illustration for results of the Random Forest training model for the following: basal area in **2402,** volume in **2404** and above-ground biomass in **2406**.

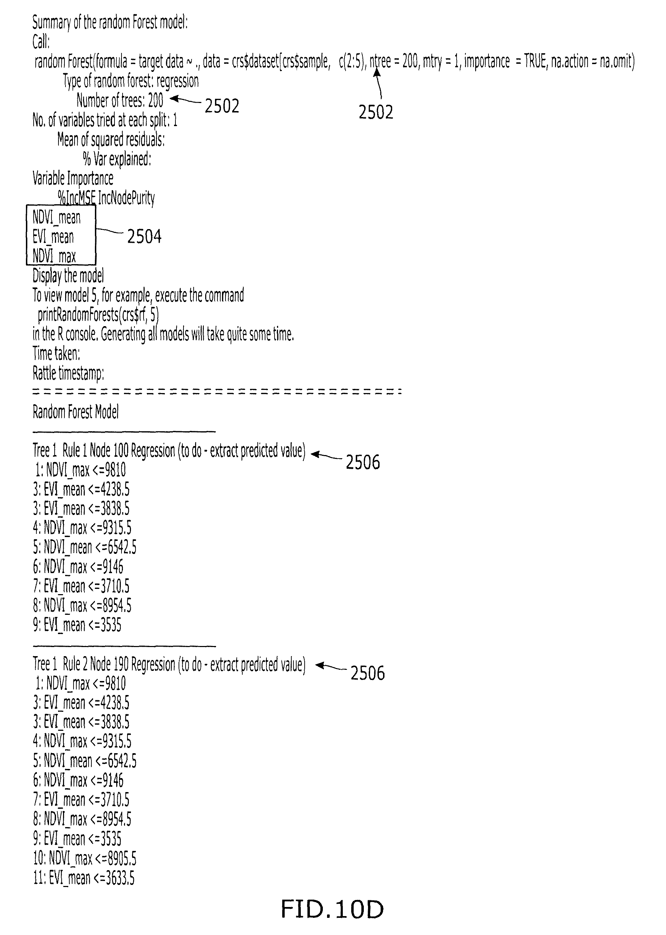

**FIG. 10D** shows the text output for results of a Random Forest training model for basal area from **2402**. The number of decision trees grown was 200, which is the minimum suggested decision trees to grow for a Random Forest model and this is shown in **2502**. The mean/max NDVI and mean EVI inputs are shown in **2504**. Examples of decision tree rules for the training model are shown in **2506**.

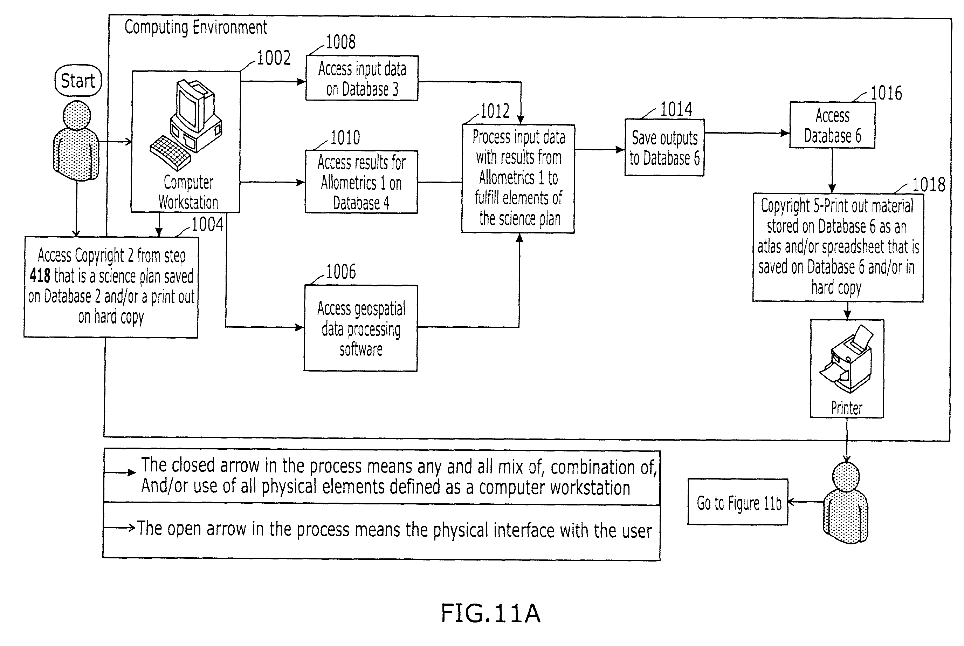

**FIG. 11 A** shows the generic process for implementing Allometric Equations 1 with input data. A user accesses a computer workstation in **1002** that includes a screen display(s), processor(s), hard drive(s), a keyboard, a mouse, a router connected to the internet and other physical elements related to a computer workstation, etc. A computer workstation **1002** in this context can store, retrieve, process and/or output data and/or communicate with other computers. Copyright 2 from **418** is viewed in **1004** as either a printed out hard copy and/or accessed in Portable Document Format (i.e., .pdf and/or similar file format) by a computer workstation **1002**. The computer workstation is used to access a geospatial data processing software in **1006**. Examples of geospatial data processing software are the following: ArcView/GIS, Erdas Imagine, Envi, Idrisi, Quantum GIS, Grass, and Land Change Modeler and/or other relevant image processing software, etc., that is copyrighted and/or copyrightable and/or in open access. The input data stored on Database 3 is accessed with the geospatial data processing software in **1008**. The results for Copyright 3 in **720** from the outputs for the development of Allometric Equations 1 are accessed on Database **4** in **1010**. In **1012,** the geospatial data processing software is used to process the input data from **1008** with Allometric Equations 1 in **1010** to fulfill the final elements for Allometric Equations 1 in the science plan from **418**. The outputs of **1012** are stored on Database 6 in **1014**. The outputs of **1012** are accessed in **1016,** printed out as a spreadsheet and/or a graphical illustration and defined as Copyright 5 in **1018** as either a Portable Document Format (i.e, .pdf and/or similar file document format) digital file and/or in hard copy with a printer **1020**.

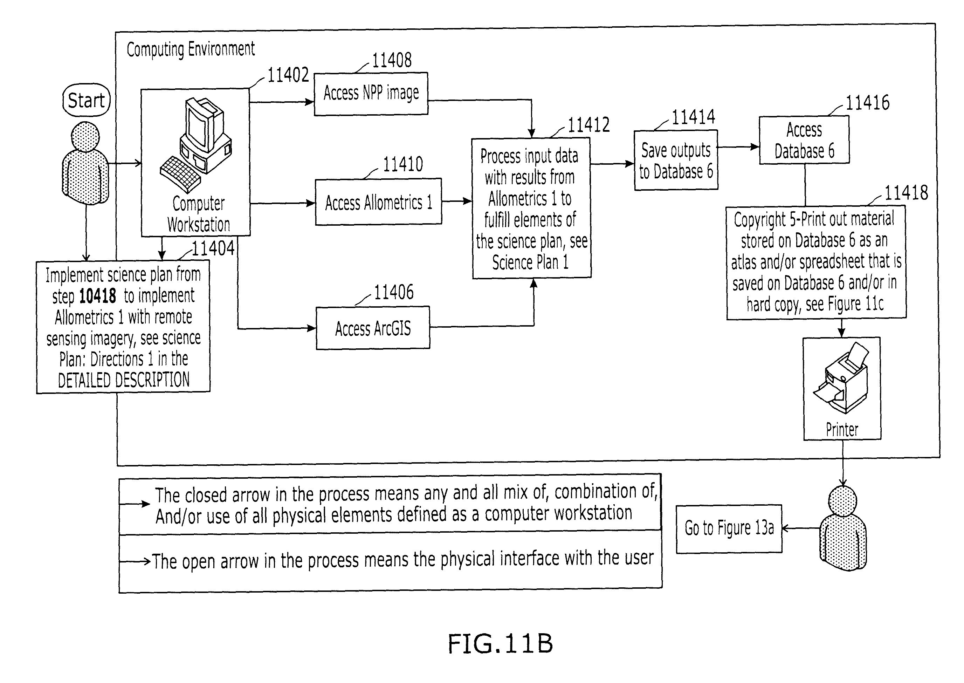

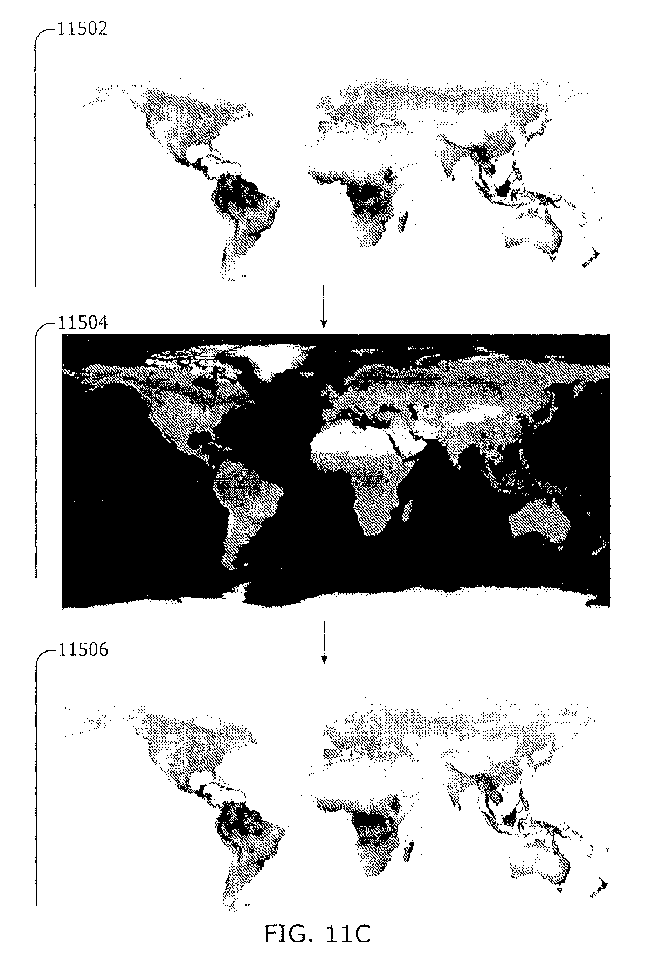

**FIG. 11 B** shows an example for the generic process for implementing Allometric Equations 1 with input data applied to the allometrics developed in **FIG. 8B** and Science Plan-Directions 1. A user accesses a computer workstation in **11402** that includes a screen display(s), processor(s), hard drive(s), a keyboard, a mouse, a router connected to the internet and other physical elements related to a computer workstation, etc. A computer workstation **11402** in this context can store, retrieve, process and/or output data and/or communicate with other computers. Copyright 2 from **10418** is viewed in **11404** as either a printed out hard copy and/or accessed in Portable Document Format (i.e., .pdf and/or similar file format) by a computer workstation **11402**. The computer workstation is used to access ArcGIS in **11406**. The input data for Net Primary Production (NPP) stored on Database 3 are accessed with ArcGIS in 11408. The results for Allometric Equations 1 from **10920** stored on Database 5 as Copyright 3 are accessed with ArcGIS in **11410**. In **11412,** ArcGIS is used to process to process NPP in **11408** with the results for Allometric Equations 1 in **11410** to fulfill the final elements of implementing Allometics 1 in the Science Plan-Directions 1 from **10418**. The outputs of **11412** are stored on Database 6 in **11414.** The outputs of **11412** are accessed in **11416,** printed out as a spreadsheet and/or a graphical illustration and defined as Copyright 5 in **11418** as either a Portable Document Format (i.e., .pdf and/or similar file format) digital file and/or in hard copy with a printer **11420. FIG. 11 c** shows an illustrative example of implementing Allometric Equations 1 with Net Primary Production in **11502**. In **11504,** the Allometric Equations for the NPP to leaf carbon ratio (reported in **Table 3** as $AB_1\_LF\_ratio$) are shown as a raster map. Equation 17 from **10418** was used to calculate leaf carbon with NPP in **11502** and the NPP to leaf carbon ratio in **11504**. The output for Equation 17 is shown in **11506.**

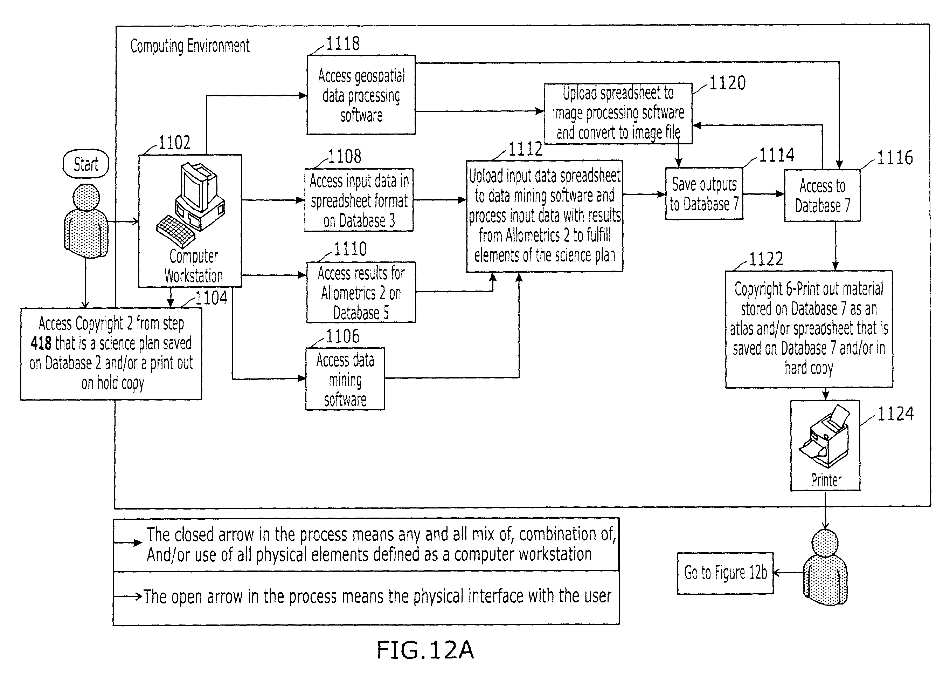

**FIG. 12A** shows the generic process for implementing Allometric Equations 2 with input data. A user accesses a computer workstation in **1102** that includes a screen display(s), processor(s), hard drive(s), a keyboard, a mouse, a router connected to the internet arid other physical elements related to a computer workstation, etc. A computer workstation **1102** in this context can store, retrieve, process and/or output data and/or communicate with other computers. Copyright 2 in **418** is viewed in **1104** as either a printed out hard copy and/or accessed in Portable Document Format (i.e, .pdf and/or similar file format) by a computer workstation in **1102**. The computer workstation is used to access: 1) data mining software in **1106,** 2) the input data stored in spreadsheet format on Database 3 in **1108** and 3) the predictive model developed from the training dataset for Allometric Equations 2 stored on Database 5 in **1110**. In **1112,** the input data in **1108** and the training model in **1110** are loaded into the data mining software from **1106**. The data mining software is processed in **1112** to evaluate and score input data from **1108** for predictions based on the training model in **1110**. The outputs are saved to Database 7 as a new spreadsheet in **1114**. The computer workstation is used to access geospatial data processing software in **1118.**. Examples of geospatial data processing software are the following: ArcView/GIS, Erdas Imagine, Envi, Idrisi, Quantum GIS, Grass, and Land Change Modeler and/or other relevant image processing software, etc., that is copyrighted and/or copyrightable and/or in open access. Database 7 is accessed in **1116,** the outputs of **1112** are loaded into the geospatial data processing software in **1120** and processed to convert the scored outputs from **1112** (that are either georeferenced and/or accompanied with a grid code coordinate) to a raster file. The outputs of **1120** are stored on Database 7 in **1114**. The outputs of **1120** are accessed in **1116,** printed out as a spreadsheet and/or an atlas and defined as Copyright 6 in **1122** as either a Portable Document Format (i.e, .pdf and/or similar file format) digital file and/or in hard copy with a printer in **1124.**

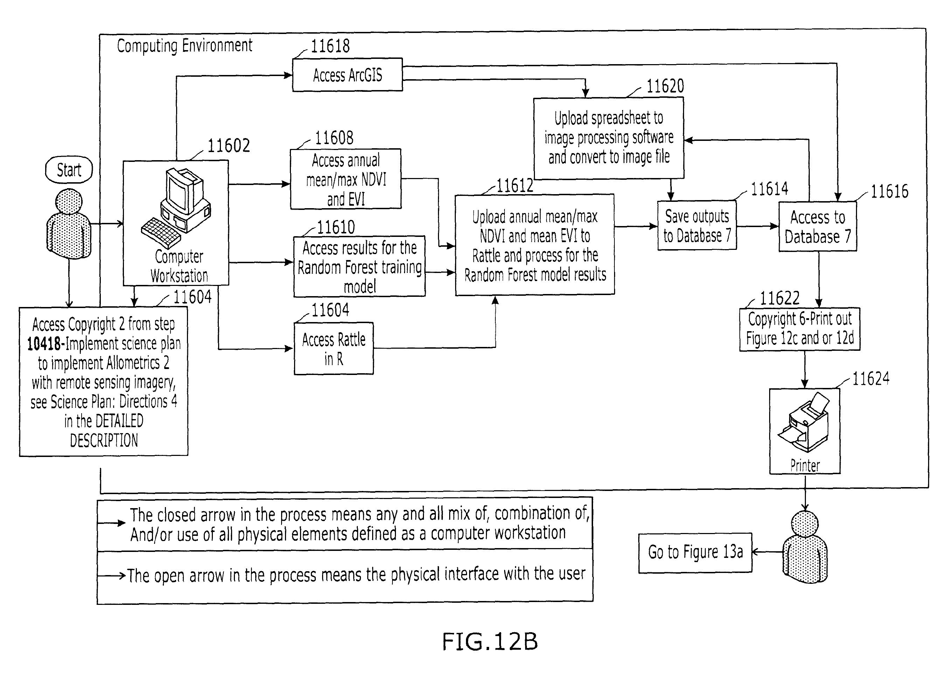

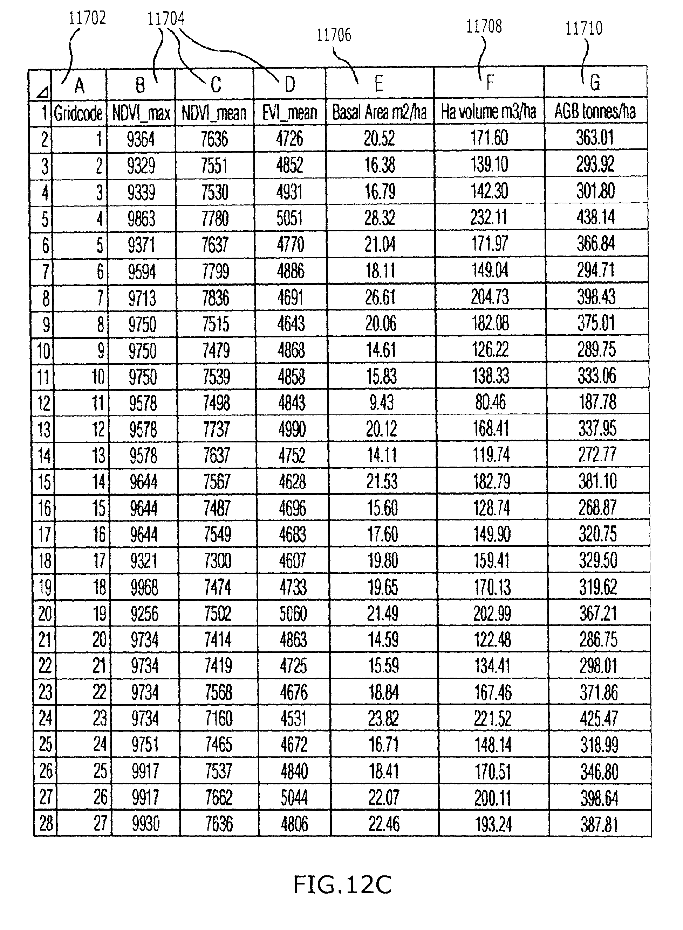

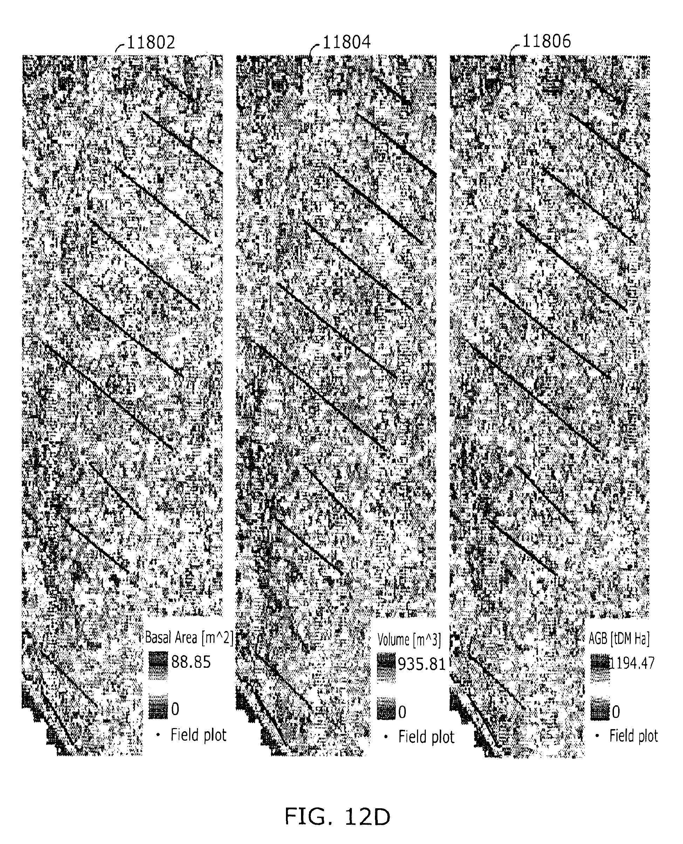

**FIG. 12B** shows an example for the generic process for implementing Allometric Equations 2 with the outputs of **FIG. 10B** and Science Plan-Directions 4. A user accesses a computer workstation in **11602** that includes a screen display(s), processor(s), hard drive(s), a keyboard, a mouse, a router connected to the internet and other physical elements related to a computer workstation, etc. A computer workstation **11602** in this context can store, retrieve, process and/or output data and/or communicate with other computers. Copyright 2 in **10418** is viewed in **11604** as either a printed out hard copy and/or accessed in Portable Document Format (i.e, .pdf and/or similar file format) by a computer workstation **11602.** The computer workstation is used to access 1) Rattle in R in **11606, 2)** MODIS annual mean/max NDVI and mean EVI stored in spreadsheet format stored on Database 3 in **11608** and 3) the predictive Random Forest model developed from the training dataset for Allometric Equations 2 stored on Database 5 in **11610.** In **11612,** the mean / max NDVI and mean EVI data in **11608** and the Random Forest training model in **11610** are loaded into Rattle. Rattle is processed in **11612** to evaluate and score the mean / max NDVI and mean EVI data from **1108** for predictions based on the Random Forest training model in **11610.** The outputs are saved to Database 7 as a new spreadsheet in **11614.** The computer workstation is used to access ArcGIS in **11618.** Database 7 is accessed in **11616,** the outputs from **11612** are loaded into ArcGIS in **11620** and processed to convert the scored outputs from **11612** (that are georeferenced and/or in grid code coordinate) to a raster file. The outputs of **11620** are stored on Database 7 in **11614.** The outputs of **11620** are accessed in **11616,** printed out as a spreadsheet and/or an atlas and/or an illustration and defined as Copyright 6 in **11622** in either a Portable Document Format (i.e, .pdf and/or similar file format) digital file and/or in hard copy with a printer **11624. FIG. 12C** is an example of the scored outputs from **11612** in a spreadsheet with a gridcode. The gridcode coordinate per sample used as a pixel in a raster image is shown in **11702.** The individual NDVI mean / max and mean EVI values per gridcode are shown in **11704.** The scored outputs show the following: basal area in **11706,** volume in **11708** and above ground biomass in **11710. FIG. 12D** is an example of the scored outputs from **11620** as an atlas and/or an illustration reflecting the raster file for the following scored outputs: basal area in **11802,** volume in **11804** and above ground biomass in **11806.**

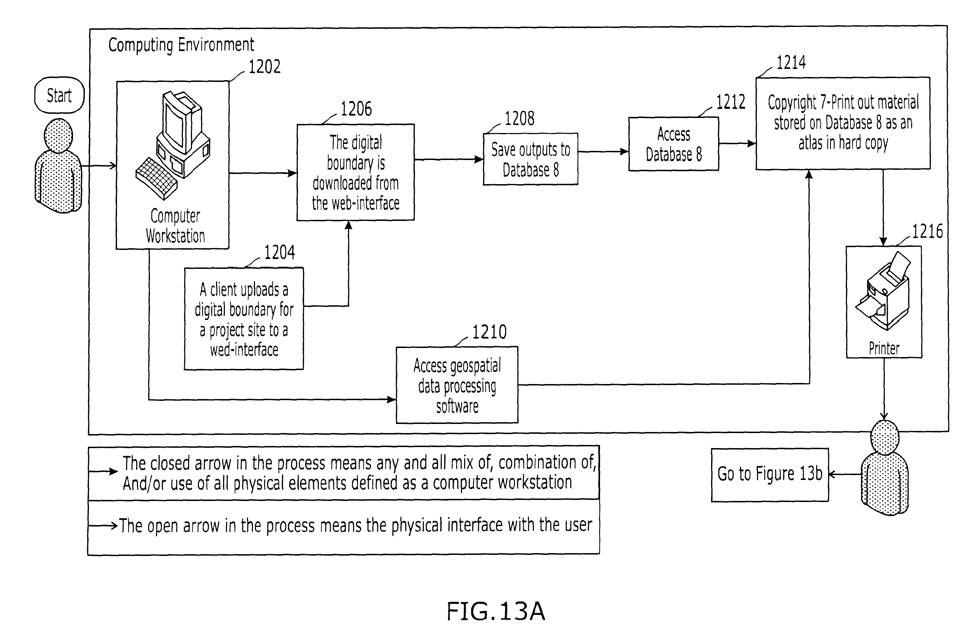

**FIG. 13A** shows the generic process for obtaining a project boundary from a client. A user accesses a computer workstation in **1202** that includes a screen display(s), processor(s), hard drive (s), a keyboard, a mouse, a router connected to the internet and other physical elements related to a computer workstation, etc. A computer workstation **1202** in this context can store, retrieve, process and/or output data and/or communicate with other computers. In **1204,** a client uploads the digital boundary of a project site to an internet interface (i.e., via email and/or a website). In **1206,** the computer workstation is used to download the digital boundary from the internet interface. The client's digital boundary is stored in Database 8 in **1208.** Geospatial data processing software is accessed in **1210.** Examples of geospatial data processing software are the following: ArcView/GIS, Erdas Imagine, Envi, Idrisi, Quantum GIS, Grass, and Land Change Modeler and/or other relevant image processing software, etc., that is copyrighted and/or copyrightable and/or in open access. The files stored on Database 8 are accessed in **1212** with the geospatial data processing software. The client's digital boundary is printed out as an illustration and/or atlas and defined as Copyright 7 in **1214** in either a Portable Document Format (i.e, .pdf and/or similar file format) digital file and/or in hard copy with a printer in **1216.**

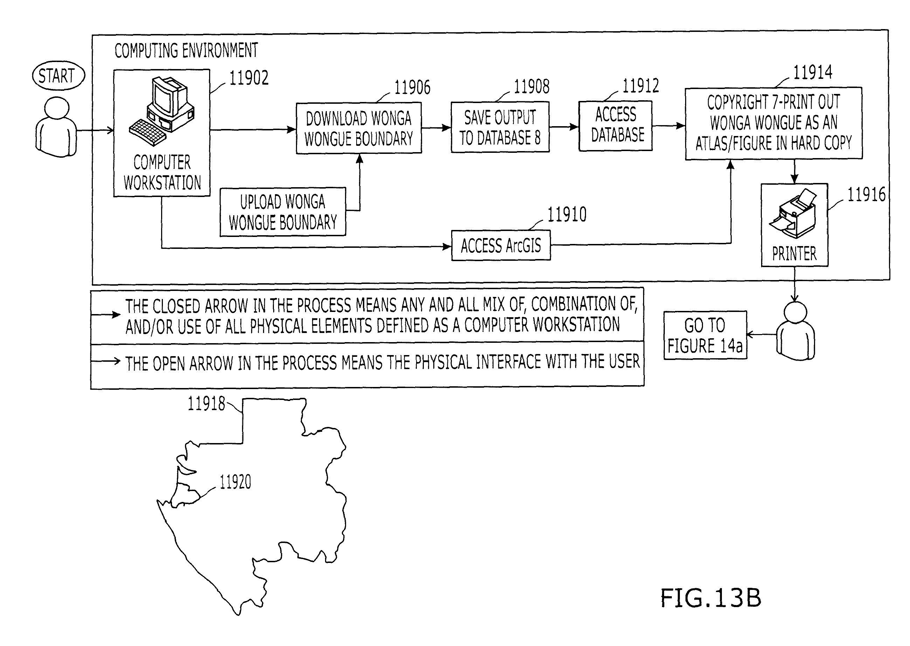

**FIG. 13B** shows an example for the generic process for obtaining a project boundary applied to the Wonga-Wongué nature reserve in Gabon. A user accesses a computer workstation in **11902** that includes a screen display(s), processor(s), hard drive(s), a keyboard, a mouse, a router connected to the internet and other physical elements related to a computer workstation, etc. A computer workstation **11902** in this context can store, retrieve, process and/or output data and/or communicate with other computers. In **11904,** a client uploads the digital boundary for the Wonga-Wongué nature reserve to a web-interface (i.e., via email and/or a website). In **11906,** the computer workstation in **11902** is used to download the digital boundary for the Wonga-Wongué nature reserve from the web-interface. The digital boundary for the Wonga-Wongué nature reserve is stored in Database 8 in **11908.** ArcGIS is accessed in **11910.** The Wonga-Wongué nature reserve boundary file stored on Database 8 is accessed in **11912** with ArcGIS. The Wonga-Wongué nature reserve boundary file is printed out as an illustration and/or atlas and defined as Copyright 7 in **11914** in either a Portable Document Format (i.e, .pdf and/or similar file format) digital file and/or in hard copy with a printer in **11916.** The output of **11914** is shown in **11918,** where the boundary of Wonga-Wongué nature reserve is 19920.

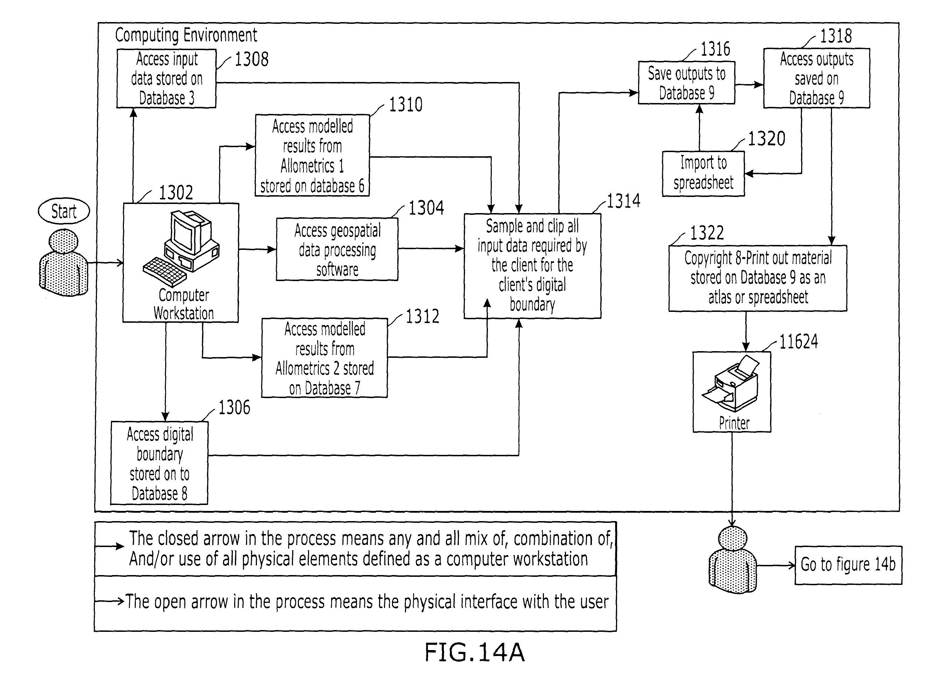

**FIG. 14A** shows the generic process for sampling input data and the outputs of Allometric Equations 1 and 2 with a client's project boundary. A user accesses a computer workstation in **1302** that includes a screen display(s), processor(s), hard drive(s), a keyboard, a mouse, a router connected to the internet and other physical elements related to a computer workstation, etc. A computer workstation **1302** in this context can store, retrieve, process and/or output data and/or communicate with other computers. The computer workstation is used to access any and/or all of the following: 1) a geospatial data processing software in **1304,** 2) the client's digital boundary stored on Database 8 in **1306,** 3) the input data stored on Database 3 in **1308, 4)** the modeled results for Allometric Equations 1 stored on Database 6 in **1310,** and 5) the modeled results for Allometric Equations 2 stored on Database 7 in **1312..** Examples of geospatial data processing software are the following: ArcView/GIS, Erdas Imagine, Envi, Idrisi, Quantum GIS, Grass, and Land Change Modeler and/or other relevant image processing software, etc., that is copyrighted and/or copyrightable and/or in open access. In **1314,** the geospatial data processing software is used to overlay the client's digital boundary of a project site from **1306** over raster input data stored on Database 3 in **1308,** raster outputs for Allometric Equations 1 stored on Database 6 in **1310** and/or raster outputs for Allometric Equations 2 stored on Database 7 in **1312.** The raster material accessed in **1308, 1310** and **1312** is then sampled and/or clipped for **1306** in **1314.** When the raster material is sampled in **1314,** the sampling can be for 1) a polygon file that is the same spatial boundary as the client's project site, 2) a raster file for land cover and/or 3) a point file (converted from a land cover raster file) intersected with a polygon file that is the same spatial boundary as the client's project site. The sampled and/or clipped outputs of **1314** are saved to Database 9 in **1316.** The sampled outputs of **1314** are accessed in **1318** and loaded into a spreadsheet software (i.e., MS Excel) in **1320.** The outputs of **1314** are printed out as a table and/or an atlas and/or an illustration and defined as Copyright 8 in **1322** in either a Portable Document Format (i.e, .pdf and/or similar file format) digital file and/or in hard copy with a printer in **1324.**

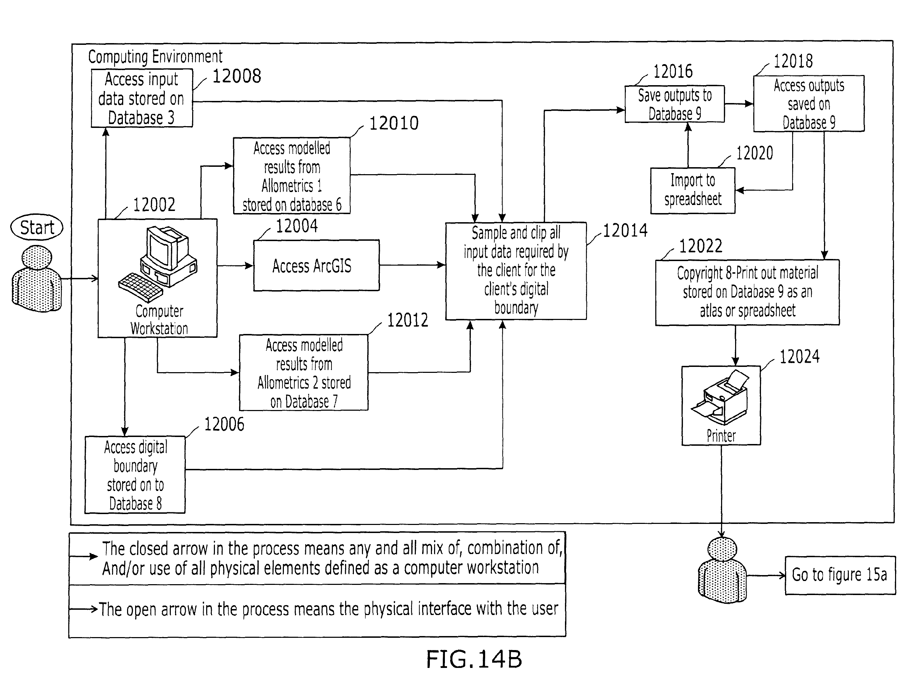

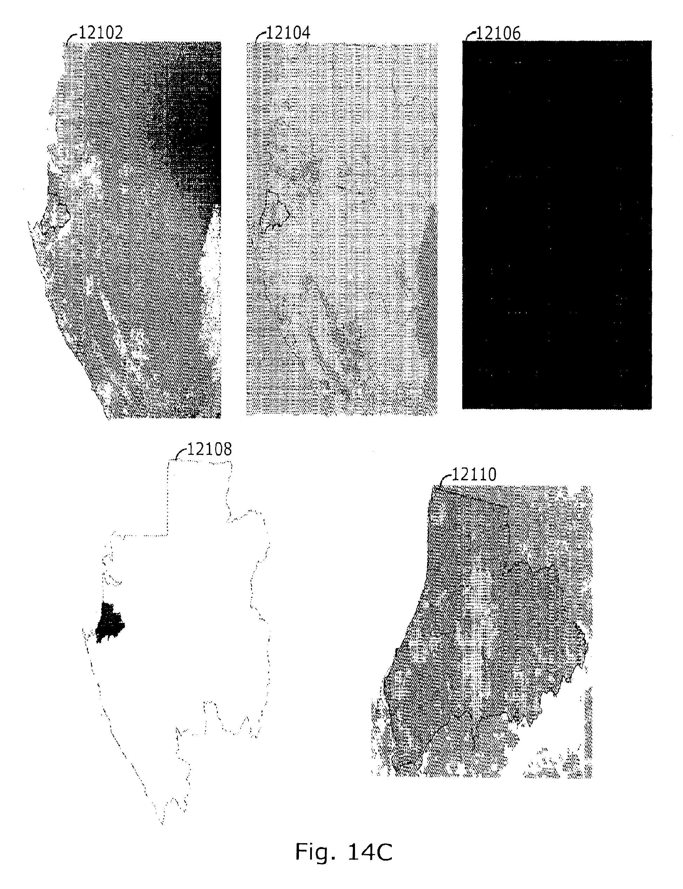

**FIG. 14B** shows an example for the generic process for sampling input data and the outputs of Allometric Equations 1 and 2 with a client's project boundary applied to the Wonga-Wongue nature reserve in Gabon. A user accesses a computer workstation in **12002** that includes a screen display(s), processor(s), hard drive(s), a keyboard, a mouse, a router connected to the internet and other physical elements related to a computer workstation, etc. A computer workstation **12002** in this context can store, retrieve, process and/or output data and/or communicate with other computers. The computer workstation is used to access the following: 1) ArcGIS in **12004,** 2) the Wonga-Wongué nature reserve digital boundary stored on Database 8 in **12006,** and 3) the modeled results for above ground carbon flux from Allometric Equations 1 stored on Database 6 in **12010.** In **12014,** ArcGIS is used to overlay the Wonga-Wongue nature reserve digital boundary (as a polygon shapefile [i.e., .shp and associated files]) from **12006** over the modeled results for the above ground carbon flux raster file in **12010.** The above ground carbon flux raster file in **12010** is then sampled and/or clipped for Wonga-Wongué nature reserve digital boundary in **12014. FIG. 14C** shows illustrative examples of the sampling from **12014** in greater detail. In **12102,** the polygon file for Wonga-Wongué nature reserve is overlaid on above ground biomass flux. The polygon is used to sample for above ground biomass flux of the gridded pixels cells within the Wonga-Wongué nature reserve boundary. A land cover raster map classified to AFOLU classes (see Section 1.22 in **10418)** is shown under the Wonga-Wongué nature reserve polygon file in **12104.** ArcGIS is used to convert the land cover raster map in **12104** to a point shape file in **12106,** where individual points have the same numerical classification value as the land cover map. ArcGIS is used to intersect the point file in **12106** with the polygon file of Wonga-Wongué in **12108.** In **12010,** the point file from **12108** is overlaid on the above ground biomass flux rater file which is sampled by the land cover numerical values in **12106.** Returning to **FIG. 14C,** the sampled and/or clipped outputs of **12014** are saved to Database 9 in **12016.** The sampled outputs of **12014** are accessed in **12018** and loaded into spreadsheet software (i.e., MS Excel) in **12020.** The outputs of **12014** are printed out as a table and/or an atlas and/or an illustration and defined as Copyright 8 in **12022** in either a Portable Document Format (i.e, .pdf and/or similar file format) digital file and/or in hard copy with a printer in **12024. Table 5** 1 a shows as example of the outputs from above ground biomass flux sampled for the Wonga-Wongué nature reserve polygon and 1 b shows as example of the outputs from above ground biomass flux sampled for the Wonga-Wongué nature reserve point file representing land cover classes. **FIG. 14C** is indicative of the atlas/illustration output of 12022.

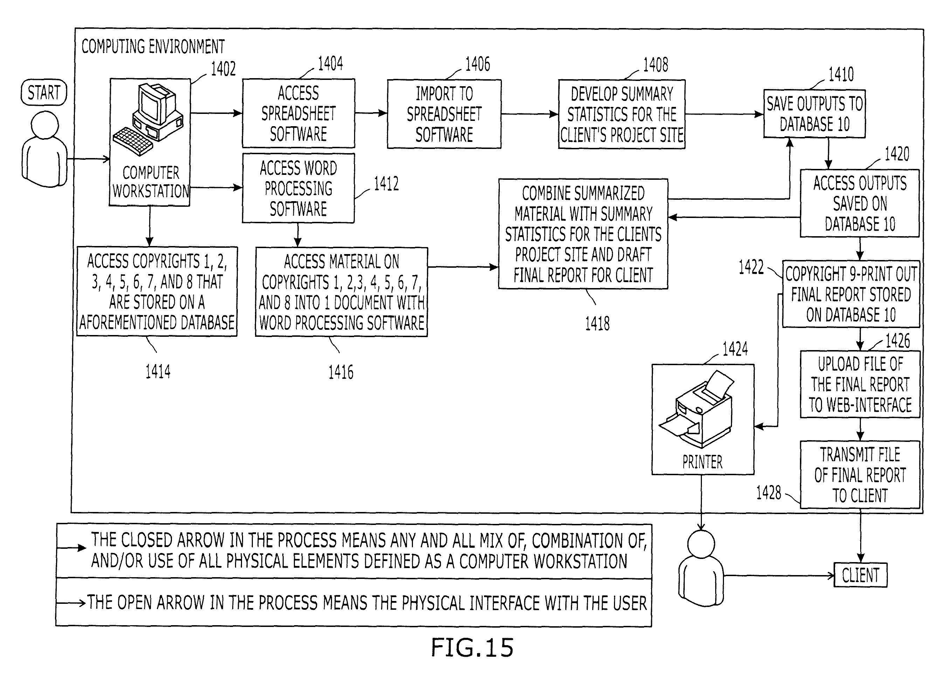

**FIG. 15** shows the generic process for reporting information to the client. A user accesses a computer workstation in **1402** that includes a screen display(s), processor(s), hard drive (s), a keyboard, a mouse, a router connected to the internet and other physical elements related to a computer workstation, etc. A computer workstation **1402** in this context can store, retrieve, process and/or output data and/or communicate with other computers. The computer workstation is used to access a spreadsheet software (i.e, MS Excel) in **1404.** In **1406,** the sampled data stored in spreadsheets on Database 9 is imported to the spreadsheet software in **1404.** In **1408,** summary statistics are calculated for sampled data at the client's project site. The summary statistics in **1408** include any and/or all of the following: 1) reporting the sampled data and the conversion to relevant annual GHGs and/or other vegetation attributes required in reporting at the project site; 2) development of a baseline for annual GHG emissions and removals at the project site for a period in time prior to project implementation; 3) predicted future annual GHG emissions and removals from the baseline for the project lifetime (i.e., the period in time in which the client's project will be implemented) without the project taking place; and 4) predicted future annual GHG emissions and removals for the project lifetime (i.e., the period in time in which the client's project will be implemented) with the project taking place; 5) assessment of the potential annual GHG offset from the project for the project lifetime; 6) an assessment of annual GHG emissions, removals and other relevant vegetation attributes for the period after the project was implemented. **Table 5** 2a and 2b shows examples above ground biomass flux for the Wonga-Wongué nature reserve converted from tonnes of carbon to tonnes of carbon dioxide used as one variable reported to the client. The summary statistics are saved to Database 10 in **1410.** A computer workstation is used to access 1) a word processing software in **1412,** and 2) Copyrights 1, 2, 3, 4, 5, 6, 7 and 8 that are stored on their respective databases. In **1416,** the Copyrighted material from **1412** is assembled into one document with the word processing software. In **1418,** the final report is drafted by combining the assembled material from **1416** with the spreadsheet for summary statistics at the project site in **1408** accessed by **1420.** A review explaining the sampled summary statistics is also drafted in **1418.** The outputs of **1418** are stored on **1410.** The outputs of **1418** are accessed in **1420,** printed out as Copyright 9 in **1422** in either a Portable Document Format (i.e, .pdf and/or similar file format) digital file and/or in hard copy with a printer in **1424.** When **1422** is printed out in a Portable Document Format (i.e, .pdf and/or similar file format) digital file, the file is uploaded to an internet interface (i,e., email and/or a web-site) in **1426.** The uploaded material in **1426** is then transmitted electronically via the internet to the client in **1428.**

Table 5

| $\Delta C_{AB}$ | MIN (tC ha$^{-1}$) | MAX (tC ha$^{-1}$) | RANGE (tC ha$^{-1}$) | MEAN (tC ha$^{-1}$) | STD (tC ha$^{-1}$) | SUM (tC) |
|---|---|---|---|---|---|---|
| 1a. Polygon | | | | | | |
| Wonga-Wongué Nature Reserve | 0.00 | 0.46 | 0.46 | 0.20 | 0.10 | 98,496.39 |
| 1b. Point | | | | | | |
| Forested land | 0.00 | 0.38 | 0.38 | 0.25 | 0.04 | 92,905.83 |
| Grassland | 0.00 | 0.46 | 0.46 | 0.05 | 0.08 | 4799.70 |
| Cropland | 0.00 | 0.01 | 0.01 | 0.01 | 0.00 | 8.00 |
| Wetland | 0.00 | 0.29 | 0.29 | 0.17 | 0.07 | 366.90 |
| $\Delta C_{AB}$ | MIN (tCO$_2$ ha$^{-1}$) | MAX (tCO$_2$ ha$^{-1}$) | RANGE (tCO$_2$ ha$^{-1}$) | MEAN (tCO$_2$ ha$^{-1}$) | STD (tCO$_2$ ha$^{-1}$) | SUM (tCO$_2$) |
| 2a. Polygon | | | | | | |
| Wonga-Wongué Nature Reserve | 0.00 | 1.69 | 1.68 | 0.75 | 0.37 | 361,153.44 |
| 2b. Point | | | | | | |
| Forested land | 0.01 | 1.39 | 1.38 | 0.92 | 0.15 | 340,654.72 |
| Grassland | 0.00 | 1.69 | 1.68 | 0.17 | 0.30 | 17,598.89 |
| Cropland | 0.01 | 0.03 | 0.01 | 0.02 | 0.00 | 29.33 |
| Wetland | 0.01 | 1.07 | 1.06 | 0.61 | 0.27 | 1345.30 |

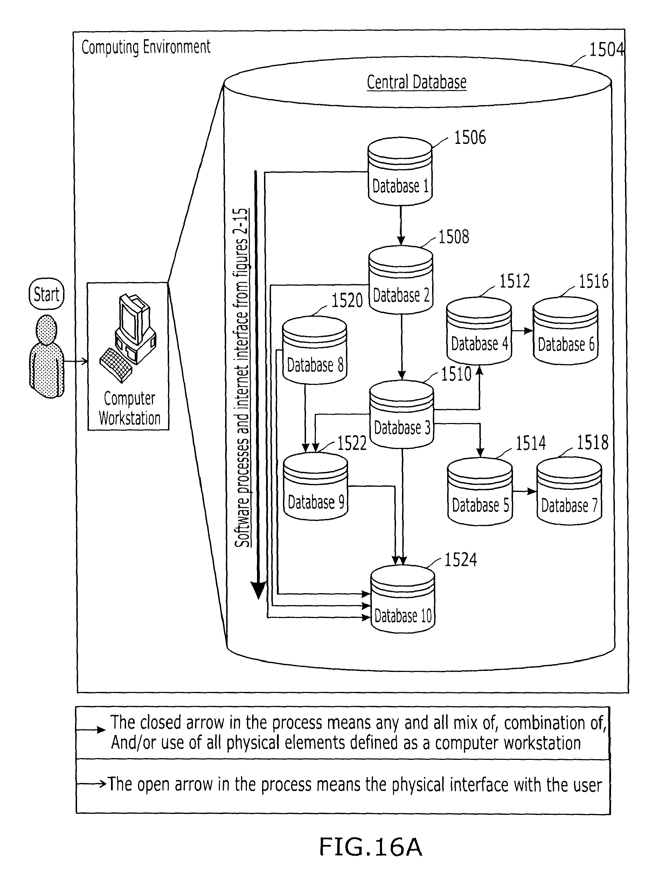

**FIG. 16A** shows the initial key interactions and structure of Database 1 through Database 10. A user accesses a computer workstation in **1502** that includes a screen display(s), processor(s), hard drive(s), a keyboard, a mouse, a router connected to the internet and other physical elements related to a computer workstation, etc. A computer workstation **1502** in this context can store, retrieve, process and/or output data and/or communicate with other computers. In **1504,** the Central Database is shown storing Database 1 through Database 10. Software processing and internet-interface from **FIG.** 2 through **FIG. 15** are used to develop all aforementioned content stored on Database 1 through Database 10 of the Central Database. The aforementioned content of Database 1 in **1506** is developed with the software processes and internet interface described herein by **FIGS. 2A-2C.** The aforementioned contents of Database 1 are used to develop the aforementioned contents in Database 2 in **1508** with software processes and internet interface described herein by **FIGS. 4A-4C.** The aforementioned contents of Database 2 are used to develop the aforementioned contents in Database 3 in **1510** software processes and internet interface described herein by **FIG. 6A** and **FIG. 7A.**

The aforementioned contents of Database 4 in **1512** are developed within Database 3 with the aforementioned contents of Database 3 and Database 2 and the software processes described herein by **FIG. 8A.** The aforementioned contents of Database 5 in **1514** are developed within Database 3 with the aforementioned contents of Database 3 and Database 2 and the software processes described herein by **FIG. 9A** and **FIG. 10A.** The aforementioned contents of Database 6 in **1516** are developed within Database 4 with the aforementioned contents of Databases 2, 3 and **4** and software processes described herein by **FIG. 11A.** The aforementioned contents of Database 7 in **1518** are developed within Database 5 with the aforementioned contents of Databases 2, 3 and 5 and the software processes described herein by **FIG. 12A.** The aforementioned contents of Database 8 in **1520** are developed with software processes and internet interface described herein by FIG. 13A. The aforementioned contents of Database 9 in **1522** are developed with the aforementioned contents of Databases 3, 6, 7 and 8 and software processes described herein by **FIG. 14A.** The aforementioned contents of Database 10 in 1524 are developed with the aforementioned contents of Databases 1, 2, 3, 4, 5, 6, 7, 8, and 9 and software processes described herein by **FIG. 15.** All aforementioned database content, interactions, software processes and internet interface within the Central Database are non-limiting within the scope of the aforementioned conceptual material and can be changed, combined, mixed, added to, adapted, updated, restructured, renamed, revised, retrieved, evolved and/or stored on a meta-database within Central Database.

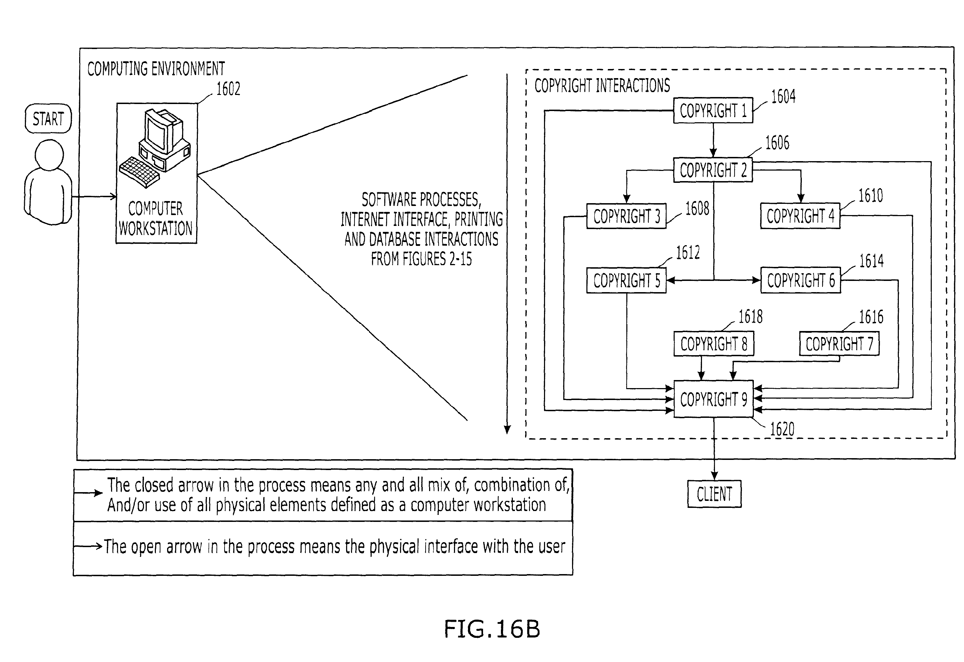

**FIG. 16B** shows the full assembly line for creating the final output of Copyright 9. A user accesses a computer workstation in **1602** that includes a screen display(s), processor(s), hard drive(s), a keyboard, a mouse, a router connected to the internet and other physical elements related to a computer workstation, etc. A computer workstation at **1602** in this context can store, retrieve, process and/or output data and/or communicate with other computers. The process starts in **1604** with Copyright 1 for the legal/policy review developed as the output from the generic process of **FIG. 2A** in **238.** Copyright 1 is used to develop Copyright 2 for a science plan from the outputs of the generic process of **FIG. 4C** in **418.** Copyright 2 in **1606** is used to inform the development and implementation of Allometric Equations 1 (in **1608** for Copyright 3 and in **1612** for Copyright 5) and Allometric Equations 2 (in **1610** for Copyright 4 and in **1614** for Copyright 6). **FIG. 8A** shows the generic process used to develop Copyright 3 as the output for **720. FIGS. 9A** and **10A** show the generic process used to develop Copyright 4 from developing Allometric Equations 2 as the output in **918. FIG. 11 A** shows the generic process used to develop Copyright 5 as output from **1018** from implementing Allometric Equations 1 with remote sensing imagery. **FIG. 12A** shows the generic process used to develop Copyright 6 as output in **1122** from implementing Allometric Equations 2 with remote sensing imagery. **FIG. 13A** shows the generic process used to develop Copyright 7 in **1214. FIG. 14A** shows the generic process used to obtain Copyright 8 from the outputs in **1322.** Copyright 7 in **1616** and Copyright 8 in **1618** are not directed linked to other copyrights. In **1620,** all copyrights are assembled into the final document as Copyright 9. The assembly is completed with a text retrieval software that organizes the copyrights in one document in ascending order by Copyright number. **FIG. 15A** shows the generic process used to develop Copyright 9 with outputs in **1422** that are electronically transmitted to the client via the internet.

In summary, the full invention relates to a computer implemented system typically with eights steps that are used to monitor and report relevant greenhouse gases for an offset project. However, the number of steps is not limited to eight, one or more of the steps can be combined, omitted, performed in any order according to application criteria. For example, the eight steps are referenced in **FIG. 1** and are the following: 1) a method for developing a legal/policy analysis; 2) a method for developing a science plan based on the legal / policy analysis, 3) the development of a geospatial database based on the science plan; 4) a method for developing an allometric model that is based on the science plan and geospatial database; 5) a method for implementing the allometric equations with remote sensing imagery based on the science plan and geospatial database; 6) a method for obtaining a client's geographical boundary of an offset through an internet interface; 7) a method for sampling a client's geographical boundary for the contents of the geospatial database, 8) a method for developing a report from the outputs of steps 1-7 that is transmitted to the client through an internet interface.

The first step of the full invention relates to a method for developing a legal /policy review for a target greenhouse gas. The full method for developing a legal/policy analysis is shown in **FIG. 2A.** The first step in the method for developing a legal / policy analysis includes developing a database for any and all relevant policy documents related to mitigating climate change. The second step in the method for developing a legal / policy analysis includes using text retrieval software to search for key words on any relevant policy document stored on the policy document database. **FIG. 2B** shows the automated method to search and retrieve text, figures and tables from policy documents. The retrieved information is used to compile parameters for monitoring a target greenhouse gas. The key words are stored on a meta-database. The third step in the method for developing a legal / policy analysis includes structuring the policy documents into tiers based on legal priority. **FIG. 2C** shows a tiered structure to assess policy documents by legal priority. The policy documents are then compared for monitoring guidance requirements between documents at different tiers. The comparison assesses whether the monitoring requirements for each tier is fungible with a tier that has greater legal priority.

The second step of the full invention relates to a method for developing a science plan based on the outputs of the legal / policy analysis. The full method for developing a science plan is shown in **FIG. 4D.** The first step in the method to develop a science plan includes developing a database on current and planned satellite missions and remote sensing instruments. The second step in the method for developing a science plan includes using text retrieval software to search for key words that describe the remote sensing instrument's monitoring capabilities for vegetation. The text retrieval is performed on the database of current and planned satellite missions and remote sensing instruments. **FIG. 4A** shows the automated method to search and retrieve information from the satellite mission and remote sensing instrument. The third step in the method for developing a science plan includes an output from the assessment of the remote sensing database. The output assesses current and future satellite missions and remote sensing instruments in relation to the data continuity requirements for the lifetime of a client's project activity. **FIG. 4B** shows an example of the output from the assessment of current and planned satellite missions and remote sensing instruments in relation to the lifetime of an offset project. The point of the output is that there must be overlapping data continuity between satellite missions to monitor a offset project. The fourth step in the method for developing a science plan includes developing a database of peer-reviewed journal for the remote sensing instrument that best meets the data continuity requirements for an offset project. The fifth step in the method for developing a science plan includes using text retrieval software to search for key words in the peer-reviewed journal articles that describe key words developed by the legal / policy review. **FIG. 4C** shows the automated method to search and retrieve information from the peer-reviewed journal articles. The sixth step in the method for developing a science plan includes directions used to monitor an offset project. The directions are developed from the information retrieved from peer-reviewed journal articles. The directions 1) define the current knowledge space in public access that does not explicitly monitor the target greenhouse gas and 2) defines new knowledge space that explicitly monitors the target greenhouse gas for the outputs from the legal / policy analysis. Examples of directions are found in **FIGs 5A-5D.** These are examples of the directions used that are used to 1) develop the allometric equations and 2) implement the allometric equations with remote sensing imagery. These examples of directions explain two methods to an develop allometic model: 1) Allometric Equations 1 that use a process-based dynamic ecosystem model to develop fractional functions that will be implemented with remote sensing imagery and 2) Allometric Equations 2 that will be used to develop regression and/or classification functions between physical samples of a vegetation attribute and samples from remote sensing imagery.

The third step of the full invention relates to developing a geospatial database from the science plan. The method for developing a geospatial database is shown in **FIG. 6A.** The geospatial database consists of 1) free geospatial data, 2) geospatial data that is not free, but can be purchased from a provider and/or, and 3) the geospatial data obtained from a client through an internet interface. The geospatial database includes the following: a standard remote sensing imagery product that fulfills data continuity requirements for monitoring a vegetation attribute within the geographical boundaries of the offset project; a secondary remote sensing imagery product at a higher resolution than the standard remote sensing imagery product, but with fewer replicates over time than the standard remote sensing imagery product; climate geospatial data; elevation geospatial data; soil geospatial data; vegetation attribute geospatial data; peer-review literature and trading mechanism reports containing a geospatial reference to vegetation attributes; and/or official government disclosures for vegetation attributes with a geospatial references and/or disclosures of geospatial data for a measurement of a vegetation attribute. The contents of the geospatial database are preprocessed in relation to the contents of the science plan. **FIG. 7A** shows an example for the preprocessing the raw downloaded remote sensing imagery with a geospatial data processing software to: 1) sub-set any condensed data files, 2) remove poor quality pixel information in the remote sensing imagery, 3) develop qualitative statistics (mean, max, min, etc) for any period in time that the database encompasses. The remote sensing imagery can also be preprocessed by any mixing and/or combining of different remote sensing images and/or bands to create an index of multiple remote sensing images. The preprocessing of the vegetation attribute geospatial data can include converting any and/or all vegetation attribute geospatial data into one consolidated vector file format. The vegetation attribute information stored on the database related peer-reviewed journal articles and/or trading mechanism reports containing a geographically referenced coordinate for measurements of vegetation attributes can be processed with text retrieval software to extract the information related to vegetation attribute(s) and the georeferenced coordinate.

The fourth step of the full invention relates to a method(s) for developing an allometric model(s) based on the outputs of the science plan and the contents of the geospatial database. Allometric Equations 1 are developed with a process-based dynamic ecosystem model for fractions. **FIG. 8A** shows the method of developing fractions with a process-based dynamic ecosystem model. The method uses a processed-based dynamic ecosystem model and input data stored on the geospatial database to develop fractions based on the directions in the science plan. Allometric Equations 2 are developed for regression / classification functions between physical sample of a vegetation attribute and samples from remote sensing imagery with a data mining software. The process includes a method to extract the geospatial information in a pixel of a remote sensing image that is at the same geographical coordinate as the geospatial data of the vegetation attribute. **FIG. 9A** shows a method for extracting remote sensing data in a raster file by a point vector file. A geospatial data processing software is used to extract the remote sensing data. The method for extracting the data includes input data from the geospatial database, including 1) geospatial data for a vegetation attribute that is in a point vector file and 2) remote sensing imagery that is in a rater file. The next step in the process is a method of developing a training model between the samples of the vegetation attribute and the samples from the remote sensing imagery with data mining software. **FIG. 10A** shows a method of developing an allometric model between a physical sample of a vegetation attribute and a sample from remote sensing imagery. The method for developing the training model uses a sample for a vegetation attribute and the sample for remote sensing imagery to develop a regression and/or a classification functions between the two samples.

The fifth step of the full invention relates to a method(s) for implementing an allometric model(s) with remote sensing imagery based on the outputs of the science plan and the contents of the geospatial database. Allometric Equations 1 use the fractional function outputs for Allometric Equations 1 developed in the fourth step with input geospatial data. The input geospatial data is for standard remote sensing products of a vegetation attribute, for example MODIS MOD 17 Net Primary Production product (NPP). The implementation is completed with a geospatatial data processing software. **FIG. 11 A** shows a method for processing the outputs of Allometic Equations 1 with remote sensing imagery. The output is a new map of geospatial data for the specific vegetation attribute defined by the directions in the science plan that meets the requirements for monitoring and/or reporting in the legal/policy analysis. Allometric Equations 2 use the regression and/or classification function outputs for Allometric Equations 2 developed in the fourth step with the full remote sensing imagery that was used as a sample when regression and/or classification function was developed. The process includes scored the information in the remote sensing imagery based on the regression and/or classification function in a data mining software. After the remote sensing imagery is scored, the scored outputs are processed with a geospatial data processing software to convert the scored outputs into a map. **FIG. 12A** shows a method for processing the outputs Allometric Equations 2 with data mining software and geospatial data processing software. The output is a new map of geospatial data for the specific vegetation attribute defined by the directions in the science plan that meets the requirements for monitoring and/or reporting in the legal/policy analysis. The outputs of Allometric Equations 1 and 2 are stored on the geospatial database.

The sixth step of the full invention relates to a method(s) for obtaining a geospatial boundary vector file from a client. **FIG. 13A** shows a method for obtaining a geospatial boundary of an offset project from a client through an internet interface. The interface is either by email and/or a web-site.

The seventh step of the full invention relates to a method(s) for sampling the client's geospatial boundary vector file for any of the contents stored on the geospatial database. **FIG. 14A** shows a method for sampling a client's geospatial boundary vector file with any of the following: 1) any geospatial contents stored on the geospatial database; and/or 2) the outputs of Allometric Equations 1; and/or 3) the outputs of Allometric Equations 2. The sampling is completed with geospatial data processing software.

The seventh step of the full invention relates to a method(s) for developing and submitting a final report to a client. The report is the assembly of all outputs from steps 1-7 of the full invention. **FIG. 15** shows a method for developing a final report and transmitting the final report to a client. The final report is transmitted to a client via an internet interface.

**FIG. 16A** shows a method of database interactions from database 1 through 10 that occurs as a result of the software processes and internet interface from steps 1-8 of the full process. All contents stored on databases 1-10 are stored on a central database. **FIG. 16B** shows the automated assembly line of Copyrights 1-9 that is implemented with text retrieval software. The final out defined as Copyright 9 is transmitted to a client through an internet interface.

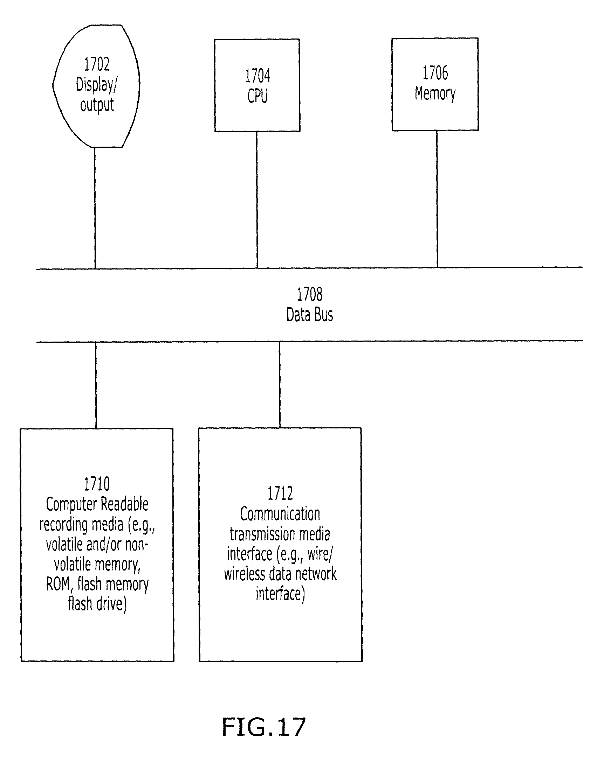

**FIG. 17** is a functional block diagram of a computer for the embodiments of the invention, namely the computer is an example of a computer workstation and/or client/server in which the embodiments can be implemented. In FIG. 17, the computer can be any computing device. Typically, the computer includes a display or output unit 1702 to display a user interface or output information or indications, such as a diode. A computer controller 1704 (e.g., a hardware central processing unit) executes instructions (e.g., a computer program or software) that control the apparatus to perform operations. Typically, a memory 1706 stores the instructions for execution by the controller 1704. According to an aspect of an embodiment, the apparatus reads/writes/processes data of any computer readable recording media 1710 and/or communication transmission media interface 1712. The display 1702, the CPU 1704 (e.g., hardware logic circuitry based computer processor that processes instructions, namely software), the memory 1706, the computer readable media 1710, and the communication transmission media interface 1712, are in communication by the data bus 1708. Any results produced can be output, for example, printed or displayed on a display for the computing hardware.

According to an aspect of the embodiments of the invention, any combinations of one or more of the described features, functions, operations, and/or benefits can be provided. A combination can be one or a plurality. The phrase 'all' includes and can be one or more, or all, or any combinations. The embodiments can be implemented as an apparatus (a machine) that includes computing hardware (i.e., computing apparatus), such as (in a non-limiting example) any computer that can store, retrieve, process and/or output data and/or communicate (network) with other computers. According to an aspect of an embodiment, the described features, functions, operations, and/or benefits can be implemented by and/or use computing hardware and/or software. The apparatus (e.g., the computer workstations, servers, etc. can comprise a controller (CPU) (e.g., a hardware logic circuitry based computer processor that processes or executes instructions, namely software/program), computer readable media, transmission communication interface (network interface), and/or an output device, for example, a display device, all in communication through a data communication bus. In addition, an apparatus can include one or more apparatuses in computer network communication with each other or other apparatuses. In addition, a computer processor can include one or more computer processors in one or more apparatuses or any combinations of one or more computer processors and/or apparatuses. An aspect of an embodiment relates to causing one or more apparatuses and/or computer processors to execute the described operations. The results produced can be output to an output device, for example, displayed on the display.

A program/software implementing the embodiments may be recorded on a computer-readable media, e.g., a non-transitory or persistent computer-readable medium. Examples of the non-transitory computer-readable media include a magnetic recording apparatus, an optical disk, a magneto-optical disk, and/or volatile and/or non-volatile semiconductor memory (for example, RAM, ROM, etc.). Examples of the magnetic recording apparatus include a hard disk device (HDD), a flexible disk (FD), and a magnetic tape (MT). Examples of the optical disk include a DVD (Digital Versatile Disc), DVD-ROM, DVD-RAM (DVD-Random Access Memory), BD (Blue-ray Disk), a CD-ROM (Compact Disc - Read Only Memory), and a CD-R (Recordable)/RW. The program/software implementing the embodiments may be transmitted over a transmission communication path, e.g., a wire and/or a wireless network implemented via hardware. An example of communication media via which the program/software may be sent includes, for example, a carrier-wave signal.

The many features and advantages of the embodiments are apparent from the detailed specification and, thus, it is intended by the appended claims to cover all such features and advantages of the embodiments that fall within the true spirit and scope thereof. Further, since numerous modifications and changes will readily occur to those skilled in the art, it is not desired to limit the inventive embodiments to the exact construction and operation illustrated and described, and accordingly all suitable modifications and equivalents may be resorted to, falling within the scope thereof.

**Claims**

1.  A method implemented by a computer for monitoring the status of [para 0053] a target vegetation attribute [0005] within a target geographical boundary [0066], comprising:

    generating an allometric model for the target vegetation attribute within the target geographical boundary from a geospatial database [0066]and
    wherein
    generating a geospatial database including remote sensing imagery [claim2 as filed] for monitoring the target vegetation attribute within the target geographical boundary;
    the generating of the allometric model includes either a regression and/or classification function [claim 2, 0064]to predict the target vegetation attribute in the target geographic boundary
    wherein the allometric functions of regressions and/or classifications are based upon a physical sample for a measurement of a vegetation attribute that has a geographical coordinate that is matched to a pixels from remote sensing images with the same geographical coordinate as the physical sample of the vegetation attribute [claims 6 and 8]
    and wherein the sample of the pixels from a remote sensing image are the result of processing by the geospatial data processing software which removes low quality and/or contaminated pixels from the remote sensing image; and creates periodic composites (i.e., monthly, quarterly, annually) for descriptive statistics from pixels within remote sensing images taken for the same location at different dates, where the descriptive statistics are used to generate a consistent time series (i.e., day data to annual data, and then annual data for different years of interest). [para 00260 and 00261] 68 to 70]

2.  The method according to claim 1, wherein a measurement of a vegetation attribute measurement within the target geographical boundary is based upon outputs of the allometric model for regressions and/or classifications, wherein the allometric model of regression and/or classification function(s) relate a physical measurement of a vegetation attribute to digital information in pixels of remote sensing imagery representing another measure for the vegetation attribute.

3.  The method according to claim 2, wherein the allometric function of a regression is generated by data mining software with a function based upon a physical sample of a target vegetation attribute and a pixel sample from remote sensing imagery.

4.  The method according to claim 2, wherein the allometric function of a classification is generated by data mining software with the function based upon a physical sample of the target vegetation attribute and a pixel sample from remote sensing imagery.

5.  The method according to claim 1, wherein the allometric model generated from the function of either a regression and/or classification is used to create a prediction or inference with data mining software that then scores any and/or all pixels in the remote sensing image or composite that was used to develop the regression and/or classification function with a target vegetation attribute.

6.  The method according to claim 5 wherein the output from the scored pixels, from the data mining software, are processed in a geospatial data processing software to create a map or image of the target vegetation attribute.

7.  The method according to claim 2, wherein the target measurement of a vegetation attribute within the target geographical boundary includes processing in geospatial data processing software mapped outputs from the allometric models of regressions and/or classifications for the target geographical boundary.

8.  A geospatial database for use with the method of claims 1 to 7, the database comprising: a set of remote sensing images taken at different dates that fulfills the data continuity requirements for monitoring a vegetation attribute within the target geographical boundaries;

    wherein the raw remote sensing imagery is downloaded and preprocessed with geospatial data processing software to:

        1) sub-set any condensed data files,
        2) remove poor quality pixel information in the remote sensing.imagery,

3) develop descriptive statistics from 2) for any period in time (i.e., month, quarter, year) and/or consistent descriptive statistics for any specified time series that the database encompasses,

and wherein, the output of 3) is connected to a physical sample of a vegetation attribute that will be used with data mining software to develop a function to predict the vegetation attribute for the target geographical boundary. [Figure 7A, paragraphs 00260 to 00264]

9. A geospatial database as claimed in claim 12 wherein the remote sensing imagery is preprocessed by any mixing and/or combining of different remote sensing images and/or raster bands at different spectral frequencies (active or passive) to create consistent descriptive statistics for any specified period in time. [Figure 7A, paragraphs 00260 to 00264]

10. A method as claimed in claim 1 wherein the geospatial data pertaining to 1) remote sensing imagery and 2) vegetation attributes in a vector file is used to overlay a vegetation attribute vector file on the remote sensing imagery, the geospatial data processing software then being used to sample the remote sensing imagery per location in the vegetation attribute vector file. [00264]

11. A geospatial database as claimed in claim wherein the remote sensing imagery is preprocessed by any mixing and/or combining of different remote sensing images and/or bands to create an index of multiple remote sensing images that may or may not be processed as descriptive statistic per pixel. [00282]

102

**1) DELEVOP LEGAL/POLICY ANALYSIS**

- DOWNLOAD LEGAL/POLICY DOCUMENTS FOR GUIDANCE ON MONITORING AND/OR REPORTING A VEGETATION ATTRIBUTE AT A CLIENT'S PROJECT SITE
- REVIEW ALL DOWNLOADED GUIDANCE DOCUMENTS FOR MONITORING AND/OR REPORTING A VEGETATION ATTRIBUTE
- SYNTHESIZE REVIEW ON GUIDANCE DOCUMENTS FOR MONITORING AND/OR REPORTING A VEGETATION ATTRIBUTE INTO ONE DOCUMENT

104

**2) DEVELOP SCIENCE PLAN**

- IDENTIFY AN APPROPRIATE SATELLITE INSTRUMENT(S) THE WILL BE USED TO 1) MONITOR A VEGETATION ATTRIBUTE AT A CLIENT'S PROJECT SITE ANS 2) USED TO DEVELOP A GEOSPATIAL DATABASE
- DEVELOP DIRECTIONS FOR ALLOMETRIC EQUATIONS THAT EXTEND EXISTING METHODS FOR MONITORING A VEGETATION ATTRIBUTE TO MEET THE REQUIRED VEGETATION ATTRIBUTE AMOUNT IDENTIFIED IN THE LEGAL/POLICY ANALYSIS
- DEVELOP DIRECTIONS FOR ALLOMETRIC EQUATIONS THAT USE DATA MINING SOFTWARE WITH INPUT SAMPLES FROM A VEGETATION ATTRIBUTE AND REMOTE SENSING IMAGERY TO MEET THE REQUIRED VEGETATION ATTRIBUTE AMOUNT IDENTIFIED IN THE LEGAL/POLICY ANALYSIS

106

**3) DEVELOP GEOSPATIAL DATABASE**

- DOWNLOAD REMOTE SENSING IMAGERY FROM THE IDENTIFIED INSTRUMENT(S) IN THE SCIENCE PLAN
- DOWNLOAD OTHER GEOSPATIAL DATA THAT IS RELEVANT TO MONITORING AND/OR REPORTING THE VEGETAION ATTRIBUTE AT A PROJECT SITE
- PRE-PROCESS ANY RAW REMOTE SENSING IMAGERY INTO A STANDARD DATA FORMAT, REMOVE POOR QUALITY INFORMATION AND DEVELOP QUALITATIVE STATISTICS
- BUILD A VECTOR FILE CONTAINING ALL FREELY AVAILABLE GEOSPATIAL DATA FROM PUBLICLY DISCLOSED VEGETATION ATTRIBUTES
- OBTAIN AND DOWNLOAD GEOSPATIAL DATA ON VEGETAION ATTRIBUTES FROM A CLIENT

108

**4) DEVELOP ALLOMETRIC EQUATIONS**

- <u>ALLOMETRICS 1:</u>RUN A PROCESSED-BASE DYNAMIC ECOSYSTEM MODELING SOFTWARE WITH INPUT DATA TO EXTEND EXISTING LEGALLY IRRELEVANT GEOSPATIAL DATA TO NEW LEGALLY RELEVANT GEOSPATIAL DATA AS DEFINED BY THE DIRECTIONS IN THE SCIENCE PLAN
- <u>ALLOMETRICS 2:</u>RUN DATA MINING SOFTWARE WITH INPUT DATA TO DEVELOP A TRAINING MODEL FOR REGRESSION AND/OR CLASSIFICATION PREDICTORS TO BE USED WITH A REMOTE SENSING IMAGE AS DEFINED BY THE DIRECTIONS IN THE SCIENCE PLAN

110

**5) IMPLEMENT ALLOMETRIC EQUATIONS**

- <u>ALLOMETRICS 1:</u>RUN NEWLY DEVELOPED ALLOMETRIC EQUATIONS 1 WITH GEOSPATIAL DATA FROM REMOTE SENSING IMAGERY TO CREATE A MAP OF VEGETATION ATTRIBUTE THE FULFILLS THE DIRECTIONS IN THE SCIENCE PLAN
- <u>ALLOMETRICS 2:</u>RUN NEWLY DEVELOPED ALLOMETRIC EQUATIONS 2 WITH GEOSPATIAL DATA FROM REMOTE SENSING IMAGERY TO CREATE A MAP OF VEGETATION ATTRIBUTE THE FULFILLS THE DIRECTIONS IN THE SCIENCE PLAN

**6) OBTAIN CLIENT'S BOUNDARY**

- OBTAIN AN ELECTRONIC GEOSPATIAL DATA FILE FOR THE SPATIAL BOUNDARY OF A PROJECT SITE FROM A CLIENT THROUGH AN INTERNET INTERFACE AND DOWNLOAD THE FILE

112

**7) SAMPLE CLIENT'S BOUNDARY**

- SAMPLE THE CLIENT'S GEOREFERENCED BOUNDARY WITH THE OUTPUTS OF ALLOMETRIC EQUATIONS 1 AND/OR ALLOMETRICS 2 AND ANY OTHER GEOSPATIAL DATA STORED ON THE GEOSPATIAL DATABASE

114

**8) DEVELOP REPORT**

- DEVELOP A REPORT ON THE SAMPLED RESULTS FOR THE CLIENT'S GEOREFERENCED BOUNDRY

116

## FIG. 1

COMPUTING ENVIRONMENT

START

**202**
COMPUTER WORKSTATION

**204**
ACCESS WEBSITE(S) FOR REGULATED AND/OR VOLUNTARY CARBON TRADING MECHANISM(S)

**206**
DOWNLOAD ANY AND/OR ALL GUIDANCE DOCUMENTS

**208**
SAVE GUIDANCE DOCUMENTS TO DATABASE 1

**210**
ACCESS GUIDANCE DOCUMENTS STORED ON DATABASE 1

**212**
ACCESS TEXT RETRIEVAL SOFTWARE

STRUCTURED ANALYSIS

**230**
**224**
SUMMARY REVIEW DOCUMENT ON GUIDANCE FOR INTERNATIONAL AND BI-LATERAL AGREEMENTS

**226**
SUMMARY REVIEW DOCUMENT ON GUIDANCE FOR INTERNATIONAL, NATIONAL AND/OR REGIONALLY REGULATED TRADING MECHANISMS

**228**
SUMMARY REVIEW DOCUMENT ON GUIDANCE FOR VOLUNTARY TRADING MECHANISMS BETWEEN LEGAL ENTITIES

**232**
LINK SUMMARY REVIEWS ON GUIDANCE FOR MONITORING AND REPORTING BASTED ON THE RELEVANT TRADING MECHANISM(S) TO THE CLIENT

**222**
ACCESS SUMMARY DOCUMENTS WITH WORD PROCESSING SOFTWARE STORED ON DATABASE 1

**220**
**216**
SAVE OUTPUTS TO DATABASE 1

KEY WORD RETRIEVAL

**214**
RETRIEVE TEXT FOR GUIDANCE DOCUMENTS FROM INTERNATIONAL MULTI-LATERAL AND BI-LATERAL AGREEMENTS

RETRIEVE TEXT FOR GUIDANCE DOCUMENTS FROM INTERNATIONAL, NATIONAL AND/OR REGIONALLY REGULATED CARBON TRADING MECHANISMS

**218**
RETRIEVE TEXT FOR GUIDANCE DOCUMENTS FROM VOLUNTARY CARBON TRADING MECHANISMS BETWEEN LEGAL ENTITIES

**234**
SAVE OUTPUT TO DATABASE 1

**236**
ACCESS DATABASE 1

**238**
COPYRIGHT 1-PRINT OUT LINKED SUMMARY AS EITHER A .PDF (OR SIMILAR DOCUMENT FORMAT) THAT IS SAVED ON DATABASE 1 AND/OR ON A HARD COPY

**240**
PRINTER

GO TO FIG.28

→ THE CLOSED ARROW IN THE PROCESS MEANS ANY AND ALL MIX OF, COMBINATION OF, AND/OR USE OF ALL PHYSICAL ELEMENTS DEFINED AS A COMPUTER WORKSTATION

→ THE OPEN ARROW IN THE PROCESS MEANS THE PHYSICAL INTERFACE WITH THE USER

## FIG. 2A

FIG.2B

COMPUTING ENVIRONMENT

START

304

ACCESS GUIDANCE DOCUMENTS STORED ON DATABASE 1

306

ACCESS WORD PROCESSING SOFTWARE

302

COMPUTER WORKSTATION

324

NATIONAL MRV

308

UN AND OTHER MULTI-LATERAL AND/OR BI-LATERAL AGREEMENTS AND GUIDANCE RELATED TO MONITOR A VEGETATION ATTRIBUTE

SUMMARY FOR REGULATED MARKETS

320

310

314

UN, NATIONAL AND/OR REGIONAL POLICY ON MONITORING A VEGETATION ATTRIBUTE UNDER A REGULATED TRADING MECHANISM

318

SUMMARY FOR VOLUNTARY MARKETS

312

316

POLICY ON MONITORING A VEGETATION ATTRIBUTE UNDER A VOLUNTARY TRADING MECHANISM

322

SAVE OUTPUT TO DATABASE 1

326

THE CLOSED ARROW IN THE PROCESS MEANS ANY AND ALL MIX OF, COMBINATION OF, AND/OR USE OF ALL PHYSICAL ELEMENTS DEFINED AS A COMPUTER WORKSTATION

THE OPEN ARROW IN THE PROCESS MEANS THE PHYSICAL INTERFACE WITH THE USER

GO TO FIG.2D

FIG.2C

COMPUTING ENVIRONMENT

START

COMPUTER WORKSTATION
10202

10204
ACCESS IPCC, UNFCCC, UNCDM, VCS AND CCB WEBSITE(S)

10206
DOWNLOAD GUIDELINES FOR MONITORING AND REPORTING

10208
SAVE GUIDANCE DOCUMENTS TO DATABASE 1

10210
ACCESS GUIDANCE DOCUMENTS STORED ON DATABASE 1

10212
ACCESS TEXT RETRIEVAL SOFTWARE

STRUCTURED ANALYSIS

10230
10224
CONDENSE RETRIEVEDD SUMMARY OF IPCC GPGs TO SPECIFIC ELEMENTS RELEVANT TO A CLIENT

10232
LINK CONDENSE SUMMARIES ON GUIDANCE FOR MONITORING AND REPORTING AND EXPLAIN WHAT THIS MEANS TO THE CLIENT

10226
CONDENSE RETRIEVED SUMMARY OF UN CDM/JI METHODS TO SPECIFIC LANGUAGE RELEVANT TO A CLIENT

10222
ACCESS GUIDANCE SUMMARY DOCUMENTS STORED ON DATABASE 1

10220
SAVE OUTPUTS TO DATABASE 1

KEY WORD RETRIEVAL

10214
RETRIEVE TEXT FROM THE KYOTO PROTOCOL AND IPCC GPGS

10216
RETRIEVE TEXT FROM UN CDM/JI METHODS DOCUMENT(S) ON AFFORESTATION AND REFORESTATION

10228
CONDENSE RETRIEVED SUMMARY OF VCS/ CCB GUIDANCE TO SPECIFIC LANGUAGE RELEVANT TO A CLIENT

10218
RETRIEVE TEXT FROM VCS AND CCB GUIDANCE DOCUMENT(S)

10234
SAVE OUTPUT TO DATABASE 1

10236
ACCESS DATABASE 1

10238
COPYRIGHT 1-PRINT OUT LINKED SUMMARY AS EITHER A .PDF (OR SIMILAR DOCUMENT FORMAT) THAT IS SAVED ON DATABASE 1 AND/OR ON A HARD COPY

10240
PRINTER

THE CLOSED ARROW IN THE PROCESS MEANS ANY AND ALL MIX OF, COMBINATION OF, AND/OR USE OF ALL PHYSICAL ELEMENTS DEFINED AS A COMPUTER WORKSTATION

THE OPEN ARROW IN THE PROCESS MEANS THE PHYSICAL INTERFACE WITH THE USER

GO TO FIG.4A

FIG. 2D

FIG.3

1702

ABOVE-GROUND BIOMASS

BELOW-GROUND BIOMASS

HARVESTED WOOD PRODUCTS

LITTER

DEAD WOOD

SOIL ORGANIC MATTER

⇨ INCREASE OF CARBON STOCKS DUE TO GROWTH

⇨ TRANSFER OF CARBON BETWEEN POOLS

➡ CARBON FLUXES DUE TO DISCRETE EVENTS, I.E., FROM HARVEST RESIDUES AND NATURAL DISTURBANCE

⇨ CARBON FLUXES DUE TO CONTINUOUS PROCESSES, I.E., DECOMPOSITION

START — 1704

ARE DETAILED DATA ON BIOMASS AVAILABLE TO ESTIMATE CHANGES IN C STOCKS USING DYNAMIC MODELS OR ALLOMETRIC EQUATIONS?

YES → USE THE DETAILED BIOMASS DATA FOR TIER 3 METHOD

BOX 3: TIER 3

NO

ARE COUNTRY-SPECIFIC BIOMASS DATA AND EMISSION/REMOVAL FACTORS AVAILABLE?

YES → USE COUNTRY-SPECIFIC BIOMASS DATA AND EMISSION/REMOVAL FACTORS FOR THE TIER 2 METHOD

BOX 2: TIER 2

NO

ARE CHANGES IN C STOCKS IN BIOMASS IN THIS LAND CLASSIFICATION A KEY CATEGORY?

NO → ARE AGGREGATE DATA ON BIOMASS GROWTH AND LOSS AVAILABLE?

NO → GATHER DATA ON BIOMASS GROWTH AND BIOMASS LOSS

YES

COLLECT DATA FOR THE TIER 3 OR TIER 2 METHOD

YES → USE AGGREGATE DATA AND DEFAULT EMISSION/REMOVAL FACTORS FOR TIER 1 METHOD

BOX 1: TIER 1

COMPUTING ENVIRONMENT

START

COMPUTER WORKSTATION
12302

ACCESS TEXT RETRIEVAL SOFTWARE
12304

ACCESS CEOS HANDBOOK DATABASE STORED ON DATABASE 2
12306

ACCESS SPREADSHEET SOFTWARE
12308

SPREADSHEET SOFTWARE

TEXT RETRIEVAL SOFTWARE

KEY WORD META-DATABASE

LEVEL 1 KEY WORD SEARCH
12310

LEVEL 2 KEY WORD SEARCH
12318

OUTPUTS FROM LEVEL 1 SEARCH
12316

TEXT SEARCH AND RETRIEVE
12324
12312

CEOS HANDBOOK DATABASE
12344

OUTPUTS FROM LEVEL 2 SEARCH
12320

SAVE OUTPUT TO DATABASE 2
12322

GOT TO FIG.4B

→ THE CLOSED ARROW IN THE PROCESS MEANS ANY AND ALL MIX OF, COMBINATION OF, AND/OR USE OF ALL PHYSICAL ELEMENTS DEFINED AS A COMPUTER WORKSTATION

→ THE OPEN ARROW IN THE PROCESS MEANS THE PHYSICAL INTERFACE WITH THE USER

FIG. 4A

EP 2 980 735 A1

12518

CLIENT'S FULL
PROJECT TIMELINE

DATA
CONTINUITY

SENSOR-6
SENSOR-5
SENSOR-4
SENSOR-3
SENSOR-2
SENSOR-1

12516

12514  12502

$t_0$ $t_1$ $t_2$ $t_3$ $t_4$ $t_5$ $t_6$ $t_7$ $t_8$ $t_9$ $t_{10}$ $t_{11}$ $t_{12}$ $t_{13}$ $t_{14}$ $t_{15}$ $t_{16}$ $t_{17}$ $t_{18}$ $t_{19}$ $t_{20}$ $t_{21}$ $t_{22}$ $t_{23}$ $t_{24}$ $t_{25}$ $t_{26}$ $t_{27}$ $t_{28}$ $t_{29}$ $t_{30}$ $t_{31}$ $t_{32}$ $t_{33}$ $t_{34}$ $t_{35}$ $t_{36}$ $t_{37}$ $t_{38}$ $t_{39}$ $t_{40}$

YEAR

12504 — INITIAL BASELINE FOR VEGETATION ATTRIBUTE

12506 → PREDICTED PROJECT OFFSET FROM BASELINE ASSESSMENT

12512 → ANNUAL MONITORING FOR PROJECT TO PROVE ACTUAL OFFSET

12508 — VALIDATION OF BASELINE

VERIFICATION | VERIFICATION | VERIFICATION | VERIFICATION | VERIFICATION | VERIFICATION

12510

IS THE LIFE OF A SATELLITE MISSION WITH A REMOTE SENSING INSTRUMENT
IS THE DATA PROVISION REQUIREMENTS FOR A PROJECT DEVELOPER'S PROJECT WITH A 10 YEAR INITIAL BASELINE AND 30 PROJECT LIFETIME

FIG.4B

FIG.4C

COMPUTING ENVIRONMENT

START

COMPUTER WORKSTATION
402

ACCESS WORD PROCESSING SOFTWARE
406

ACCESS LEGAL/ POLICY REVIEW AND PULL TEXT REVIEWING WHAT IS REQUIRED IN MONITORING A VEGETATION ATTRIBUTE
408

ACCESS RETRIEVED TEXT FROM THE OUTPUTS FROM THE INTELLIGENCE ANALYSIS, EDIT AND SYNTHESIS TEXT
410

SPECIFY EQUATIONS, METHODS, SOFTWARE AND INPUT DATA TO FULFILL COMPLIANCE REQUIREMENTS
412

SAVE OUTPUT TO DATABASE 2
414

ACCESS DATABASE 2
416

COPYRIGHT 2- PRINT OUT SCIENCE PLAN AS EITHER A .PDF THAT IS SAVED ON DATABASE 2 AND/OR IN HARD COPY
418

ACCESS COPYRIGHT 1 FROM STEP **238** THAT IS A LEGAL/POLICY ANALYSIS SAVED ON DATABASE 1 AND/OR PRINTED OUT ON HARD COPY
404

PRINTER
420

THE CLOSED ARROW IN THE PROCESS MEANS ANY AND ALL MIX OF, COMBINATION OF, AND/OR USE OF ALL PHYSICAL ELEMENTS DEFINED AS A COMPUTER WORKSTATION

THE OPEN ARROW IN THE PROCESS MEANS THE PHYSICAL INTERFACE WITH THE USER

GO TO FIG.4E

FIG. 4D

COMPUTING ENVIRONMENT

START

ACCESS COPYRIGHT 1-
SEE EXAMPLE OF
LEGAL/POLICY REVIEW
FROM STEP **10238** IN
THE DETAILED
DESCRIPTION

10404

COMPUTER
WORKSTATION

10402

ACCESS WORD
PROCESSING
SOFTWARE

10406

REVIEW THE
SPECIFICATIONS
FOR COMPLIANCE-
SEE SECTION 1
IN THE EXAMPLE
OF A SCIENCE
PLAN

10408

ACCESS RETRIEVED
TEXT FOR MODIS
RELATED JOURNAL
ARTICLES, EDIT AND
SYNTHESIS TEXT-
SEE SECTION 1.27
IN THE EXAMPLE OF
A SCIENCE PLAN

10410

SPECIFY EQUATIONS,
METHODS, SOFTWARE
AND INPUT DATA TO
FULFILL COMPLIANCE
REQUIREMENTS-SEE
SECTIONS 2-5
IN THE EXAMPLE OF
A SCIENCE PLAN

10412

SAVE OUTPUT
TO DATABASE 2          10414

ACCESS DATABASE 2          10416

COPYRIGHT 2-PRINT OUT
SCIENCE PLAN AS EITHER
A .PDF THAT IS SAVED ON
DATABASE 2 AND/OR IN
HARD COPY          10418

PRINTER          10420

THE CLOSED ARROW IN THE PROCESS MEANS ANY AND ALL MIX OF, COMBINATION OF,
AND/OR USE OF ALL PHYSICAL ELEMENTS DEFINED AS A COMPUTER WORKSTATION

THE OPEN ARROW IN THE PROCESS MEANS THE PHYSICAL INTERFACE WITH THE USER

GO TO
FIG.6A

FIG. 4E

EP 2 980 735 A1

62

FIG.5A

FIG.5B

FIG.5C

FIG.5D-1

| 8-DAY INCREMENT | AVERAGE COEFFICIENT OF VARIATION (%) FOR FOREST LAND BETWEEN 2001-2009 | | | | | |
|---|---|---|---|---|---|---|
| | NDVI | EVI | RED | NIR | BLUE | MIR |
| 1 | 13.78 | 14.75 | 21.31 | 14.37 | 29.00 | 18.54 |
| 9 | 23.47 | 14.79 | 21.12 | 15.53 | 32.10 | 19.92 |
| 17 | 19.92 | 20.60 | 26.77 | 20.63 | 35.82 | 24.26 |
| 25 | 24.54 | 16.35 | 24.94 | 16.26 | 33.47 | 20.30 |
| 33 | 15.50 | 16.74 | 23.42 | 17.42 | 30.74 | 20.99 |
| 41 | 23.38 | 15.74 | 24.74 | 16.20 | 33.24 | 20.10 |
| 49 | 11.74 | 13.61 | 20.24 | 14.69 | 28.33 | 18.18 |
| 57 | 23.56 | 16.56 | 26.03 | 17.45 | 32.49 | 21.18 |
| 65 | 17.07 | 18.01 | 25.55 | 18.80 | 35.39 | 21.83 |
| 73 | 13.45 | 14.69 | 14.76 | 15.36 | 32.48 | 19.24 |
| 81 | 9.64 | 11.48 | 19.41 | 12.38 | 36.08 | 15.70 |
| 89 | 22.85 | 15.25 | 27.18 | 16.05 | 34.82 | 20.16 |
| 97 | 17.94 | 18.79 | 26.49 | 19.42 | 32.01 | 22.25 |
| 105 | 27.97 | 19.41 | 30.64 | 19.88 | 38.36 | 23.41 |
| 113 | 15.21 | 15.95 | 25.05 | 16.06 | 30.04 | 20.12 |
| 121 | 21.90 | 13.06 | 25.68 | 13.61 | 32.72 | 17.77 |
| 129 | 17.26 | 17.48 | 25.80 | 17.78 | 31.00 | 20.85 |
| 137 | 25.32 | 15.72 | 27.64 | 16.02 | 34.78 | 20.42 |
| 145 | 29.59 | 29.80 | 37.32 | 30.04 | 41.46 | 33.15 |
| 153 | 18.59 | 18.78 | 29.63 | 18.98 | 35.84 | 23.55 |
| 161 | 29.73 | 29.90 | 36.99 | 29.89 | 41.09 | 33.30 |
| 169 | 24.92 | 15.74 | 24.42 | 15.60 | 31.44 | 20.09 |
| 177 | 21.83 | 22.38 | 28.07 | 22.35 | 33.32 | 25.90 |
| 185 | 22.11 | 14.90 | 22.75 | 14.66 | 30.23 | 19.07 |
| 193 | 28.03 | 22.25 | 27.94 | 22.10 | 32.71 | 25.98 |
| 201 | 41.61 | 34.82 | 41.34 | 34.44 | 47.36 | 38.22 |
| 209 | 20.30 | 21.01 | 26.90 | 20.81 | 31.87 | 24.54 |
| 217 | 45.20 | 39.66 | 46.85 | 39.38 | 52.88 | 43.03 |
| 225 | 40.50 | 40.98 | 46.59 | 41.09 | 49.60 | 44.01 |
| 233 | 44.50 | 44.31 | 52.32 | 44.27 | 59.09 | 47.72 |
| 241 | 48.22 | 48.84 | 54.12 | 49.04 | 56.36 | 51.84 |
| 249 | 52.40 | 46.56 | 54.88 | 46.70 | 62.80 | 49.95 |
| 257 | 68.77 | 62.98 | 68.77 | 63.35 | 72.61 | 65.81 |
| 265 | 46.70 | 40.46 | 49.91 | 41.01 | 59.18 | 44.21 |
| 273 | 62.06 | 62.19 | 69.47 | 62.61 | 76.42 | 65.38 |
| 281 | 35.81 | 31.02 | 41.07 | 31.71 | 51.22 | 34.88 |
| 289 | 54.77 | 54.99 | 62.29 | 55.37 | 67.29 | 57.79 |
| 297 | 22.11 | 23.16 | 34.52 | 23.99 | 46.51 | 27.42 |
| 305 | 43.81 | 43.80 | 52.03 | 44.17 | 60.26 | 46.54 |
| 313 | 19.75 | 20.64 | 32.34 | 21.38 | 43.76 | 25.05 |
| 321 | 40.00 | 40.11 | 48.11 | 40.38 | 57.54 | 43.25 |
| 329 | 18.24 | 18.84 | 29.62 | 19.40 | 41.05 | 23.41 |
| 337 | 28.59 | 28.75 | 36.60 | 28.95 | 47.36 | 32.23 |
| 345 | 18.40 | 18.97 | 28.46 | 19.13 | 38.90 | 23.60 |
| 353 | 17.93 | 18.59 | 25.04 | 18.44 | 32.55 | 22.41 |
| 361 | 12.57 | 13.51 | 21.35 | 13.15 | 29.80 | 17.41 |
| ANNUAL MEAN | 3.14 | 3.65 | 11.62 | 3.72 | 18.98 | 7.22 |
| ANNUAL MAXIMUM | 2.57 | 6.66 | 33.72 | 7.62 | 36.63 | 23.69 |
| ANNUAL MINIMUM | 46.49 | 18.11 | 31.76 | 16.32 | 54.36 | 20.90 |

2226

FIG.5D-2

**Computing Environment**

Start

Computer Workstation ~502

Access Copyright 2 from step 418 that is a science plan saved on Database 2 and/or a print out on hard copy ~504

Access website(s) for standard input geospatial data ~506

Access website for pay per acquisition remote sensing imagery ~508

Obtain project specific geospatial data from client ~510

Download input variables ~512

Database 3 ~514

- Standard database for freely available MODIS class imagery;
- Secondary database for freely available Landsat class imagery;
- Freely available climate data;
- Freely available elevation data;
- Freely available soil data;
- Freely available vegetation attribute data;
- Peer-review literature and trading mechanism reports containing geospatial reference to vegetation attributes;
- Official government disclosures for vegetation attributes;
- Vegetation attribute disclosures to trading mechanisms;
- Other freely available digital geospatial data that is useful for monitoring and/or reporting a vegetation attribute(s) at a project site and/or defined by the Science Plan;
- Other remote sensing imagery and relevant geospatial data that iks not freely available, but is defined as necessary to monitor the project site by either the Science Plan and/or the client

The closed arrow in the process means any and all mix of, combination of, And/or use of all physical elements defined as a computer workstation

The open arrow in the process means the physical interface with the user

Go to figure 7a

FIG.6A

```
ncols          4800
nrows          4800
xllcorner      1111950.519667
yllcorner      -1111950.519667
cell size      231.65636826396
NODATA value   -9999
6923 7254 7485 7047 7033 7178 7058 6552
6495 6887 7347 7090 7327 7193 6943 6733
6745 6806 6935 6711 7078 7351 7037 6800
6955 6730 6842 6579 6749 7391 7384 7092
6980 6745 7123 6816 6892 7589 7404 7233
6783 6856 7123 7022 7018 7534 7322 7540
6946 6947 6866 7134 7077 7479 7308 7348
7136 7019 6834 7030 6804 7273 7432 7487
7119 7164 7062 6902 6874 7060 7390 7312
7204 7144 7038 6837 6945 6945 7170 6977
7014 6910 7071 6865 6955 6864 7013 6603
6954 7150 7197 6871 6670 6841 6465 6348
7107 7420 7458 6920 6675 6855 6863 6633
6743 7032 7548 7129 7032 7249 7319 6860
6356 6796 7417 7053 7203 7440 7500 7126
6602 7104 7395 7145 7267 7450 7362 7001
6768 6955 7148 7285 7321 7337 7316 6901
6477 6864 6975 7071 7215 6969 7085 6788
6594 6663 6799 7100 7229 7093 7030 7317
6739 6704 6814 6857 6747 6760 6553 6710
6716 6713 6810 7010 6699 6705 6433 6433
6814 6626 6658 6918 6817 6927 6839 6999
7150 6856 6781 6866 7017 6757 6741 6487
7095 6780 6732 6757 6660 6613 6670 6330
7077 6774 6840 6831 6685 6849 6660 6491
6585 6456 6893 6796 7040 6760 6604 6517
6877 6671 7346 7333 7033 6966 6840 6620
6815 6960 7301 7354 7197 7127 7297 6985
7231 7219 7475 7444 7156 6998 7303 7324
7317 7357 7732 7351 7264 7106 7494 7189
7144 7213 7564 7453 7083 7038 7269 6866
6896 7088 7125 7360 6982 6859 6929 7425
7070 7039 7072 7093 7134 7174 7145 7416
6803 6907 7145 7323 7511 7671 7501 7577
6708 6711 6775 6961 7282 7417 7235 7247
6787 6413 6591 7035 7031 7203 7134 7457
6672 6505 6908 7152 7188 7061 7286 7350
6817 6651 6666 7019 6832 7375 7059 7260
6805 6934 7255 7328 7076 7456 7321 7240
6840 7101 7425 7278 7188 7095 7148 7084
7109 6989 7073 7283 7464 7203 7232 7094
6962 7030 6871 6762 6915 7124 6988 6680
```

| ◿ | A | B | C |
|---|---|---|---|
| 1 | FID | Gridcode | NDVI |
| 2 | 0 | 1 | 7636 |
| 3 | 1 | 2 | 7551 |
| 4 | 2 | 3 | 7530 |
| 5 | 3 | 4 | 7780 |
| 6 | 4 | 5 | 7637 |
| 7 | 5 | 6 | 7551 |
| 8 | 6 | 7 | 7836 |
| 9 | 7 | 8 | 7515 |
| 10 | 8 | 9 | 7479 |
| 11 | 9 | 10 | 7539 |
| 11 | 10 | 11 | 7498 |
| 13 | 11 | 12 | 7737 |
| 14 | 11 | 13 | 7637 |
| 15 | 13 | 14 | 7567 |
| 16 | 14 | 15 | 7487 |
| 17 | 15 | 16 | 7549 |
| 18 | 16 | 17 | 7300 |
| 19 | 17 | 18 | 7474 |
| 20 | 18 | 19 | 7502 |
| 21 | 19 | 20 | 7414 |
| 22 | 20 | 21 | 7419 |
| 23 | 21 | 22 | 7568 |
| 24 | 22 | 23 | 7160 |
| 25 | 23 | 24 | 7465 |
| 26 | 24 | 25 | 7537 |
| 27 | 25 | 26 | 7662 |
| 28 | 26 | 27 | 7636 |

FIG.6B

69

COMPUTING ENVIRONMENT

ACCESS DATABASE 3 — 608

ACCESS GEOSPATIAL DATA PROCESSING SOFTWARE — 606

PROCESS REMOTE SENSING IMAGERY FROM CONDENSED FILES TO SUBSETS

PROCESS REMOTE SENSING IMAGERY FOR QUALITY CONTROL

PROCESS INCREMENTAL COMPOSITES BY QUALITATIVE STATISTICS

START

COMPUTER WORKSTATION

610

612

614

602

ACCESS STANDARD GEOSPATIAL DATA FROM KNOWN OBSERVATIONS FOR VEGETATION ATTRIBUTE(S) — 616

SAVE OUTPUTS TO DATABASE 3

622

PROCESS REMOTE SENSING IMAGERY FROM CONDENSED FILES TO SUBSETS TO PROCESS GEOSPATIAL DATA FOR VEGETATION ATTRIBUTES INTO ONE VECTOR FILE — 620

ACCESS COPYRIGHT 2 FROM STEP 418 ACCESS THAT IS A SCIENCE PLAN SAVED ON DATABASE 2 AND/OR A PRINT OUT ON HARD COPY

ACCESS CLIENT'S GEOSPATIAL DATA FOR OBSERVATIONS FOR VEGETATION ATTRIBUTE(S) AT A PROJECT SITE — 618

604

THE CLOSED ARROW IN THE PROCESS MEANS ANY AND ALL MIX OF, COMBINATION OF, AND/OR USE OF ALL PHYSICAL ELEMENTS DEFINED AS A COMPUTER WORKSTATION

THE OPEN ARROW IN THE PROCESS MEANS THE PHYSICAL INTERFACE WITH THE USER

GO TO FIG.7B

FIG.7A

EP 2 980 735 A1

COMPUTING ENVIRONMENT

PROCESS 8/16-DAY EVI TO ANNUAL EVI COMPOSITE — 10714

PROCESS MODIS MOD 13 EVI FOR BAND 3 AND/OR 12 — 10712

PROCESS MODIS MOD 13 FROM CONDENSED .HDF FILE TO SUB-SET FILE FOR EVI — 10710

SAVE OUTPUTS TO DATABASE 3 — 10722

10720

PROCESS REMOTE SENSING IMAGERY FROM CONDENSED FILES TO SUBSETS TO PROCESS GEOSPATIAL DATA FOR VEGETATION ATTRIBUTES INTO ONE VECTOR FILE — 10718

GO TO FIG.8A AND/OR 9A

ACCESS DATABASE 3 — 10708

ACCESS ArcGIS — 10706

ACCESS STANDARD GEOSPATIAL DATA FROM KNOWN OBSERVATIONS FOR VEGETATION ATTRIBUTE(S) — 10716

ACCESS CLIENT'S GEOSPATIAL DATA FOR OBSERVATIONS FOR VEGETATION ATTRIBUTE(S) AT A PROJECT SITE

COMPUTER WORKSTATION — 10702

START

ACCESS COPYRIGHT 2-IMPLEMENT PRE-MODELING IMAGE PROCESSING, SEE SCIENCE PLAN: DIRECTIONS 3 FROM STEP 10418 IN THE DETAILED DESCRIPTIONS — 10704

→ THE CLOSED ARROW IN THE PROCESS MEANS ANY AND ALL MIX OF, COMBINATION OF, AND/OR USE OF ALL PHYSICAL ELEMENTS DEFINED AS A COMPUTER WORKSTATION

→ THE OPEN ARROW IN THE PROCESS MEANS THE PHYSICAL INTERFACE WITH THE USER

FIG.7B

**Computing Environment**

Start

702 — Computer Workstation

704 — Access Copyright 2 from step **418** that is a science plan saved on Database 2 and/or a print out on hard copy

706 — Access dynamic ecosystem modelling software

708 — Access input data on Database 3

712 — Access geospatial data processing software

710 — Process input data with dynamic ecosystem model to fulfill elements of the science plan

714 — Access outputs of 710 and collect summary statistics for Allometrics 1 and input into spread sheet

716 — Save outputs to Database 4

718 — Access Database 4

720 — Copyright 3-Print out material stored on Database 4 as a spreadsheet save on Database 4 and/or in hard copy

722 — Printer

Go to Figure 8b

The closed arrow in the process means any and all mix of, combination of, And/or use of all physical elements defined as a computer workstation

The open arrow in the process means the physical interface with the user

FIG.8A

**Computing Environment**

Start → Computer Workstation

10902 → Access ArcGIS (10912)

10914 — Sample and collect summary statistics for Allometrics 1, such as allometrics A and B in science plan 1

10916 — Save outputs to Database 4

10918 — Access Database 4

10908 — Access input data for Biome-BGC, such as climate data, land cover map, elevation data etc.

10904 — Access Copyright 2-Implement science plan from step **10418** to develop Allometrics 1, see Science Plan: Directions 1 in the DETAILED DESCRIPTION

10906 — Access Biome-BGC

10910 — Process Biome-BGC with input data to fulfill elements of the science plans 1 and 2

Copyright 3-Print out material stored on Database 4 as a spreadsheet, such a Tables 2-4

10922 — Printer

→ The closed arrow in the process means any and all mix of, combination of, And/or use of all physical elements defined as a computer workstation

→ The open arrow in the process means the physical interface with the user

Go to Figure 11a

FIG.8B

Computing Environment

Start

802
Computer
Workstation

806
Access geospatial
data processing
software

804
Access Copyright 2 from step
**418** that is a science plan saved
on Database 2 and/or a print out
on hard copy

808
Access
geospatial data
on Database 3

810
Overlay point file
with remote sensing
imagery and sample
pixel in image for
individual points

812
Enter outputs in
spreadsheet

814
Save outputs
to Database 5

Go to Figure 9b

→ The closed arrow in the process means any and all mix of, combination of,
And/or use of all physical elements defined as a computer workstation

⇨ The open arrow in the process means the physical interface with the user

FIG.9A

EP 2 980 735 A1

COMPUTING ENVIRONMENT

START

11002

COMPUTER
WORKSTATION

11006
ACCESS ArcGIS

11008
ACCESS 2009 MEAN/MAX
NDVI AND MEAN EVI AND
SFM-AFRICA POINT
FILE DATABASE 3

11010
OVERLAY SFM-AFRICA
POINT FILE ON MAX
NDVI AND MEAN EVI
IMAGERY AND SAMPLE
IMAGERY FOR
INDIVIDUAL POINTS

11012
ENTER OUTPUTS
IN SPREADSHEET

11014
SAVE OUTPUT TO
DATABASE 5

11004
ACCESS COPYRIGHT 2
FROM STEP **10418**-
IMPLEMENT SCIENCE PLAN
TO DEVELOP ALLOMETRICS
2, SEE SCIENCE PLAN:
DIRECTIONS 4 IN THE
DETAILED DESCRIPTION

The closed arrow in the process means any and all mix of, combination of,
And/or use of all physical elements defined as a computer workstation

The open arrow in the process means the physical interface with the user

GO TO FIG.10A

FIG.9B

EP 2 980 735 A1

11104      11108

11102      11106

| FID | SHAPE | PLOTCODE | ZONE | ForType | LONGITUDE | LATITUDE |
|---|---|---|---|---|---|---|
| 0 | Point | 1011 | 12 | 30 | 10.7926 | -3.495 |
| 1 | Point | 1013 | 12 | 30 | 10.791 | -3.4942 |
| 2 | Point | 1015 | 12 | 30 | 10.7894 | -3.4936 |
| 3 | Point | 1017 | 12 | 30 | 10.7874 | -3.4923 |
| 4 | Point | 1019 | 12 | 30 | 10.786 | -3.4915 |
| 5 | Point | 1021 | 12 | 62 | 10.7838 | -3.4904 |
| 6 | Point | 1023 | 12 | 62 | 10.7821 | -3.4895 |
| 7 | Point | 1025 | 12 | 62 | 10.7811 | -3.4893 |
| 8 | Point | 1027 | 12 | 62 | 10.7797 | -3.4883 |
| 9 | Point | 1029 | 12 | 62 | 10.7785 | -3.4874 |
| 10 | Point | 1031 | 12 | 10 | 10.7768 | -3.4865 |
| 11 | Point | 1037 | 12 | 10 | 10.7719 | -3.4842 |
| 12 | Point | 1041 | 12 | 41 | 10.7684 | -3.4825 |
| 13 | Point | 1043 | 12 | 41 | 10.7669 | -3.4808 |
| 14 | Point | 1045 | 12 | 41 | 10.7654 | -3.4808 |
| 15 | Point | 1047 | 12 | 41 | 10.764 | -3.4801 |
| 16 | Point | 1051 | 12 | 41 | 10.7608 | -3.4785 |
| 17 | Point | 1053 | 12 | 41 | 10.7592 | -3.4777 |
| 18 | Point | 1055 | 12 | 41 | 10.7578 | -3.477 |
| 19 | Point | 1057 | 12 | 41 | 10.7562 | -3.4762 |
| 20 | Point | 1059 | 12 | 41 | 10.7547 | -3.4754 |
| 21 | Point | 1061 | 11 | 62 | 10.7532 | -3.4746 |
| 22 | Point | 1063 | 11 | 62 | 10.7516 | -3.4738 |
| 23 | Point | 1065 | 11 | 62 | 10.7402 | -3.473 |
| 24 | Point | 1067 | 11 | 62 | 10.7486 | -3.4723 |
| 25 | Point | 1069 | 11 | 62 | 10.747 | -3.4713 |
| 26 | Point | 1071 | 11 | 62 | 10.7455 | -3.4706 |
| 27 | Point | 1073 | 11 | 62 | 10.744 | -3.4698 |
| 28 | Point | 1075 | 11 | 62 | 10.7424 | -3.4689 |

FIG. 9C

COMPUTING ENVIRONMENT

START

COMPUTER WORKSTATION
902

ACCESS COPYRIGHT 2 FROM STEP **418** THAT IS A SCIENCE PLAN SAVED ON DATABASE 2 AND/OR PRINTED OUT ON HARD COPY
904

ACCESS DATA MINING SOFTWARE
906

ACCESS DATABASE 5
908

INPUT OBSERVED VEGETATION ATTRIBUTES AND INFORMATION SAMPLED FROM REMOTE SENSING IMAGERY TO DATA MINING SOFTWARE
910

PROCESS INPUT DATA FOR REGRESSION AND/OR CLASSIFICATION PREDICTION(S) TO FULFILL ELEMENTS OF THE SCIENCE PLAN
912

SAVE OUTPUT TO DATABASE 5
914

ACCESS DATABASE 5
916

COPYRIGHT 4- PRINT OUT MATERIAL STORED ON DATABASE 5 AS AN ILLUSTRATION, TEXT, AND/OR SPREADSHEET AND/OR ON HARD COPY
918

PRINTER
920

GO TO FIG.10B

THE CLOSED ARROW IN THE PROCESS MEANS ANY AND ALL MIX OF, COMBINATION OF, AND/OR USE OF ALL PHYSICAL ELEMENTS DEFINED AS A COMPUTER WORKSTATION

THE OPEN ARROW IN THE PROCESS MEANS THE PHYSICAL INTERFACE WITH THE USER

FIG.10A

COMPUTING ENVIRONMENT

START

COMPUTER WORKSTATION

11202

ACCESS RATTLE IN R

11206

ACCESS DATABASE 5

11208

INPUT OBSERVED VEGETATION ATTRIBUTES FROM 551 SAMPLES AND THE SAMPLED INFORMATION FROM 2009 MEAN/ MAX NDVI AND MEAN EVI TO RATTLE

11210

PROCESS RATTLE FOR A RANDOM FOREST REGRESSION MODEL PREDICTION TO FULFILL ELEMENTS OF SCIENCE PLAN 4

11212

SAVE OUTPUT TO DATABASE 5

11214

ACCESS DATABASE 5

11216

COPYRIGHT 4- PRINT OUT RESULTS FROM 11212 AS AN ILLUSTRATION AND/OR IN TEXT-SEE FIGURES 10C AND 10D FOR EXAMPLES

11218

ACCESS COPYRIGHT 2 FROM STEP **10418-** IMPLEMENT SCIENCE PLAN TO DEVELOP ALLOMETRICS 2, SEE SCIENCE PLAN: DIRECTIONS 4 IN TH DETAILED DESCRIPTIONS

1104

11220

PRINTER

THE CLOSED ARROW IN THE PROCESS MEANS ANY AND ALL MIX OF, COMBINATION OF, AND/OR USE OF ALL PHYSICAL ELEMENTS DEFINED AS A COMPUTER WORKSTATION

THE OPEN ARROW IN THE PROCESS MEANS THE PHYSICAL INTERFACE WITH THE USER

GO TO FIG.12A

FIG.10B

FIG.10C

Summary of the random Forest model:
Call:
random Forest(formula = target data ~ ., data = crs$dataset[crs$sample, c(2:5), ntree = 200, mtry = 1, importance = TRUE, na.action = na.omit)
        Type of random forest: regression
            Number of trees: 200 ◄── 2502

2502

No. of variables tried at each split: 1
        Mean of squared residuals:
            % Var explained:
Variable Importance
        %IncMSE IncNodePurity

NDVI_mean
EVI_mean   ── 2504
NDVI_max

Display the model
To view model 5, for example, execute the command
  printRandomForests(crs$rf, 5)
in the R console. Generating all models will take quite some time.
Time taken:
Rattle timestamp:
= = = = = = = = = = = = = = = = = = = = = = = = = = = = = = = = = :

Random Forest Model

_____

Tree 1  Rule 1 Node 100 Regression (to do - extract predicted value) ◄── 2506
  1: NDVI_max <=9810
  3: EVI_mean <=4238.5
  3: EVI_mean <=3838.5
  4: NDVI_max <=9315.5
  5: NDVI_mean <=6542.5
  6: NDVI_max <=9146
  7: EVI_mean <=3710.5
  8: NDVI_max <=8954.5
  9: EVI_mean <=3535

_____

Tree 1  Rule 2 Node 190 Regression (to do - extract predicted value) ◄── 2506
  1: NDVI_max <=9810
  3: EVI_mean <=4238.5
  3: EVI_mean <=3838.5
  4: NDVI_max <=9315.5
  5: NDVI_mean <=6542.5
  6: NDVI_max <=9146
  7: EVI_mean <=3710.5
  8: NDVI_max <=8954.5
  9: EVI_mean <=3535
  10: NDVI_max <=8905.5
  11: EVI_mean <=3633.5

## FID.10D

EP 2 980 735 A1

Computing Environment

Start

Computer Workstation — 1002

Access input data on Database 3 — 1008

Access results for Allometrics 1 on Database 4 — 1010

Access geospatial data processing software — 1006

Access Copyright 2 from step **418** that is a science plan saved on Database 2 and/or a print out on hard copy — 1004

Process input data with results from Allometrics 1 to fulfill elements of the science plan — 1012

Save outputs to Database 6 — 1014

Access Database 6 — 1016

Copyright 5-Print out material stored on Database 6 as an atlas and/or spreadsheet that is saved on Database 6 and/or in hard copy — 1018

Printer

The closed arrow in the process means any and all mix of, combination of, And/or use of all physical elements defined as a computer workstation

The open arrow in the process means the physical interface with the user

Go to Figure 11b

FIG.11A

FIG.11B

FIG. 11C

FIG.12A

EP 2 980 735 A1

Computing Environment

11618
Access ArcGIS

11620
Upload spreadsheet to image processing software and convert to image file

Start

11602
Computer Workstation

11608
Access annual mean/max NDVI and EVI

11610
Access results for the Random Forest training model

11612
Upload annual mean/max NDVI and mean EVI to Rattle and process for the Random Forest model results

11614
Save outputs to Database 7

11616
Access to Database 7

11604
Access Copyright 2 from step **10418**-Implement science plan to implement Allometrics 2 with remote sensing imagery, see Science Plan: Directions 4 in the DETAILED DESCRIPTION

11604
Access Rattle in R

11622
Copyright 6-Print out Figure 12c and or 12d

11624
Printer

The closed arrow in the process means any and all mix of, combination of, And/or use of all physical elements defined as a computer workstation

The open arrow in the process means the physical interface with the user

Go to Figure 13a

FIG.12B

| | A | B | C | D | E | F | G |
|---|---|---|---|---|---|---|---|
| 1 | Gridcode | NDVI_max | NDVI_mean | EVI_mean | Basal Area m2/ha | Ha volume m3/ha | AGB tonnes/ha |
| 2 | 1 | 9364 | 7636 | 4726 | 20.52 | 171.60 | 363.01 |
| 3 | 2 | 9329 | 7551 | 4852 | 16.38 | 139.10 | 293.92 |
| 4 | 3 | 9339 | 7530 | 4931 | 16.79 | 142.30 | 301.80 |
| 5 | 4 | 9863 | 7780 | 5051 | 28.32 | 232.11 | 438.14 |
| 6 | 5 | 9371 | 7637 | 4770 | 21.04 | 171.97 | 366.84 |
| 7 | 6 | 9594 | 7799 | 4886 | 18.11 | 149.04 | 294.71 |
| 8 | 7 | 9713 | 7836 | 4691 | 26.61 | 204.73 | 398.43 |
| 9 | 8 | 9750 | 7515 | 4643 | 20.06 | 182.08 | 375.01 |
| 10 | 9 | 9750 | 7479 | 4868 | 14.61 | 126.22 | 289.75 |
| 11 | 10 | 9750 | 7539 | 4858 | 15.83 | 138.33 | 333.06 |
| 12 | 11 | 9578 | 7498 | 4843 | 9.43 | 80.46 | 187.78 |
| 13 | 12 | 9578 | 7737 | 4990 | 20.12 | 168.41 | 337.95 |
| 14 | 13 | 9578 | 7637 | 4752 | 14.11 | 119.74 | 272.77 |
| 15 | 14 | 9644 | 7567 | 4628 | 21.53 | 182.79 | 381.10 |
| 16 | 15 | 9644 | 7487 | 4696 | 15.60 | 128.74 | 268.87 |
| 17 | 16 | 9644 | 7549 | 4683 | 17.60 | 149.90 | 320.75 |
| 18 | 17 | 9321 | 7300 | 4607 | 19.80 | 159.41 | 329.50 |
| 19 | 18 | 9968 | 7474 | 4733 | 19.65 | 170.13 | 319.62 |
| 20 | 19 | 9256 | 7502 | 5060 | 21.49 | 202.99 | 367.21 |
| 21 | 20 | 9734 | 7414 | 4863 | 14.59 | 122.48 | 286.75 |
| 22 | 21 | 9734 | 7419 | 4725 | 15.59 | 134.41 | 298.01 |
| 23 | 22 | 9734 | 7568 | 4676 | 18.84 | 167.46 | 371.86 |
| 24 | 23 | 9734 | 7160 | 4531 | 23.82 | 221.52 | 425.47 |
| 25 | 24 | 9751 | 7465 | 4672 | 16.71 | 148.14 | 318.99 |
| 26 | 25 | 9917 | 7537 | 4840 | 18.41 | 170.51 | 346.80 |
| 27 | 26 | 9917 | 7662 | 5044 | 22.07 | 200.11 | 398.64 |
| 28 | 27 | 9930 | 7636 | 4806 | 22.46 | 193.24 | 387.81 |

11702 11704 11706 11708 11710

FIG.12C

FIG. 12D

Computing Environment

Start

**1202** Computer Workstation

**1204** A client uploads a digital boundary for a project site to a wed-interface

**1206** The digital boundary is downloaded from the web-interface

**1208** Save outputs to Database 8

**1210** Access geospatial data processing software

**1212** Access Database 8

**1214** Copyright 7-Print out material stored on Database 8 as an atlas in hard copy

**1216** Printer

Go to Figure 13b

The closed arrow in the process means any and all mix of, combination of, And/or use of all physical elements defined as a computer workstation

The open arrow in the process means the physical interface with the user

FIG.13A

EP 2 980 735 A1

COMPUTING ENVIRONMENT

START

COMPUTER WORKSTATION — 11902

DOWNLOAD WONGA WONGUE BOUNDARY — 11906

SAVE OUTPUT TO DATABASE 8 — 11908

ACCESS DATABASE — 11912

COPYRIGHT 7-PRINT OUT WONGA WONGUE AS AN ATLAS/FIGURE IN HARD COPY — 11914

UPLOAD WONGA WONGUE BOUNDARY

ACCESS ArcGIS — 11910

PRINTER — 11916

THE CLOSED ARROW IN THE PROCESS MEANS ANY AND ALL MIX OF, COMBINATION OF, AND/OR USE OF ALL PHYSICAL ELEMENTS DEFINED AS A COMPUTER WORKSTATION

THE OPEN ARROW IN THE PROCESS MEANS THE PHYSICAL INTERFACE WITH THE USER

GO TO FIGURE 14a

11918

11920

FIG.13B

Computing Environment

Access input data stored on Database 3 — 1308

Access modelled results from Allometrics 1 stored on database 6 — 1310

Start

— 1302
Computer Workstation

Access geospatial data processing software — 1304

Access modelled results from Allometrics 2 stored on Database 7 — 1312

Access digital boundary stored on to Database 8 — 1306

Sample and clip all input data required by the client for the client's digital boundary — 1314

1316 — Save outputs to Database 9

1318 — Access outputs saved on Database 9

Import to spreadsheet — 1320

Copyright 8-Print out material stored on Database 9 as an atlas or spreadsheet — 1322

Printer — 11624

The closed arrow in the process means any and all mix of, combination of, And/or use of all physical elements defined as a computer workstation

The open arrow in the process means the physical interface with the user

Go to figure 14b

FIG.14A

Computing Environment

Access input
data stored on
Database 3                    — 12008

Access modelled
results from
Allometrics 1
stored on database 6          — 12010

Start

— 12002

Computer
Workstation

Access ArcGIS               — 12004

Access modelled
results from
Allometrics 2 stored
on Database 7               — 12012

Access digital
boundary
stored on to
Database 8                  — 12006

Sample and clip all
input data required by
the client for the
client's digital
boundary                    — 12014

Save outputs to
Database 9                  — 12016

Access outputs
saved on
Database 9                  — 12018

Import to
spreadsheet                 — 12020

Copyright 8-Print out material
stored on Database 9 as an
atlas or spreadsheet        — 12022

Printer                     — 12024

Go to figure 15a

The closed arrow in the process means any and all mix of, combination of,
And/or use of all physical elements defined as a computer workstation

The open arrow in the process means the physical interface with the user

FIG.14B

EP 2 980 735 A1

Fig. 14C

COMPUTING ENVIRONMENT

START

COMPUTER WORKSTATION — 1402

ACCESS SPREADSHEET SOFTWARE — 1404

IMPORT TO SPREADSHEET SOFTWARE — 1406

DEVELOP SUMMARY STATISTICS FOR THE CLIENT'S PROJECT SITE — 1408

SAVE OUTPUTS TO DATABASE 10 — 1410

ACCESS WORD PROCESSING SOFTWARE — 1412

ACCESS COPYRIGHTS 1, 2, 3, 4, 5, 6, 7, AND 8 THAT ARE STORED ON A AFOREMENTIONED DATABASE — 1414

ACCESS MATERIAL ON COPYRIGHTS 1, 2,3, 4, 5, 6, 7, AND 8 INTO 1 DOCUMENT WITH WORD PROCESSING SOFTWARE — 1416

COMBINE SUMMARIZED MATERIAL WITH SUMMARY STATISTICS FOR THE CLIENTS PROJECT SITE AND DRAFT FINAL REPORT FOR CLIENT — 1418

ACCESS OUTPUTS SAVED ON DATABASE 10 — 1420

COPYRIGHT 9-PRINT OUT FINAL REPORT STORED ON DATABASE 10 — 1422

UPLOAD FILE OF THE FINAL REPORT TO WEB-INTERFACE — 1426

TRANSMIT FILE OF FINAL REPORT TO CLIENT — 1428

PRINTER — 1424

CLIENT

→ THE CLOSED ARROW IN THE PROCESS MEANS ANY AND ALL MIX OF, COMBINATION OF, AND/OR USE OF ALL PHYSICAL ELEMENTS DEFINED AS A COMPUTER WORKSTATION

→ THE OPEN ARROW IN THE PROCESS MEANS THE PHYSICAL INTERFACE WITH THE USER

FIG.15

FIG.16A

COMPUTING ENVIRONMENT

START

1602

COMPUTER WORKSTATION

SOFTWARE PROCESSES, INTERNET INTERFACE, PRINTING AND DATABASE INTERACTIONS FROM FIGURES 2-15

COPYRIGHT INTERACTIONS

COPYRIGHT 1 — 1604

1606

COPYRIGHT 2

COPYRIGHT 3 — 1608

COPYRIGHT 4 — 1610

COPYRIGHT 5 — 1612

COPYRIGHT 6 — 1614

COPYRIGHT 8 — 1618

COPYRIGHT 7 — 1616

COPYRIGHT 9

1620

CLIENT

→ The closed arrow in the process means any and all mix of, combination of, And/or use of all physical elements defined as a computer workstation

→ The open arrow in the process means the physical interface with the user

FIG.16B

1702
Display/
output

1704
CPU

1706
Memory

1708
Data Bus

1710
Computer Readable
recording media (e.g.,
volatile and/or non-
volatile memory,
ROM, flash memory
flash drive)

1712
Communication
transmission media
interface (e.g., wire/
wireless data network
interface)

FIG.17

**Europäisches Patentamt**
**European Patent Office**
**Office européen des brevets**

# EUROPEAN SEARCH REPORT

**Application Number**

EP 15 07 5016

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2009/086158 A2 (IMAGETREE CORP [US]; KELLE OLAVI [US]; MACOM ERIC [US]; PLISZKA ROBERT) 9 July 2009 (2009-07-09)<br>* page 5 - page 10 *<br>* page 23 - page 26 *<br>----- | 1-11 | INV.<br>G06Q10/06<br>G06Q40/04 |
| A | US 2008/177563 A1 (ZIMMERMAN PATRICK ROBERT [US]) 24 July 2008 (2008-07-24)<br>* the whole document *<br>----- | 1-11 | |

**TECHNICAL FIELDS SEARCHED (IPC)**

G06Q

The present search report has been drawn up for all claims

| Place of search | Date of completion of the search | Examiner |
|---|---|---|
| Munich | 9 October 2015 | Breidenich, Markus |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

**ANNEX TO THE EUROPEAN SEARCH REPORT
ON EUROPEAN PATENT APPLICATION NO.**                    EP 15 07 5016

This annex lists the patent family members relating to the patent documents cited in  the above-mentioned European search report.
The members are as contained in the European Patent Office EDP file on
The European Patent Office is in no way liable for these particulars which are merely given for the purpose of information.

09-10-2015

| Patent document cited in search report | | Publication date | Patent family member(s) | | Publication date |
|---|---|---|---|---|---|
| WO 2009086158 | A2 | 09-07-2009 | AU | 2008345628 A1 | 09-07-2009 |
| | | | CA | 2709377 A1 | 09-07-2009 |
| | | | EP | 2225698 A2 | 08-09-2010 |
| | | | NZ | 586188 A | 25-05-2012 |
| | | | US | 2010040260 A1 | 18-02-2010 |
| | | | WO | 2009086158 A2 | 09-07-2009 |
| US 2008177563 | A1 | 24-07-2008 | AR | 043140 A1 | 20-07-2005 |
| | | | AU | 2004211783 A1 | 26-08-2004 |
| | | | CA | 2531181 A1 | 26-08-2004 |
| | | | EP | 1631871 A2 | 08-03-2006 |
| | | | NZ | 542322 A | 30-11-2007 |
| | | | US | 2004158478 A1 | 12-08-2004 |
| | | | US | 2008177563 A1 | 24-07-2008 |
| | | | US | 2010235293 A1 | 16-09-2010 |
| | | | WO | 2004072801 A2 | 26-08-2004 |
| | | | ZA | 200507299 A | 25-10-2006 |

## REFERENCES CITED IN THE DESCRIPTION

### Patent documents cited in the description

- US 12847370 B, Matthew G. Tyburski
- US 61230235 B, Matthew G. Tyburski

### Non-patent literature cited in the description

- 2006 IPCC Guidelines for National Greenhouse Gas Inventories. *IPCC,* 2006, vol. 4
- Gross Primary Production (GPP) is the uptake of CO2 through photosynthesis. IPCC. 2006, vol. 4, 1.6-1.8
- GPG-2006 stated that the "carbon stock change that is reported in national greenhouse gas inventories for land-use categories is equal to net biome production. IPCC. 2006, vol. 4, 1-7
- IPCC Good Practice Guidance. IPCC. 2006, vol. 4
- IPCC. 2006, vol. 4, 3.6-3.7
- IPCC. 2006, vol. 4, 2-8
- IPCC. 2006, vol. 2, 2-14
- IPCC. 2006, vol. 4
- IPCC. 2006, vol. 4, 1-9
- IPCC GPG. 2006, vol. 4, 2.50-2.53
- IPCC 2006-GPG. 2006, 10-27
- GPG. 2006, vol. 4, 4-34
- IPCC Degradation Report stated that they found "very few published definitions of devegetation and they are essentially the corollaries of deforestation. *IPCC,* 2003, 17
- *IPCC,* 2003, 17
- IPCC. *VCS,* 2008, 31
- IPCC GPG. 2006, 10-27
- IPCC. 2006, vol. 4, 3-25